# EUROPEAN PATENT SPECIFICATION

(11) **EP 0 438 230 B1**
(45) Date of publication and mention of the grant of the patent: **23.04.1997**
(21) Application number: 91300180.6
(22) Date of filing: 10.01.1991
(51) Int. Cl.: C07D 413/04, C07D 413/06, C07D 413/14, C07D 417/06, A61K 31/40, A61K 31/41

(54) **Indole-substituted five-membered heteroaromatic compounds**
Indol-substituierte heteroaromatische Verbindungen
Composés hétéroaromatiques substitués par indole

(30) Priority: 17.01.1990 GB 9001018; 17.04.1990 GB 9008587
(43) Date of publication of application: 24.07.1991
(73) Proprietor: MERCK SHARP & DOHME LTD., Hoddesdon Hertfordshire EN11 9BU (GB)
(72) Inventor: Baker, Raymond, M.S.D. Research Laboratories, Harlow, Essex CM20 2QR (GB); Reeve, Austin J., M.S.D. Research Laboratories, Harlow, Essex CM20 2QR (GB); Street, Leslie J., M.S.D. Research Laboratories, Harlow, Essex CM20 2QR (GB)
(74) Representative: Thompson, John Dr.

(56) References cited:
- EP-A- 0 225 726
- EP-A- 0 313 397
- EP-A- 0 328 200
- Pharmacologie, R.Schorderet Decision Making in Drug Research, 1983, p 173-88

## Description

The present invention relates to a class of indole-substituted five-membered heteroaromatic compounds which act on 5-hydroxytryptamine (5-HT) receptors, being specific agonists of so-called "5-HT₁-like" receptors. They are therefore useful in the treatment of clinical conditions for which a selective agonist of these receptors is indicated.

5-HT₁-like receptor agonists which exhibit selective vasoconstrictor activity have recently been described as being of use in the treatment of migraine (see, for example, A. Doenicke et al., The Lancet, 1988, Vol. 1, 1309-11). The compounds of the present invention, being specific 5-HT₁-like receptor agonists, are accordingly of particular use in the treatment of migraine and associated conditions, e.g. cluster headache, chronic paroxysmal hemicrania and headache associated with vascular disorders.

EP-A-0313397 describes a class of tryptamine derivatives substituted by a five-membered heteroaliphatic ring, which are stated to act as specific agonists of a particular type of "5-HT₁-like" receptor and thus to be effective therapeutic agents for the treatment of clinical conditions, particularly migraine, requiring this activity. However, EP-A-0313397 neither discloses nor suggests the heteroaromatic compounds provided by the present invention.

EP-A-0225726 relates to indole derivatives having potent and selective vasoconstrictor activity which are stated to be useful in treating pain originating from dilation of the carotid vascular bed, in particular migraine and cluster headache. There is, however, no disclosure nor any suggestion in EP-A-0225726 of the five-membered heteroaromatic ring-containing compounds provided by the present invention.

EP-A-0328200 describes a class of 5-membered heterocyclic compounds having at least one heteroatom, substituted on the heterocyclic ring by an azacyclic or azabicyclic ring system or an amino substituent. These compounds are stated to be useful in the treatment of psychotic disorders (e.g. schizophrenia and mania); anxiety; alcohol or drug withdrawal; pain; gastric stasis; gastric dysfunction; migraine, nausea and vomiting; and presenile and senile dementia. However, they have no action on the 5-HT₁-like receptors of which the heteroaromatic compounds of the present invention are specific agonists, and therefore elicit their effect by a different mechanism.

The present invention provides a compound of formula I, or a salt thereof: 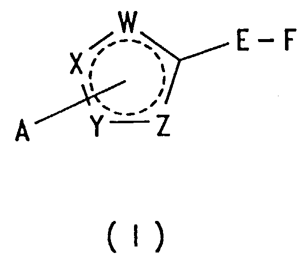 wherein the broken circle represents two non-adjacent double bonds in any position in the five-membered ring;
the five-membered ring containing the substituents W to Z represents a 1,2,4-oxadiazole, 1,3,4-oxadiazole, 1,2,4-thiadiazole, 1,3,4-thiadiazole, 1,3-oxazole or 1,3-thiazole ring;
A represents methyl, methoxymethyl, aminomethyl, dimethylaminomethyl, acetylaminomethyl, benzoylaminomethyl, t-butoxy-carbonylaminomethyl, methylsulphonylaminomethyl, phenylsulphonylaminomethyl, aminocarbonylmethyl, ethyl, aminoethyl, acetylaminoethyl, benzoylaminoethyl, methoxycarbonylaminoethyl, ethoxycarbonylaminoethyl, t-butoxycarbonylaminoethyl, methylsulphonylaminoethyl, aminocarbonylaminoethyl, methylaminocarbonylaminoethyl, t-butylaminocarbonylaminoethyl, phenylaminocarbonylaminoethyl, pyrrolidylcarbonylaminoethyl, cyclopropyl, phenyl, methylsulphonylaminophenyl, aminocarbonylphenyl, methylaminocarbonylphenyl, methylsulphonylaminomethylphenyl, aminosulphonylmethylphenyl, methylaminosulphonylmethylphenyl, dimethylaminosulphonylmethylphenyl, naphthyl, benzyl, diphenylmethyl, trifluoromethylbenzyl, methoxybenzyl, acetylaminobenzyl, methylsulphonylaminobenzyl, aminocarbonylaminobenzyl, aminocarbonylbenzyl, methylaminocarbonylbenzyl, methylsulphonylbenzyl, methylaminosulphonylbenzyl, phenethyl, phenylpropyl, acetylpiperazinyl, methoxycarbonylpiperazinyl, t-butoxycarbonylpiperazinyl, methylaminocarbonylpiperazinyl, methylsulphonylpiperazinyl, phenylsulphonylpiperazinyl, pyridylmethyl, methoxypyridylmethyl, amino, methylamino, benzylamino, dimethylamino, t-butoxycarbonylaminoethylamino, methylsulphonylaminoethylamino, aminocarbonyl, methylaminocarbonyl, azetidinylcarbonyl or pyrrolidylcarbonyl;
E represents a bond or a straight or branched alkylene chain containing from 1 to 4 carbon atoms;
F represents a group of formula 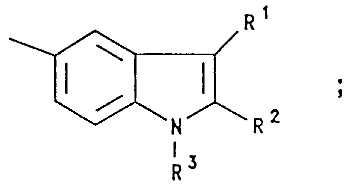
R¹ represents aminoethyl, N-methylaminoethyl, N,N-dimethylaminoethyl or 1-methyl-4-piperidyl; and
R² and R³ independently represent hydrogen or C₁₋₆ alkyl.

For use in medicine, the salts of the compounds of formula I will be non-toxic pharmaceutically acceptable salts. Other salts may, however, be useful in the preparation of the compounds according to the invention or of their non-toxic pharmaceutically acceptable salts. Suitable pharmaceutically acceptable salts of the compounds of this invention include acid addition salts which may, for example, be formed by mixing a solution of the compound according to the invention with a solution of a pharmaceutically acceptable non-toxic acid such as hydrochloric acid, fumaric acid, maleic acid, succinic acid, acetic acid, citric acid, tartaric acid, carbonic acid or phosphoric acid. Furthermore, where the compounds of the invention carry an acidic moiety, suitable pharmaceutically acceptable salts thereof may include alkali metal salts, e.g. sodium or potassium salts; alkaline earth metal salts, e.g. calcium or magnesium salts; and salts formed with suitable organic ligands, e.g. quaternary ammonium salts.

Suitable alkyl groups include straight-chained and branched alkyl groups containing from 1 to 6 carbon atoms. Typical examples include methyl and ethyl groups, and straight-chained or branched propyl and butyl groups. Particular alkyl groups are methyl, ethyl and t-butyl.

Where the compounds according to the invention have at least one asymmetric centre, they may accordingly exist as enantiomers. Where the compounds according to the invention possess two or more asymmetric centres, they may additionally exist as diastereoisomers. It is to be understood that all such isomers and mixtures thereof are encompassed within the scope of the present invention.

Preferably, the five-membered heteroaromatic ring in formula I containing the substituents W to Z is a 1,2,4-oxadiazole, 1,2,4-thiadiazole, 1,3-oxazole or 1,3-thiazole ring.

The alkylene chain E may be, for example, methylene, ethylene, 1-methylethylene, propylene or 2-methylpropylene. Alternatively, the group E may represent a single bond such that the indole moiety F in formula I is attached directly to the five-membered heteroaromatic ring.

Preferably, R¹ represents aminoethyl or N,N-dimethylaminoethyl.

The groups R² and R³ independently represent hydrogen or C₁₋₆ alkyl, in particular hydrogen or methyl.

A particular sub-class of compounds according to the invention is represented by the compounds of formula IIA, and salts thereof: 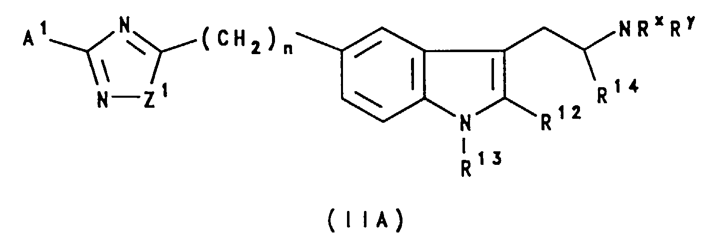 wherein
Z¹ represents oxygen or sulphur;
n is zero, 1, 2 or 3;
A¹ corresponds to the group A as defined above;
R¹², R¹³ and R¹⁴ each represents hydrogen; and
R^{x} and R^{y} independently represent hydrogen or methyl.

In a preferred embodiment, A¹ represents amino.

Another sub-class of compounds according to the invention is represented by the compounds of formula IIB, and salts thereof: 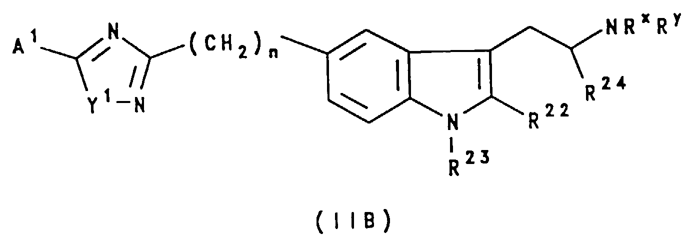 wherein
Y¹ represents oxygen or sulphur;
n is zero, 1, 2 or 3;
A¹ is as defined with reference to formula IIA above;
R²², R²³ and R²⁴ each represents hydrogen; and
R^{x} and R^{y} independently represent hydrogen or methyl.

Particular values of A¹ with respect to formula IIB include methyl and benzyl.

A further sub-class of compounds according to the invention is represented by the compounds of formula IIC, and salts thereof: 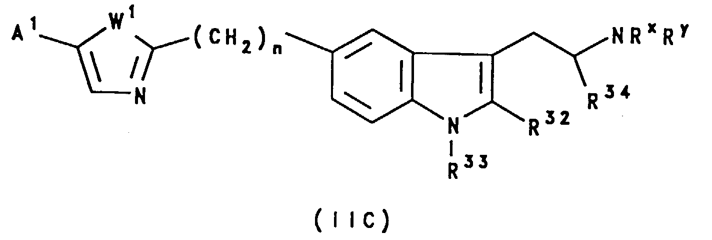 wherein
W¹ represents oxygen or sulphur;
n is zero, 1, 2 or 3;
A¹ is as defined with reference to formula IIA above;
R³², R³³ and R³⁴ each represents hydrogen; and
R^{x} and R^{y} independently represent hydrogen or methyl.

A particular value of A¹ with respect to formula IIC is methyl.

A still further sub-class of compounds according to the invention is represented by the compounds of formula IID, and salts thereof: 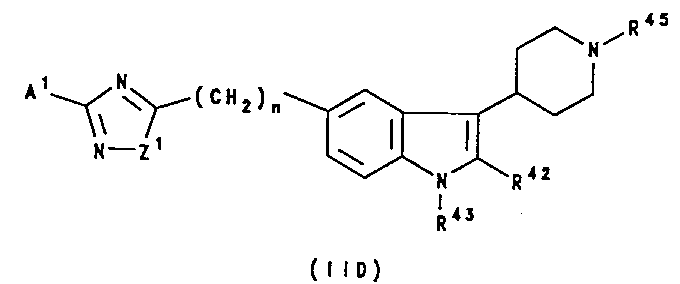 wherein
Z¹ represents oxygen or sulphur;
n is zero, 1, 2 or 3;
A¹ is as defined with reference to formula IIA above;
R⁴² and R⁴³ each represents hydrogen; and
R⁴⁵ represents methyl.

Particular values of A¹ with respect to formula IID include amino, and optionally substituted benzyl or pyridylmethyl, especially methylsulphonylaminobenzyl.

Specific compounds within the scope of the present invention include:
2-[5-(3-benzyl-1,2,4-oxadiazol-5-yl)-1H-indol-3-yl]ethylamine;
2-[5-(3-methyl-1,2,4-oxadiazol-5-yl)-1H-indol-3-yl]ethylamine;
N,N-dimethyl-2-[5-(3-benzyl-1,2,4-oxadiazol-5-yl)-1H-indol-3-yl]ethylamine;
2-[5-(3-benzyl-1,2,4-oxadiazol-5-ylmethyl)-1H-indol-3-yl]ethylamine;
2-[5-(3-methyl-1,2,4-oxadiazol-5-ylmethyl)-1H-indol-3-yl]ethylamine;
2-[5-(3-amino-1,2,4-oxadiazol-5-ylmethyl)-1H-indol-3-yl]ethylamine;
2-[5-(3-phenyl-1,2,4-oxadiazol-5-ylmethyl)-1H-indol-3-yl]ethylamine;
2-[5-[3-(2-methoxybenzyl)-1,2,4-oxadiazol-5-ylmethyl]-1H-indol-3-yl]ethylamine;
N,N-dimethyl-2-[5-(3-benzyl-1,2,4-oxadiazol-5-ylmethyl)-1H-indol-3-yl]ethylamine;
2-[5-[2-(3-benzyl-1,2,4-oxadiazol-5-yl)ethyl]-1H-indol-3-yl]ethylamine;
N,N-dimethyl-2-[5-(3-methyl-1,2,4-oxadiazol-5-ylmethyl)-1H-indol-3-yl]ethylamine;
2-[5-(3-diphenylmethyl-1,2,4-oxadiazol-5-yl)-1H-indol-3-yl]ethylamine;
2-[5-(3-phenyl-1,2,4-oxadiazol-5-yl)-1H-indol-3-yl]ethylamine;
2-[5-[3-(2-methoxybenzyl)-1,2,4-oxadiazol-5-yl]-1H-indol-3-yl]ethylamine;
2-[5-[3-(3-benzyl-1,2,4-oxadiazol-5-yl)propyl]-1H-indol-3-yl]ethylamine;
2-[5-(3-phenethyl-1,2,4-oxadiazol-5-yl)-1H-indol-3-yl]ethylamine;
2-[5-(5-benzyl-1,2,4-oxadiazol-3-yl)-1H-indol-3-yl]ethylamine;
2-[5-(5-benzyl-1,2,4-oxadiazol-3-ylmethyl)-1H-indol-3-yl]ethylamine;
N,N-dimethyl-2-[5-[3-(2-methoxybenzyl)-1,2,4-oxadiazol-5-yl]-1H-indol-3-yl]ethylamine;
N,N-dimethyl-2-[5-[2-(3-benzyl-1,2,4-oxadiazol-5-yl)ethyl]-1H-indol-3-yl]ethylamine;
2-[5-[3-(1-naphthyl)-1,2,4-oxadiazol-5-ylmethyl]-1H-indol-3-yl]ethylamine;
2-[5-[3-(3-methyl-1,2,4-oxadiazol-5-yl)propyl]-1H-indol-3-yl]ethylamine;
2-[5-[3-(3-cyclopropyl-1,2,4-oxadiazol-5-yl)propyl]-1H-indol-3-yl]ethylamine;
2-[5-[3-(3-methoxybenzyl)-1,2,4-oxadiazol-5-yl]-1H-indol-3-yl]ethylamine;
2-[5-[3-(4-methoxybenzyl)-1,2,4-oxadiazol-5-yl]-1H-indol-3-yl]ethylamine;
2-[5-[3-(4-acetylaminobenzyl)-1,2,4-oxadiazol-5-yl]-1H-indol-3-yl]ethylamine;
2-[5-[3-(4-methylsulphonylaminobenzyl)-1,2,4-oxadiazol-5-yl]-1H-indol-3-yl]ethylamine;
2-[5-[3-(3-phenylpropyl)-1,2,4-oxadiazol-5-yl]-1H-indol-3-yl]ethylamine;
2-[5-(3-cyclopropyl-1,2,4-oxadiazol-5-yl)-1H-indol-3-yl]ethylamine;
2-[5-(3-ethyl-1,2,4-oxadiazol-5-yl)-1H-indol-3-yl]ethylamine;
2-[5-[3-(4-trifluoromethylbenzyl)-1,2,4-oxadiazol-5-yl]-1H-indol-3-yl]ethylamine;
N,N-dimethyl-2-[5-[3-(4-acetylaminobenzyl)-1,2,4-oxadiazol-5-yl]-1H-indol-3-yl]ethylamine;
N,N-dimethyl-2-[5-[3-(4-methylsulphonylaminobenzyl)-1,2,4-oxadiazol-5-yl]-1H-indol-3-yl]ethylamine;
N,N-dimethyl-2-[5-(3-amino-1,2,4-oxadiazol-5-ylmethyl)-1H-indol-3-yl]ethylamine;
2-[5-[2-(3-amino-1,2,4-oxadiazol-5-yl)ethyl]-1H-indol-3-yl]ethylamine;
2-[5-[2-(3-dimethylamino-1,2,4-oxadiazol-5-yl)ethyl]-1H-indol-3-yl]ethylamine;
2-[5-(5-methyl-1,2,4-oxadiazol-3-yl)-1H-indol-3-yl]ethylamine;
2-[5-(5-methyl-1,2,4-oxadiazol-3-ylmethyl)-1H-indol-3-yl]ethylamine;
N,N-dimethyl-2-[5-(5-benzyl-1,2,4-oxadiazol-3-ylmethyl)-1H-indol-3-yl]ethylamine;
2-[5-(3-methoxymethyl-1,2,4-oxadiazol-5-yl)-1H-indol-3-yl]ethylamine;
2-[5-[3-(4-methylaminocarbonylbenzyl)-1,2,4-oxadiazol-5-yl]-1H-indol-3-yl]ethylamine;
2-[5-[3-(4-methylaminocarbonylphenyl)-1,2,4-oxadiazol-5-yl]-1H-indol-3-yl]ethylamine;
2-[5-[3-(4-methylaminosulphonylbenzyl)-1,2,4-oxadiazol-5-yl]-1H-indol-3-yl]ethylamine;
2-[5-[3-(4-methylsulphonylbenzyl)-1,2,4-oxadiazol-5-yl]-1H-indol-3-yl]ethylamine;
2-[5-[3-(3-methylsulphonylaminobenzyl)-1,2,4-oxadiazol-5-yl]-1H-indol-3-yl]ethylamine;
2-[5-[3-(4-aminocarbonylaminobenzyl)-1,2,4-oxadiazol-5-yl]-1H-indol-3-yl]ethylamine;
2-[5-(3-amino-1,2,4-oxadiazol-5-yl)-1H-indol-3-yl]ethylamine;
2-[5-(3-acetylaminomethyl-1,2,4-oxadiazol-5-yl)-1H-indol-3-yl]ethylamine;
2-[5-[3-(2-acetylaminoethyl)-1,2,4-oxadiazol-5-yl]-1H-indol-3-yl]ethylamine;
2-[5-(3-aminomethyl-1,2,4-oxadiazol-5-yl)-1H-indol-3-yl]ethylamine;
N,N-dimethyl-2-[5-(3-amino-1,2,4-oxadiazol-5-yl)-1H-indol-3-yl]ethylamine;
N,N-dimethyl-2-[5-(3-acetylaminomethyl-1,2,4-oxadiazol-5-yl)-1H-indol-3-yl]ethylamine;
N,N-dimethyl-2-[5-[3-(2-acetylaminoethyl)-1,2,4-oxadiazol-5-yl]-1H-indol-3-yl]ethylamine;
N,N-dimethyl-2-[5-[3-(4-aminocarbonylaminobenzyl)-1,2,4-oxadiazol-5-yl]-1H-indol-3-yl]ethylamine;
N,N-dimethyl-2-[5-[3-(2-(t-butoxycarbonylamino)ethyl)-1,2,4-oxadiazol-5-yl]-1H-indol-3-yl]ethylamine;
N,N-dimethyl-2-[5-[3-(4-methylaminocarbonylbenzyl)-1,2,4-oxadiazol-5-yl]-1H-indol-3-yl]ethylamine;
N,N-dimethyl-2-[5-[3-(2-aminoethyl)-1,2,4-oxadiazol-5-yl]-1H-indol-3-yl]ethylamine;
N,N-dimethyl-2-[5-[3-(2-methylsulphonylaminoethyl)-1,2,4-oxadiazol-5-yl]-1H-indol-3-yl]ethylamine;
N,N-dimethyl-2-[5-[3-(2-aminocarbonylaminoethyl)-1,2,4-oxadiazol-5-yl]-1H-indol-3-yl]ethylamine;
N,N-dimethyl-2-[5-[3-(2-methylaminocarbonylaminoethyl)-1,2,4-oxadiazol-5-yl]-1H-indol-3-yl]ethylamine;
N,N-dimethyl-2-[5-[3-(2-methylaminocarbonylaminoethyl)-1,2,4-oxadiazol-5-yl]-1H-indol-3-yl]ethylamine;
N,N-dimethyl-2-[5-[3-(2-methoxycarbonylaminoethyl)-1,2,4-oxadiazol-5-yl]-1H-indol-3-yl]ethylamine;
N,N-dimethyl-2-[5-[3-(2-ethoxycarbonylaminoethyl)1,2,4-oxadiazol-5-yl]-1H-indol-3-yl]ethylamine;
N,N-dimethyl-2-[5-[2-(3-amino-1,2,4-oxadiazol-5-yl)ethyl]-1H-indol-3-yl]ethylamine;
N,N-dimethyl-2-[5-(3-methylamino-1,2,4-oxadiazol-5-ylmethyl)-1H-indol-3-yl]ethylamine;
N,N-dimethyl-2-[5-[3-(4-aminocarbonylbenzyl)-1,2,4-oxadiazol-5-ylmethyl]-1H-indol-3-yl]ethylamine;
N,N-dimethyl-2-[5-[3-(4-acetylaminobenzyl)-1,2,4-oxadiazol-5-ylmethyl]-1H-indol-3-yl]ethylamine;
N,N-dimethyl-2-[5-[3-(4-methylaminosulphonylbenzyl)-1,2,4-oxadiazol-5-ylmethyl]-1H-indol-3-yl]ethylamine;
N,N-dimethyl-2-[5-[3-(4-aminocarbonylaminobenzyl)-1,2,4-oxadiazol-5-ylmethyl]-1H-indol-3-yl]ethylamine;
N,N-dimethyl-2-[5-[3-(4-methylsulphonylaminobenzyl)-1,2,4-oxadiazol-5-ylmethyl]-1H-indol-3-yl]ethylamine;
N,N-dimethyl-2-[5-[3-(4-methylaminocarbonylphenyl)-1,2,4-oxadiazol-5-ylmethyl]-1H-indol-3-yl]ethylamine;
N,N-dimethyl-2-[5-(3-acetylaminomethyl-1,2,4-oxadiazol-5-ylmethyl)-1H-indol-3-yl]ethylamine;
N,N-dimethyl-2-[5-(3-methylsulphonylaminomethyl-1,2,4-oxadiazol-5-ylmethyl)-1H-indol-3-yl]ethylamine;
N,N-dimethyl-2-[5-(3-aminocarbonylmethyl-1,2,4-oxadiazol-5-ylmethyl)-1H-indol-3-yl]ethylamine;
N,N-dimethyl-2-[5-[3-(3-methylsulphonylaminobenzyl)-1,2,4-oxadiazol-5-ylmethyl]-1H-indol-3-yl]ethylamine;
N,N-dimethyl-2-[5-[3-(3-acetylaminobenzyl)-1,2,4-oxadiazol-5-ylmethyl]-1H-indol-3-yl]ethylamine;
N,N-dimethyl-2-[5-[3-(4-aminocarbonylphenyl)-1,2,4-oxadiazol-5-ylmethyl]-1H-indol-3-yl]ethylamine;
N,N-dimethyl-2-[5-[3-(3-aminocarbonylphenyl)-1,2,4-oxadiazol-5-ylmethyl]-1H-indol-3-yl]ethylamine;
N,N-dimethyl-2-[5-[3-(4-methylsulphonylaminophenyl)-1,2,4-oxadiazol-5-ylmethyl]-1H-indol-3-yl]ethylamine;
N,N-dimethyl-2-[5-[3-(4-methylaminosulphonylmethylphenyl)-1,2,4-oxadiazol-5-ylmethyl]-1H-indol-3-yl]ethylamine;
N,N-dimethyl-2-[5-[3-(3-methylaminosulphonylmethylphenyl)-1,2,4-oxadiazol-5-ylmethyl]-1H-indol-3-yl]ethylamine;
N,N-dimethyl-2-[5-[3-(4-aminosulphonylmethylphenyl)-1,2,4-oxadiazol-5-ylmethyl]-1H-indol-3-yl]ethylamine;
N,N-dimethyl-2-[5-[3-(4-dimethylaminosulphonylmethylphenyl)-1,2,4-oxadiazol-5-ylmethyl]-1H-indol-3-yl]ethylamine;
N,N-dimethyl-2-[5-[3-(t-butoxycarbonylamino)methyl-1,2,4-oxadiazol-5-ylmethyl]-1H-indol-3-yl]ethylamine;
N,N-dimethyl-2-[5-[3-(2-(t-butoxycarbonylamino)ethyl)-1,2,4-oxadiazol-5-ylmethyl]-1H-indol-3-yl]ethylamine;
N,N-dimethyl-2-[5-(3-aminomethyl-1,2,4-oxadiazol-5-ylmethyl)-1H-indol-3-yl]ethylamine;
N,N-dimethyl-2-[5-(3-methoxycarbonylaminomethyl-1,2,4-oxadiazol-5-ylmethyl)-1H-indol-3-yl]ethylamine;
N,N-dimethyl-2-[5-(3-dimethylaminomethyl-1,2,4-oxadiazol-5-ylmethyl)-1H-indol-3-yl]ethylamine;
N,N-dimethyl-2-[5-[3-(2-methylsulphonylaminoethyl)-1,2,4-oxadiazol-5-ylmethyl]-1H-indol-3-yl]ethylamine;
N,N-dimethyl-2-[5-[3-(2-ethoxycarbonylaminoethyl)-1,2,4-oxadiazol-5-ylmethyl]-1H-indol-3-yl]ethylamine;
N,N-dimethyl-2-[5-(3-benzoylaminomethyl-1,2,4-oxadiazol-5-ylmethyl)-1H-indol-3-yl]ethylamine;
N,N-dimethyl-2-[5-[3-(2-benzoylaminoethyl)-1,2,4-oxadiazol-5-ylmethyl]-1H-indol-3-yl]ethylamine;
N,N-dimethyl-2-[5-[3-(2-phenylaminocarbonylaminoethyl)-1,2,4-oxadiazol-5-ylmethyl]-1H-indol-3-yl]ethylamine;
N,N-dimethyl-2-[5-[3-(2-(t-butylaminocarbonylamino)ethyl)-1,2,4-oxadiazol-5-ylmethyl]-1H-indol-3-yl]ethylamine;
N-methyl-2-[5-(3-amino-1,2,4-oxadiazol-5-ylmethyl)-1H-indol-3-yl]ethylamine;
N,N-dimethyl-2-[5-[3-(4-(t-butoxycarbonyl)piperazin-1-yl)-1,2,4-oxadiazol-5-ylmethyl]-1H-indol-3-yl]ethylamine;
N,N-dimethyl-2-[5-[3-(4-methylsulphonylpiperazin-1-yl)-1,2,4-oxadiazol-5-ylmethyl]-1H-indol-3-yl]ethylamine;
N,N-dimethyl-2-[5-[3-(4-methoxycarbonylpiperazin-1-yl)-1,2,4-oxadiazol-5-ylmethyl]-1H-indol-3-yl]ethylamine;
N,N-dimethyl-2-[5-[3-(4-methylaminocarbonylpiperazin-1-yl)-1,2,4-oxadiazol-5-ylmethyl]-1H-indol-3-yl]ethylamine;
N,N-dimethyl-2-[5-[3-(4-acetylpiperazin-1-yl)-1,2,4-oxadiazol-5-ylmethyl]-1H-indol-3-yl]ethylamine;
N,N-dimethyl-2-[5-[3-(4-methylsulphonylaminomethylphenyl)-1,2,4-oxadiazol-5-yl-methyl]-1H-indol-3-yl]ethylamine;
N,N-dimethyl-2-[5-(3-phenylsulphonylaminomethyl-1,2,4-oxadiazol-5-ylmethyl)-1H-indol-3-yl]ethylamine;
N,N-dimethyl-2-[5-(3-benzylamino-1,2,4-oxadiazol-5-ylmethyl)-1H-indol-3-yl]ethylamine;
N,N-dimethyl-2-[5-[3-(3-pyridyl)methyl-1,2,4-oxadiazol-5-ylmethyl]-1H-indol-3-yl]ethylamine;
N,N-dimethyl-2-[5-[3-(2-methoxypyrid-5-yl)methyl-1,2,4-oxadiazol-5-ylmethyl]-1H-indol-3-yl]ethylamine;
2-[5-[3-(4-acetylaminobenzyl)-1,2,4-oxadiazol-5-ylmethyl]-1H-indol-3-yl]ethylamine;
2-[5-[3-(4-methylsulphonylaminobenzyl)-1,2,4-oxadiazol-5-ylmethyl]-1H-indol-3-yl]ethylamine;
2-[5-[2-[3-(4-acetylaminobenzyl)-1,2,4-oxadiazol-5-yl]ethyl]-1H-indol-3-yl]ethylamine;
2-[5-[2-[3-(4-methoxybenzyl)-1,2,4-oxadiazol-5-yl]ethyl]-1H-indol-3-yl]ethylamine;
N,N-dimethyl-2-[5-(5-methyl-1,3-oxazol-2-yl)-1H-indol-3-yl]ethylamine;
N,N-dimethyl-2-[5-[2-(5-methyl-1,3-oxazol-2-yl)ethyl]-1H-indol-3-yl]ethylamine;
1-methyl-4-[5-(3-amino-1,2,4-oxadiazol-5-yl)-1H-indol-3-yl]piperidine;
1-methyl-4-[5-(3-amino-1,2,4-oxadiazol-5-ylmethyl)-1H-indol-3-yl]piperidine;
1-methyl-4-[5-[3-(4-methylsulphonylaminobenzyl)-1,2,4-oxadiazol-5-ylmethyl]-1H-indol-3-yl]piperidine;
1-methyl-4-[5-[3-(3-pyridyl)methyl-1,2,4-oxadiazol-5-ylmethyl]-1H-indol-3-yl]piperidine;
N,N-dimethyl-2-[5-[3-(4-pyridyl)methyl-1,2,4-oxadiazol-5-ylmethyl]-1H-indol-3-yl]ethylamine;
N,N-dimethyl-2-[5-[3-(2-(t-butoxycarbonylamino)ethyl)amino-1,2,4-oxadiazol-5-ylmethyl]-1H-indol-3-yl]ethylamine;
2-[5-(3-aminocarbonyl-1,2,4-oxadiazol-5-ylmethyl)-1H-indol-3-yl]ethylamine;
2-[5-(3-methylaminocarbonyl-1,2,4-oxadiazol-5-ylmethyl)-1H-indol-3-yl]ethylamine;
2-[5-[3-(pyrrolid-1-yl)carbonyl-1,2,4-oxadiazol-5-ylmethyl]-1H-indol-3-yl]ethylamine;
2-[5-[3-(azetidin-1-yl)carbonyl-1,2,4-oxadiazol-5-ylmethyl]1H-indol-3-yl]ethylamine;
N,N-dimethyl-2-[5-[3-(4-phenylsulphonylpiperazin-1-yl)-1,2,4-oxadiazol-5-ylmethyl]-1H-indol-3-yl]ethylamine;
N,N-dimethyl-2-[5-[3-(2-(pyrrolid-1-ylcarbonylamino)ethyl)-1,2,4-oxadiazol-5-ylmethyl]-1H-indol-3-yl]ethylamine;
N,N-dimethyl-2-[5-[3-(2-methylsulphonylaminoethyl)amino-1,2,4-oxadiazol-5-ylmethyl]-1H-indol-3-yl]ethylamine;
N,N-dimethyl-2-[5-(3-amino-1,4-thiadiazol-5-ylmethyl)-1H-indol-3-yl]ethylamine;
and salts thereof.

The invention also provides pharmaceutical compositions comprising one or more compounds of this invention in association with a pharmaceutically acceptable carrier. Preferably these compositions are in unit dosage forms such as tablets, pills, capsules, powders, granules, sterile parenteral solutions or suspensions, or suppositories, for oral, parenteral or rectal administration. For preparing solid compositions such as tablets, the principal active ingredient is mixed with a pharmaceutical carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present invention, or a non-toxic pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation composition is then subdivided into unit dosage forms of the type described above containing from 0.1 to about 500 mg of the active ingredient of the present invention. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, cetyl alcohol and cellulose acetate.

The liquid forms in which the novel compositions of the present invention may be incorporated for administration orally or by injection include aqueous solutions, suitably flavoured syrups, aqueous or oil suspensions, and flavoured emulsions with edible oils such as cottonseed oil, sesame oil, coconut oil or peanut oil, as well as elixirs and similar pharmaceutical vehicles. Suitable dispersing or suspending agents for aqueous suspensions include synthetic and natural gums such as tragacanth, acacia, alginate, dextran, sodium carboxymethylcellulose, methylcellulose, polyvinylpyrrolidone or gelatin.

In the treatment of migraine, a suitable dosage level is about 0.01 to 250 mg/kg per day, preferably about 0.05 to 100 mg/kg per day, and especially about 0.05 to 5 mg/kg per day. The compounds may be administered on a regimen of 1 to 4 times per day.

The oxadiazole compounds of this invention may be prepared by a process which comprises reacting a reactive derivative of a carboxylic acid of formula R^{c}-CO₂H with a compound either of formula III or of formula IV, or a salt thereof: 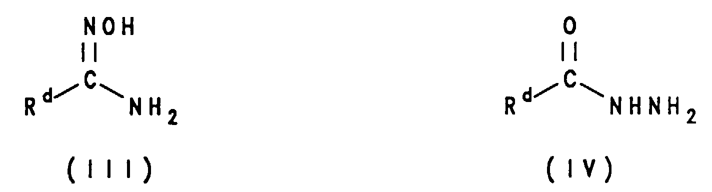 wherein one of R^{c} and R^{d} is a group of formula A, and the other is a group of formula -E-F, as defined with reference to formula I above.

Suitable reactive derivatives of the acid R^{c}-CO₂H include esters, for example C₁₋₄ alkyl esters; thioesters, for example pyridylthioesters; acid anhydrides, for example (R^{c}CO)₂O; acid halides, for example acid chlorides; orthoesters; and primary, secondary and tertiary amides.

A preferred reactive derivative of the acid R^{c}-CO₂H is the iminoether derivative of formula V: 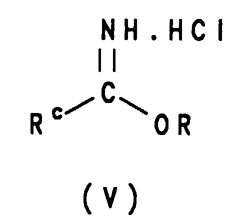 where R is C₁₋₄ alkyl.

When the compound of formula III is employed the product of the reaction is a 1,2,4-oxadiazole. It will be appreciated that the compound III can also be considered as the alternative tautomeric form IIIA: 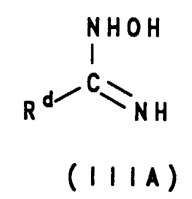 wherein R^{d} is as defined above.

A 3-substituted-1,2,4-oxadiazol-5-yl compound is produced if R^{c} represents a group -E-F and R^{d} in formula III represents a group A; whereas a 5-substituted-1,2,4-oxadiazol-3-yl compound is produced by the process of this invention when R^{c} represents a group A and R^{d} represents a group -E-F. A preferred reactive derivative of the acid R^{c}-CO₂H in this case is a C₁₋₄ alkyl ester. The reaction is conveniently carried out in the presence of a strong base, e.g. sodium hydride, using a suitable solvent, for example tetrahydrofuran, dimethylformamide or a lower alkanol such as ethanol, propanol or isopropanol, at about 20°C to 100°C for about 1 to 6 hours.

When the compound of formula IV is employed, the product of the process of this invention is a 1,3,4-oxadiazole. In this case, a preferred reactive derivative of the acid R^{c}-CO₂H is an orthoester of formula R^{c}C(OR^{p})₃ where R^{p} represents C₁₋₃ alkyl. The process is conveniently effected by heating the hydrazide IV with the orthoester in a solvent such as methanol at reflux temperature for about 2 to 8 hours. An intermediate of formula R^{d}.CO.NH.N=C(R^{c})OR^{p} may be isolated by evaporation of the solvent. The intermediate is then treated with a strong base such as potassium t-butoxide or 1,8-diazabicyclo[5.4.0]undec-7-ene, in butanol for about 10 to 24 hours at about 90°C to 150°C.

The reactive derivative of a carboxylic acid of formula R^{c}-CO₂H or the compound of formula III or IV, wherein R^{c} or R^{d} represents a group of formula -E-F, may be prepared by reacting a compound of formula VI: 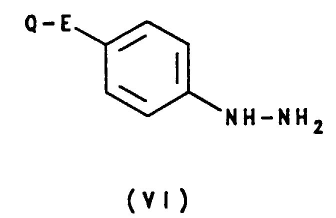 wherein Q represents a reactive carboxylate moiety, or a group of formula -C(NON)NH₂ or -CONHNH₂ or a protected derivative thereof or precursor thereto; and E is as defined above; with a compound of formula VII or a carbonyl-protected form thereof: 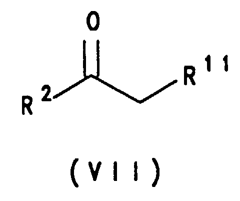 wherein R² is as defined above and R¹¹ corresponds to the group R¹ as defined above or represents a group of formula -CH₂.CHR⁴D, in which R⁴ is hydrogen and D represents a readily displaceable group; followed, where required, by N-alkylation by standard methods to introduce the moiety R³.

When the moiety Q in the compounds of formula VI represents a precursor to a group of formula -C(NOH)NH₂ or -CONHNH₂, this group is suitably a nitrile group.

Suitable carbonyl-protected forms of the compounds of formula VII include the dimethyl acetal or ketal derivatives.

The readily displaceable group D in the compounds of formula VII suitably represents a halogen group, preferably chlorine. When the moiety R¹¹ in the compounds of formula VII is a group of formula -CH₂.CHR⁴D, the substituent D is displaced in situ under the prevailing reaction conditions to afford a final product of formula I wherein R¹ represents a group of formula -CH₂.CHR⁴.NH₂. The terminal amino group can subsequently, if desired, be further elaborated using techniques known from the art to give a compound of formula I wherein R¹ represents N-methylaminoethyl or N,N-dimethylaminoethyl.

The reaction of compounds VI and VII may be carried out in a single step (Fischer indole synthesis) or by an initial non-cyclising step at a lower temperature to give a compound of formula VIII: 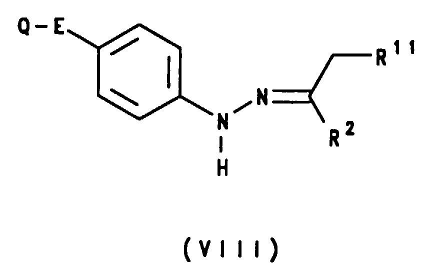 wherein Q, E, R² and R¹¹ are as defined above; followed by cyclisation using a suitable reagent, such as a polyphosphate ester, to give a compound of formula Q-E-F.

The hydrazines of formula VI may be prepared from the corresponding anilines of formula IX: 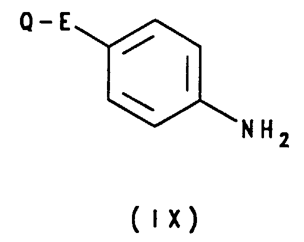 wherein Q and E are as defined above; by diazotisation followed by reduction. Diazotisation is typically carried out using sodium nitrite/conc. HCl and the resulting diazo product reduced in situ using, for example, tin(II) chloride/conc. HCl.

The anilines of formula IX may be prepared by reduction of the corresponding nitro compounds of formula X: 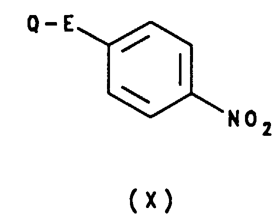 wherein Q and E are as defined above; typically by catalytic hydrogenation or using tin(II) chloride.

Where they are not commercially available, the nitro compounds of formula X may be synthesized by standard methods well known to those skilled in the art.

The 1,2,4-thiadiazoles of formula I may be prepared by a process which comprises the cyclisation of a compound of formula XI: 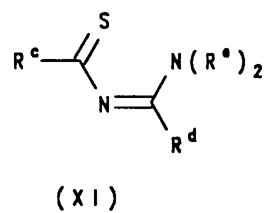 wherein R^{c} and R^{d} are as defined above, and R^{e} is hydrogen or an alkyl group.

Cyclisation of compound XI can be achieved using an aminating agent such as hydroxylamine-O-sulphonic acid in a lower alkanol such as methanol, ethanol or propanol, in the presence of pyridine, at between -20°C and 50°C for about 1-6 hours.

Cyclisation of compounds of formula XI in which R^{e} is hydrogen may also be achieved by use of an oxidising agent such as bromine, iodine, hydrogen peroxide or nitric acid.

The compounds of formula XI above may be prepared by the processes described in Comprehensive Heterocyclic Chemistry, ed. A.R. Katritzky and C.W. Rees, Pergamon Press, 1984, Vol. 6, p. 496, or by methods analogous thereto.

The 1,2,4-thiadiazoles may also be prepared by cycloaddition of a nitrile sulphide R^{c}-C≡N⁺-S⁻ with a nitrile of formula R^{d}-CN where R^{c} and R^{d} are as defined above.

1,3,4-Thiadiazoles of this invention may be prepared by dehydration of a thiosemicarbazide of formula R^{c}CSNHNHCONR^{s}R^{t}, where R^{c} is as defined above and R^{s} and R^{t} are hydrogen or an alkyl group, with a dehydrating agent such as sulphuric acid, polyphosphoric acid or methanesulphonic acid; followed by attachment of the R^{d} group by conventional means.

The oxazoles and thiazoles of this invention may be prepared by reaction of an amide or thioamide of formula XII with a α-haloketone of formula XIII: 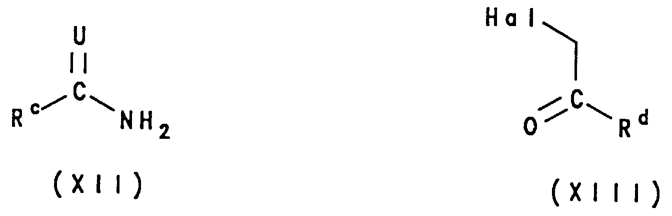 wherein U is oxygen or sulphur, Hal represents halogen, and R^{c} and R^{d} are as defined above. The conditions for this reaction are as described in Synthesis, 1975, 389.

In an alternative process, the compounds according to the invention may be prepared by a method which comprises reacting a compound of formula XV: 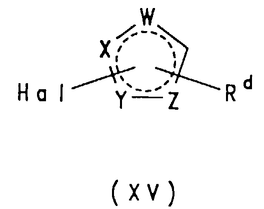 with a reagent which provides an anion ⁻R^{c}, where W, X, Y, Z, R^{c} and R^{d} are as previously defined and Hal represents halogen.

Compound XV may be prepared by conventional procedures known from the art. For example, if compound XV is a 1,2,4-thiadiazole, this compound may be prepared by the general method described in Chem. Ber., 1957, 90, 182.

Reagents which may provide the anion ⁻R^{c} include Grignard reagents R^{c}MgHal (where Hal = halogen); organocuprate reagents such as LiR^{c}₂Cu; organolithium reagents R^{c}Li; or compounds which stabilise the anion by means of an adjacent activating group such as an ester or enolisable ketone function. In this case, the adjacent ester or ketone function may be retained after the process is complete, or may be removed. For example, an ester moiety may be hydrolysed and decarboxylated.

In a further process, the compounds according to the invention may be prepared by a method which comprises reacting a compound of formula XVI: 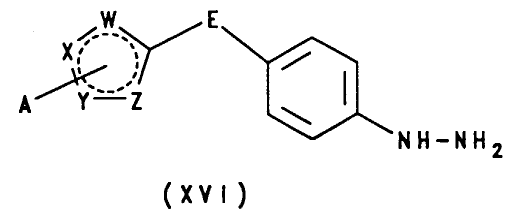 wherein W, X, Y, Z, A and E are as defined above; with a compound of formula VII as defined above, or a carbonyl-protected form thereof, e.g. the dimethyl acetal or ketal; followed, where required, by N-alkylation by standard methods to introduce the moiety R³.

As with that between compounds VI and VII, the reaction between compounds XVI and VII may be carried out in a single step (Fischer indole synthesis) or by an initial non-cyclising step at a lower temperature to give a compound of formula XVII: 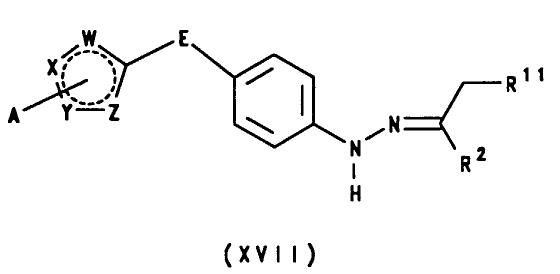 wherein W, X, Y, Z, A, E, R² and R¹¹ are as defined above; followed by cyclisation using a suitable reagent, e.g. a polyphosphate ester.

The hydrazines of formula XVI may be prepared from the corresponding anilines of formula XVIII: 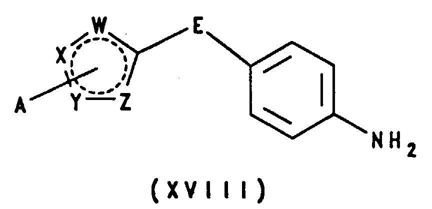 wherein W, X, Y, Z, A and E are as defined above; by methods analogous to those described above with reference to the compounds of formula IX.

The anilines of formula XVIII may be prepared from those of formula IX above by appropriate modification of the moiety Q using, for example, methods analogous to those described above with reference to the compounds of formulae III and IV. Thus, for example, when Q in the compounds of formula IX represents a group of formula -C(NOH)NH₂ or -CONHNH₂, the compounds of formula XVIII may be prepared therefrom by reaction with a reactive derivative of a carboxylic acid of formula A-CO₂H, where A is as defined previously. Alternatively, when Q in the compounds of formula IX represents a reactive carboxylate moiety, the compounds of formula XVIII may be prepared therefrom by reaction with a compound of formula A-C(NOH)NH₂ or A-CONHNH₂.

It will be appreciated that any compound of formula I initially obtained from any of the above processes may, where appropriate, subsequently be elaborated into a further compound of formula I by techniques known from the art. In particular, a compound of formula I wherein R³ is hydrogen initially obtained may be converted into a compound of formula I wherein R³ represents C₁₋₆ alkyl by standard alkylation techniques, for example by treatment with an alkyl iodide, e.g. methyl iodide, typically under basic conditions, e.g. sodium hydride in dimethylformamide. Similarly, a compound of formula I wherein R¹ represents aminoethyl initially obtained may be converted into a compound of formula I wherein R¹ represents N-methylaminoethyl or N,N-dimethylaminoethyl by conventional N-alkylation techniques, e.g. by treatment with the appropriate aldehyde in the presence of a reducing agent such as sodium cyanoborohydride.

Where the above-described processes for the preparation of the compounds according to the invention give rise to mixtures of stereoisomers, these isomers may be separated by conventional techniques such as preparative chromatography.

The novel compounds may be prepared in racemic form, or individual enantiomers may be prepared either by enantiospecific synthesis or by resolution. The novel compounds may, for example, be resolved into their component enantiomers by standard techniques, such as the formation of diastereomeric pairs by salt formation with an optically active acid, such as (-)-di-p-toluoyl-d-tartaric acid and/or (+)-di-p-toluoyl-1-tartaric acid followed by fractional crystallization and regeneration of the free base. The novel compounds may also be resolved by formation of diastereomeric esters or amides, followed by chromatographic separation and removal of the chiral auxiliary.

During any of the above synthetic sequences it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press, 1973; and T.W. Greene, Protective Groups in Organic Synthesis, John Wiley & Sons, 1981. The protecting groups may be removed at a convenient subsequent stage using methods known from the art.

The following Examples illustrate the preparation of compounds according to the invention.

The ability of test compounds to bind to 5-HT₁-like receptors was measured in membranes prepared from pig caudate using the procedure described in J. Neurosci., 1987, 7, 894. Binding was determined using 2 nM 5-hydroxytryptamine creatinine sulphate, 5-[1,2-³H(N)] as a radioligand. Cyanopindolol (100 nM) and mesulergine (100 nM) were included in the assay to block out 5-HT_{1A} and 5-HT_{1C} binding sites respectively. The concentration of the compounds of the accompanying Examples required to displace 50% of the specific binding (IC₅₀) is below 1 µM in each case.

The activity of test compounds as agonists of the 5-HT₁-like receptor was measured in terms of their ability to mediate contraction of the saphenous vein of New Zealand White rabbits, using the procedure described in Arch. Pharm., 1990, 342, 111. Agonist potencies were calculated as -log₁₀EC₅₀ (pEC₅₀) values, from plots of percentage 5-HT (1 µm) response against the concentration of the agonist. The compounds of accompanying Examples 1, 4, 6, 19-21, 27, 28, 30, 39-42, 44, 45, 49, 52, 53, 56, 61, 65, 88, 93 and 110 were tested and were found to possess pEC₅₀ values in this assay of not less than 5.0 in each case.

### EXAMPLE 1

### 2-[5-(5-(3-Benzyl-1,2,4-oxadiazol)-yl)-1H-indol-3-yl]ethylamine Hydrogen Oxalate Hydrate

### 1. Ethyl-p-hydrazinobenzoate. Hydrochloride.

A solution of sodium nitrite (17.0g, 0.24mol) in water (90ml) was added to a cooled solution of ethyl-p-amino benzoate (40g, 0.24mol) in concentrated hydrochloric acid (225ml) at such a rate that the temperature did not exceed 0°C. The mixture was stirred at 0°C for 0.1h before adding to a stirred solution of tin (II) chloride dihydrate (202g, 0.89mol) in concentrated hydrochloric acid (135ml) at such a rate that the temperature did not exceed -5°C. The resulting suspension was allowed to warm to room temperature over a 1h period, filtered and washed with ether, mp 215-217°C, δ (360MHz, D₂O) 1.38 (3H, t, J = 7.1Hz, Me), 4.37 (2H, q, J = 7.1Hz, CH₂), 7.06 (1H, d, J = 9Hz, aromatic-H), 8.03 (1H, d, J = 9Hz, aromatic-H).

### 2. 2-(5-Carboethoxy-1H-indol-3-yl)ethylamine. Hydrogen Maleate.

A solution of ethyl-p-hydrazinobenzoate hydrochloride (10g, 46mmol) and 4-chlorobutanal dimethyl acetal (7.8g, 46mmol) in ethanol/water (5:1, 500ml) was heated at reflux for 2h. The solvent was removed under vacuum and the residue chromatographed through silica-gel eluting with dichloromethane/ethanol/ammonia (40:8:1) to give the title-indole as an oil (3.69g). The hydrogen maleate salt was prepared, mp 127°C; (Found: C, 59.46; H, 5.96; N, 8.47. C₁₃H₁₆N₂O₂.C₄H₄O₄ requires C, 59.68; H, 5.93; N, 8.54%), m/e 232 (M⁺), δ (360MHz, D₂O) 1.43 (3H, t, J = 7.1Hz, Me); 3.21 (2H, t, J = 7.0Hz, CH₂); 3.37 (2H, t, J = 7.0Hz, CH₂); 4.42 (2H, q, J = 7.1Hz, CH₂); 6.23 (2H, s, maleate-H); 7.40 (1H, s, indole-H); 7.56 (1H, d, J = 8.8Hz, aromatic-H); 7.88 (1H, dd, J = 1.6 and 8.8Hz, aromatic-H); 8.38 (1H, d, J = 1.6Hz, aromatic-H).

### 3. 2-[5-(5-(3-Benzyl-1,2,4-oxadiazol)yl)-1H-indol-3-yl]ethylamine. Hydrogen Oxalate Hydrate.

Sodium hydride (0.33g of an 80% dispersion in oil, 11.0mmol) was added to a stirred solution of phenyl acetamide oxime (1.74g, 11.6mmol) in anhydrous THF (50ml) and the reaction mixture heated at reflux for 0.5h. 2-(5-Carboethoxy-1H-indol-3-yl)ethylamine (1.19g, 5.0mmol) in THF (10ml) was added and the reaction heated under reflux for 2h. The mixture was allowed to cool to room temperature before adding water (20ml) and extracting with dichloromethane (3 x 100ml). The crude product remaining after removal of solvent under vacuum was chromatographed through silica-gel eluting with dichloromethane/ethanol/ammonia (40:8:1) to give the title-product (0.68g). The hydrogen oxalate salt was prepared, mp 229°C; (Found: C, 59.42; H, 4.92: N, 13.02. C₁₉H₁₈N₄O. C₂H₂O₄. 0.85H₂O requires C, 59.53; H, 5.16; N, 13.22%); δ (360MHz, D₂O) 3.18 (2H, t, J = 7.4Hz, CH₂); 3.31 (2H, t, J = 7.4Hz, CH₂); 4.17 (2H, s, CH₂-Ph); 7.35-7.43 (6H, m, indole-H and aromatics); 7.63 (1H, d, J = 8.6Hz, aromatic-H); 7.87 (1H, d, J = 8.6Hz, aromatic-H); 8.40 (1H, s, aromatic-H).

### EXAMPLE 2

### 2-[5-(5-(3-Methyl-1,2,4-oxadiazol)yl)-1H-indol-3-yl]ethylamine. Hydrogen Oxalate.

This was prepared from methyl acetamide oxime and 2-(5-carboethoxy-1H-indol-3-yl)ethylamine as described for Example 1. The hydrogen oxalate salt was prepared mp 230°C. (Found: C, 52.91; H, 4.85; N, 16.01. C₁₃H₁₄N₄O. 1.2(C₂H₂O₄) requires C, 52.78; H, 5.02; N, 16.41%); m/e 243 (M+N)⁺; δ (360MHz, D₂O) 2.26 (3H, s, Me); 3.09 (2H, t, J = 7.3Hz, CH₂); 3.32 (2H, t, J = 7.3Hz, CH₂); 7.28 (1H, s, indole-H); 7.41 (1H, d, J = 8.6Hz, aromatic-H); 7.53 (1H, dd, J = 1.6 and 8.6Hz, aromatic-H); 7.86 (1H, d, J = 1.6Hz, aromatic-H).

### EXAMPLE 3

### N,N-Dimethyl-2-[5-(5-(3-benzyl-1,2,4-oxadiazol)yl)-1H-indol-3-yl]ethylamine. Sesquioxalate.

### 1. N,N-Dimethyl-2-(5-carboethoxy-1H-indol-3-yl)ethylamine. Oxalate

Solutions of sodium borohydride (1.1g, 2.2mmol) in water (15ml) and formaldehyde (7.5ml) in methanol (7.5ml) were added dropwise, simultaneously, over a 0.25h period, to a solution of 2-(5-carboethoxy-1H-indol-3-yl)ethylamine (0.75g, 4.3mmol) in methanol (15ml), at room temperature. The mixture was stirred for 0.25h before adding concentrated hydrochloric acid (10ml) and concentrating in vacuo. A second portion of c.HCl was added (7.5ml) and the solution then basified with potassium carbonate (6.1g). Extraction into ethyl acetate and chromatography of the crude residue through silica-gel eluting with dichloromethane/ethanol/ammonia (60:8:1) gave the title-N,N-dimethyl amine (0.64g). The oxalate salt was prepared, mp 150°C; (Found C, 52.76; H, 5.67; N, 6.65. C₁₅H₂₀N₂O₂.1.8. C₂H₂O₄ requires C, 52.89; H, 5.63; N, 6.63%); δ (360MHz, D₂O) 1.42 (3H, t, J = 7.1Hz, Me); 2.94 (6H, s, N(Me)₂); 3.27 (2H, t, J = 7.0Hz, CH₂); 3.52 (2H, t, J = 7.0Hz, CH₂); 4.42 (2H, q, J = 7.1Hz, CH₂); 7.40 (1H, s, indole-H); 7.56 (1H, d, J = 8.6Hz, aromatic-H); 7.88 (1H, dd, J = 1.6 and 8.6Hz, aromatic-H); 8.36 (1H, d, J = 1.6Hz, aromatic-H).

### 2. N,N-Dimethyl-2-[5-(5-(3-benzyl-1,2,4-oxadiazol)yl)-1H-indol-3-yl]ethylamine. Sesquioxalate.

The title-compound was prepared from phenyl acetamide oxime and N,N-dimethyl-2-(5-carboethoxy-1H-indol-3-yl)ethylamine as described for Example 1. The sesquioxalate salt was prepared, mp 157-158°C; (Found: C, 59.14; H, 5.29; N, 11.35. C₂₁H₂₂N₄O. 1.6. C₂H₂O₄ requires C, 59.26; H, 5.19; N, 11.42%); m/e 347 (M+H)⁺; δ (360MHz, D₂O) 2.88 (6H, s, N(Me)₂); 3.02 (2H, br t, J = 7.3Hz, CH₂); 3.32 (2H, br t, J = 7.3Hz, CH₂); 3.99 (2H, s, CH₂-phenyl); 7.13 (1H, s, indole-H); 7.34-7.49 (7H, m, aromatics); 7.82 (1H, s, aromatic-H).

### EXAMPLE 4

### 2-[5-(5-(3-Benzyl-1,2,4-oxadiazol)yl)methyl)-1H-indol-3-yl]ethylamine. Hydrogen Oxalate.

### 1. Ethyl-p-hydrazinophenylacetate. Hydrochloride.

This was prepared from ethyl-p-amino-phenyl acetate as described for Example 1, mp 188-190°C, δ (360MHz, D₆-DMSO) 1.44 (3H, t, J = 7.1Hz, Me); 3.88 (2H, s, CH₂); 4.36 (2H, t, J = 7.1Hz, CH₂); 7.20 (2H, d, J = 8.5Hz, aromatics); 7.50 (2H, d, J = 8.5Hz, aromatics).

### 2. 2-(5-Carboethoxymethyl-1-H-indol-3-yl)ethylamine. Hydrogen Maleate.

The title-compound was prepared from ethyl-p-hydrazinophenylacetate and 4-chlorobutanal dimethyl acetal as described for Example 1. The hydrogen maleate salt was prepared, mp 105-108°C; (Found: C, 59.31; H, 6.07; N, 7.43. C₁₄H₁₈N₂O₂.C₄H₄O₄.0.1H₂O requires C, 59.36; H, 6.14; N, 7.69%); m/e 246 (M⁺); δ (360MHz, D₂O) 1.23 (3H, t, J = 7.1Hz, Me); 3.16 (2H, t, J = 7.0Hz, CH₂); 3.33 (2H, t, J = 7.0Hz, CH₂); 3.82 (2H, s, CH₂); 4.18 (2H, q, J = 7.1Hz, CH₂Me); 6.29 (2H, s, maleate-H); 7.17 (1H, dd, J = 1.6 and 8.4Hz, aromatic-H); 7.32 (1H, s, indole-H); 7.49 (1H, d, J = 8.4Hz, aromatic-H); 7.56 (1H, s, aromatic-H).

### 3. 2-[5-(5-(3-Benzyl-1,2,4-oxadiazol)-ylmethyl)-1H-indol-3-yl]ethylamine. Hydrogen Oxalate

This was prepared from the preceding ester and phenylacetamide oxime as described for Example 1. The hydrogen oxalate salt was prepared, mp 176-178°C (isopropyl alcohol); (Found: C, 62.37; H, 5.34; N, 13.15. C₂₀H₂₀N₄O. C₂H₂O₄ requires C, 62.55; H, 5.25; N, 13.26%); m/e 333 (M+N)⁺; δ (360MHz, D₂O) 3.10 (2H, t, J = 6.9Hz, CH₂); 3.28 (2H, t, J = 6.9Hz, CH₂); 4.01 (2H, s, CH₂); 4.29 (2H, s, CH₂); 7.11 (1H, dd, J = 1.6 and 8.4Hz, aromatic-H); 7.25-7.38 (6H, m, 5 x aromatic-H and 1 x indole-H); 7.44 (1H, d, J = 8.4Hz, aromatic-H); 7.53 (1H, d, J = 1.6Hz, aromatic-H).

### EXAMPLE 5

### 2-[5-(5-(3-Methyl-1,2,4-oxadiazol)ylmethyl)-1H-indol-3-yl]ethylamine. Hydrogen Oxalate Hydrate.

Prepared from methyl acetamide oxime and 2-(5-carboethoxymethyl-1H-indol-3-yl)ethylamine as described for Example 1. The hydrogen oxalate salt was prepared, mp 72-74°C; (Found: C, 53.45; H, 5.26; N, 15.15. C₁₄H₁₆N₄O. C₂H₂O₄.0.75H₂O requires C, 53.40; H, 5.46; N, 15.56%); δ (360MHz, D₂O) 2.32 (3H, s, Me); 3.15 (2H, t, J = 7.1Hz, CH₂); 3.33 (2H, t, J = 7.1Hz, CH₂); 4.37 (2H, s, CH₂); 7.20 (1H, dd, J = 1.6 and 8.4Hz, aromatic-H); 7.32 (1H, s, indole-H); 7.51 (1H, d, J = 8.4Hz, aromatic-H); 7.62 (1H, s, aromatic-H).

### EXAMPLE 6

### 2-[5-(5-(3-Amino-1,2,4-oxadiazol)ylmethyl-1H-indol-3-yl]ethylamine. Hydrogen Oxalate.

Hydroxyguanidine sulphate (2.76g, 10.4mmol) was added to a stirred solution of sodium (0.91g, 39mmol) in ethanol (40ml). The mixture was stirred for 0.5h before adding a solution of 2-(5-carboethoxymethyl-1H-indol-3-yl)ethylamine (0.85g, 3.5mmol) in ethanol (20ml) and refluxing for 2h. After cooling to room temperature the ethanol was removed under vacuum and the residue chromatographed through silica-gel eluting with dichloromethane/ethanol/ammonia (40:8:1) to give the title-product. The hydrogen oxalate salt was prepared, mp 85-87°C; (Found: C , 47.07; H, 5.28; N, 20.71. C₁₃H₁₅N₅O.C₂H₂O₄.1.2H₂O.0.3 (C₁H₅N₃O) requires C, 46.73; H, 5,41; N, 21.01%); m/e 257 (M⁺); δ (360MHz, D₂O) 3.14 (2H, t, J = 7.0Hz, CH₂); 3.32 (2H, t, J = 7.0Hz, CH₂); 4.23 (2H, s, CH₂); 7.17 (1H, dd, J = 1.6 and 8.4Hz, aromatic-H); 7.32 (1H, s, indole-H); 7.49 (1H, d, J = 8.4Hz, aromatic-H); 7.58 (1H, d, J = 1.6Hz, aromatic-H).

### EXAMPLE 7

### 2-[5-(5-(3-Phenyl-1,2,4-oxadiazol)ylmethyl)-1H-indol-3-yl]ethylamine. Hydrogen Oxalate.

The title-compound was prepared from phenyl amide oxime and 2-(5-carboethoxymethyl-1H-indol-3-yl)ethylamine as described for Example 1. The hydrogen oxalate salt was prepared, mp 82-84°C; (Found: C, 61.57; H, 5.12; N, 13.03. C₁₉H₁₈N₄O.C₂H₂O₄.0.3 C₂H₅OH requires C, 61.44; H, 5.20; N, 13.27%); m/e 318 (M⁺); δ (360MHz, D₂O) 3.13 (2H, t, J = 7.0Hz, CH₂); 3.31 (2H, t, J = 7.0Hz); 4.44 (2H, s, CH₂); 7.23 (1H, d, J = 7.6Hz, aromatic-H); 7.30 (1H, s, indole-H); 7.49-7.60 (4H, m, aromatic-Hs); 7.65 (1H, s, aromatic-H); 7.90 (2H, d, J = 7.6Hz, aromatic Hs).

### EXAMPLE 8

### 2-[5-(5-(3-[2-Methoxybenzyl]-1,2,4-oxadiazol)ylmethyl)-1H-indol-3-yl]ethylamine. Hydrogen Oxalate.

Prepared from 2-methoxybenzyl amide oxime and 2-(5-carboethoxymethyl-1H-indol-3-yl)ethylamine as described for Example 1. The hydrogen oxalate salt was prepared, mp 68-70°C; (Found: C, 59.73; H, 5.33; N, 11.97. C₂₁H₂₂N₄O₂.1.2 C₂H₂O₄ requires C, 59.74; H, 5.23; N, 11.91%); m/e 363 (M+N)⁺; δ (360MHz, D₂O) 3.10 (2H, t, J = 7.0Hz, CH₂); 3.29 (2H, t, J = 7.0Hz, CH₂); 3.68 (3H, s, OMe); 3.99 (2H, s, CH₂); 4.31 (2H, s, CH₂); 6.96-7.01 (2H, m, aromatic-Hs); 7.12 (1H, dd, J = 1.6 and 8.4Hz, aromatic-H); 7.23 (1H, d, J = 8.4Hz, aromatic-H); 7.29 (1H, s, indole-H); 7.29-7.35 (1H, m, aromatic-H); 7.45 (1H, d, J = 8.4Hz, aromatic-H); 7.56 (1H, s, aromatic-H).

### EXAMPLE 9

### N,N-Dimethyl-2-[5-(5-(3-benzyl-1,2,4-oxadiazol)ylmethyl)-1H-indol-3-yl]ethylamine. Hydrogen Oxalate.

### 1. N,N-Dimethyl-2-(5-carboethoxymethyl-1H-indol-3-yl)ethylamine.

Prepared as described for Example 3. δ (360MHz, CDCl₃) 1.26 (3H, t, J = 7.0Hz, Me); 2.36 (6H, s, N(Me)₂); 2.62 (2H, t, J = 7.0Hz, CH₂); 2.92 (2H, t, J = 7.0Hz, CH₂); 3.70 (2H, s, CH₂); 4.16 (2H, q, J = 7.0Hz, CH₂-Me); 6.98 (1H, br s, indole-H); 7.10 (1H, dd, J = 1.6 and 8.6Hz, aromatic-H); 7.28 (1H, d, J = 8.6Hz, aromatic-H); 7.48 (1H, s, aromatic-H).

### 2. N,N-Dimethyl-2-[5-(5-(3-benzyl-1,2,4-oxadiazol)ylmethyl)-1H-indol-3-yl]ethylamine. Hydrogen Oxalate.

The title-compound was prepared from phenylacetamide oxime and the preceding ester as described for Example 1. The hydrogen oxalate salt was prepared, mp 174-176°C (isopropyl alcohol); (Found: C, 63.79; H, 5.91; N, 12.31. C₂₂H₂₄N₄O. C₂H₂O₄ requires C, 63.99; H, 5.82; N, 12.44%); m/e 361 (M+H)⁺; δ (250MHz, D₂O) 2.88 (6H, s, N(Me)₂); 3.16 (2H, t, J = 7.3Hz, CH₂); 3.41 (2H, t, J = 7.3Hz, CH₂); 4.06 (2H, s, CH₂); 4.35 (2H, s, CH₂); 7.15 (1H, dd, J = 1.6 and 8.4Hz, aromatic-H); 7.29-7.40 (6H, m, 1 x indole-H and 5 x aromatics); 7.46 (1H, d, J = 8.5Hz, aromatic-H); 7.58 (1H, br s, aromatic-H).

### EXAMPLE 10

### N,N-Dimethyl-2-[5-(5-(3-Methyl-1,2,4-oxadiazol)ylmethyl)-1H-indol-3-yl]ethylamine. Sesquioxalate.

Prepared from methylacetamide oxime and N,N-dimethyl-2-(5-carboethoxymethyl-1H-indol-3-yl)ethyl amine as described for Example 1. The sesquioxalate salt was prepared, mp 159-160°C (isopropyl alcohol); (Found: C, 54.03; H, 5.61; N, 13.31. C₁₆H₂₀N₄.1.5 (C₂H₂O₄).0.1H₂O requires C, 54.17; H, 5.55; N, 13.30%); δ (360MHz, D₂O) 2.32 (3H, s, Me); 2.91 (6H, s, N(Me)₂); 3.09 (1H, t, J = 7.4Hz, CH₂); 3.21 (1H, t, J = 7.4Hz, CH₂); 4.36 (2H, s, CH₂); 7.19 (1H, dd, J = 1.6 and 8.4Hz, aromatic-H); 7.34 (1H, s, indole-H); 7.50 (1H, d, J = 8.4Hz, aromatic-H); 7.61 (1H, s, aromatic-H).

### EXAMPLE 11

### 2-[5-(2-(5-[3-Benzyl-1,2,4-oxadiazol]yl)ethyl)-1H-indol-3-yl]ethylamine. Maleate

### 1. 2-[5-(2-(Carboethoxy)ethyl)-1H-indol-3-yl]ethylamine. Hydrogen Maleate

Prepared from ethyl-p-hydrazinophenylpropionate and 4-chlorobutanal dimethylacetal as described for Example 1. The hydrogen maleate salt was prepared, mp 114-116°C (isopropyl alcohol); (Found: C, 60.67; H, 6.49; N, 7.43. C₁₅H₂₀N₂O₂. C₄H₄O₄ requires C, 60.63; H, 6.43; N, 7.44%); m/e 260 (M⁺); δ (360MHz, D₂O) 1.15 (3H, t, J = 7.2Hz, Me); 2.75 (2H, t, J = 7.4Hz, CH₂); 3.06 (2H, t, J = 7.3Hz, CH₂); 3.15 (2H, t, J = 7.3Hz, CH₂); 3.32 (2H, t, J = 7.4Hz, CH₂); 4.08 (2H, q, J = 7.2Hz, CH₂); 6.29 (2H, s, maleate-Hs); 7.14 (1H, dd, J = 1 and 8.4Hz, aromatic-H); 7.29 (1H, s, indole-H); 7.46 (1H, d, J = 8.4Hz, aromatic-H); 7.50 (1H, s, aromatic-H).

### 2. 2-[5-(2-(5-[3-Benzyl-1,2,4-oxadiazol]yl)ethyl)-1H-indol-3-yl]ethylamine. Maleate.

Prepared from the preceding tryptamine and phenylacetamide oxime as described for Example 1. The maleate salt was prepared, mp 113-114°C (isopropylalcohol/ether); (Found: C, 68.40; H, 6.06; N, 13.84. C₂₁H₂₂N₄O. C₂H₂O₂ requires C, 68.30; H, 5.98; N, 13.85%); m/e 346 (M⁺); δ (360MHz, D₂O) 3.05 (2H, t, J = 7.0Hz, CH₂); 3.21 (4H, t, J = 6.8Hz, 2 of CH₂); 4.00 (2H, s, CH₂); 6.01 (1H, s, maleate-H); 6.98 (1H, dd, J = 1.6 and 8.3Hz, aromatic-H); 7.06 (2H, d, J = 6.7Hz, indole-H and aromatic-H); 7.22-7.37 (6H, m, aromatic-Hs).

### EXAMPLE 12

### 2-[5-(3-(5-[3-Benzyl-1,2,4-oxadiazol]yl)propyl)-1H-indol-3-yl]ethylamine. Oxalate

### 1. 2-[5-Carboethoxyprop-3-yl-1H-indol-3-yl]ethylamine

Prepared from ethyl-p-hydrazinophenylbutyrate and 4-chlorobutanal dimethyl acetal as described for Example 1; δ (250MHz, CDCl₃) 1.24 (3H, t, J = 7.2Hz, Me); 1.94-2.06 (2H, m, CH₂); 2.34 (2H, t, J = 7.4Hz, CH₂); 2.76 (2H, t, J = 7.4Hz, CH₂); 2.90 (2H, t, J = 7.3Hz, CH₂); 3.03 (2H, t, J = 7.3Hz, CH₂); 4.12 (2H, q, J = 7.2Hz, CH₂); 7.01 (1H, s, indole-H); 7.02 (1H, dd, J = 1.0 and 8.4Hz, aromatic-H); 7.28 (1H, d, J = 8.4Hz, aromatic-H); 7.40 (1H,s,aromatic-H); 8.00(1H, br s, NH).

### 2. 2-[5-(3-(5-[3-Benzyl-1,2,4-oxadiazol]yl)propyl)-1H-indol-3-yl]ethylamine. Oxalate

The title compound was prepared from 2-[5-carboethoxyprop-3-yl-1H-indol-3-yl]ethylamine and phenyl acetamide oxime using the general procedure. The oxalate salt was prepared, mp 188-189°C; (Found: C, 68.32; H, 6.30; N, 13.76. C₂₂H₁₄N₄O. 0.5(C₂H₂O₄) requires C, 68.13; H, 6.22; N, 13.82%); δ (360MHz, D₆-DMSO) 1.98-2.07 (2H, m, CH₂); 2.70 (2H, t, J = 7.3Hz, CH₂); 2.83-2.96 (6H, m, 3 of CH₂); 4.05 (2H, s, CH₂); 6.91 (1H, d, J = 8.3Hz, aromatic-H); 7.14 (1H, s, indole-H); 7.22-7.34 (7H, m, aromatic-H's).

### EXAMPLE 13

### 2-[5-(3-(5-[3-Methyl-1,2,4-oxadiazol]yl)propyl)-1H-indol-3-yl]ethylamine. Hydrogen Maleate

Prepared from 2-[5-carboethoxyprop-3-yl-1H-indol-3-yl]ethylamine and acetamide oxime using the general procedure. The hydrogen maleate salt: mp 136-137°C (isopropylalcohol/ether); (Found: C, 60.33; H, 6.14; N, 14.35. C₁₆H₂₀N₄O. 0.9 (C₄H₄O₄) requires C, 60.54; H, 6.12; N, 14.41%); m/e 284 (M⁺); δ (360MHz, D₂O) 2.14 (3H, s, Me); 2.80 (2H, t, J = 7.05Hz, CH₂); 2.87 (2H, t, J = 7.05Hz, CH₂); 3.13 (2H, t, J = 7.1Hz, CH₂); 3.34 (2H, t, J = 7.1Hz, CH₂); 7.05 (1H, dd, J = 1.5 and 8.4Hz, aromatic-H); 7.27 (1H, s, indole-H); 7.38 (1H, s, aromatic-H); 7.39 (1H, d, J = 8.4Hz, aromatic-H).

### EXAMPLE 14

### 2-[5-(3-(5-[3-Cyclopropyl-1,2,4-oxadiazol]yl)propyl)-1H-indol-3-yl]ethylamine. Hydrogen Maleate

Prepared from 2-[5-carboethoxyprop-3-yl-1H-indol-3-yl]ethylamine and cyclopropyl amide oxime as described for Example 1. The hydrogen maleate salt was prepared, mp 130-132°C; (Found: C, 61.36; H, 6.15; N, 12.90. C₁₈H₂₂N₄O. 0.25H₂O requires C, 61.31; H, 6.19; N, 13.00%); m/e 310 (M⁺); δ (360MHz, D₆-DMSO) 0.83-0.88 (2H, m, CH₂); 1.00-1.06 (2H, m, CH₂); 1.98-2.11 (3H, m, CH and CH₂); 2.71 (2H, t, J = 7.6Hz, CH₂); 2.90 (2H, t, J = 7.6Hz, CH₂); 3.00 (2H, t, J = 7.13Hz, CH₂); 3.08 (2H, t, J = 7.13Hz, CH₂); 6.95 (1H, dd, J = 1.4 and 8.2Hz, aromatic-H); 7.19 (1H, d, J = 1.4Hz, aromatic-H); 7.29 (1H, d, J = 8.2Hz, aromatic-H); 7.33 (1H, s, indole-H); 7.70 (1H, br s, NH).

### EXAMPLE 15

### 2-[5-(5-(3-Phenyl-1,2,4-oxadiazol)yl)-1H-indol-3-yl]ethylamine. Hydrogen Oxalate

Prepared from 2-(5-carboethoxy-1H-indol-3-yl)ethylamine and phenyl amide oxime using the general procedure. The hydrogen oxalate salt was prepared, mp 212-213°C (methanol); (Found: C, 61.90; H, 4.97; N, 14.64. C₁₈H₁₆N₄O. 0.85 (C₂H₂O₄) requires C, 62.12; H, 4.68; N, 14.71%); δ (250MHz, CDCl₃, free base) 3.00 (2H, t, J = 7.4Hz, CH₂); 3.10 (2H, t, J = 7.4Hz, CH₂); 7.16 (1H, s, indole-H); 7.46-7.54 (5H, m, aromatic-H); 8.05 (1H, dd, J = 1.8 and 8.4Hz, aromatic-H); 8.18-8.22 (2H, m, aromatic-H); 8.18 (1H, br s, NH); 8.54 (1H, s, aromatic-H).

Examples 16-26 were prepared from 2-(5-carboethoxy-1H-indol-3-yl)ethylamine and the appropriate amide oxime using the procedure described for Example 1, unless otherwise stated.

### EXAMPLE 16

### 2-[5-(5-(3-Diphenylmethyl-1,2,4-oxadiazol)yl)-1H-indol-3-yl]ethylamine. Sesquioxalate.

The crude product was chromatographed through silica gel eluting with dichloromethane/ethanol/ammonia (60:8:1). The sesquioxalate salt was prepared, mp 117-118°C; (Found: C, 63.22; H, 5.40; N, 9.90. C₂₅H₂₂N₄O. 1.4 (C₂H₄O₂). 0.7. C₂H₅OH requires C, 63.44; H, 5.29; N, 10.14%); δ (360MHz, D₆-DMSO) 3.06 (4H, br s, 2 of CH₂); 5.81 (1H, s, CH); 7.26-7.43 (11H, m, aromatic-H's); 7.57 (1H, d, J = 8.5Hz, aromatic-H); 7.83 (1H, dd, J = 1.4 and 8.5Hz, aromatic-H); 7.95 (1H, br s, NH); 8.37 (1H, s, aromatic-H).

### EXAMPLE 17

### 2-[5-(5-(3-(2-Methoxybenzyl)-1,2,4-oxadiazol)yl)-1H-indol-3-yl]ethylamine. Oxalate

The oxalate salt was prepared, mp 244-245°C (isopropyl alcohol/ether); (Found: C, 63.45; H, 5.47; N, 13.97. C₂₀H₂₀N₄O₂. 0.6 (C₂H₂O₄) requires C, 63.27; H, 5.31; N, 13.92%); m/e 349 (M⁺+1); δ (360MHz, CF₃CO₂D) 3.88 (2H, br s, CH₂); 4.31 (2H, br s, CH₂); 4.43 (3H, s, OMe); 4.93 (2H, s, CH₂); 7.35 (1H, br s, NH); 7.57 (2H, d, J = 7.5Hz, aromatic-H's); 7.85 (1H, d, J = 7.5Hz, aromatic-H); 7.92-7.96 (2H, m, aromatic-H's); 8.22 (1H, d, J = 8.8Hz, aromatic-H); 8.53 (1H, d, J = 8.8Hz, aromatic-H); 9.10 (1H, s, aromatic-H).

### EXAMPLE 18

### 2-[5-[5-(3-(3-Methoxybenzyl)-1,2,4-oxadiazol)yl]-1H-indol-3-yl]ethylamine. Hydrogen Maleate

Hydrogen maleate salt, mp 173-175°C (isopropyl alcohol/ether); (Found: C, 61.82; H, 5.33; N, 11.92. C₂₀H₂₀N₄O₂. C₄H₄O₄. 0.1H₂O requires C, 61.82; H, 5.23; N, 12.01%); δ (360MHz, D₆-DMSO) 3.07 (4H, br s, 2 of CH₂); 3.75 (3H, s, OMe); 4.12 (2H, s, CH₂); 6.85 (1H, dd, J = 2.2 and 8.6Hz, aromatic-H); 6.92 (1H, d, J = 7.3Hz, aromatic-H); 6.93 (1H, s, aromatic-H); 7.27 (1H, dd, J = 7.7 and 7.7Hz, aromatic-H); 7.42 (1H, d, J = 2.2Hz, aromatic-H); 7.56 (1H, d, J = 8.6Hz, aromatic-H); 7.71 (2H, br s, NH₂); 7.82 (1H, dd, J = 1.5 and 8.6Hz, aromatic-H); 8.37 (1H, s, aromatic-H); 11.48 (1H, s, NH).

### EXAMPLE 19

### 2-[5-[5-(3-(4-Methoxybenzyl)-1,2,4-oxadiazol)yl]-1H-indol-3-yl]ethylamine. Hydrogen Maleate

Hydrogen maleate salt, mp 195-196°C (isopropyl alcohol/ether); (Found: C, 61.95; H, 5.30; N, 11.99. C₂₀H₂₀N₄O₂. C₄H₄O₄ requires C, 62.06; H, 5.21; N, 12.06%); δ (360MHz, D₆-DMSO/D₂O) 3.15 (2H, t, J = 7.3Hz, CH₂); 3.32 (2H, t, J = 7.3Hz, CH₂); 3.80 (3H, s, Me); 4.04 (2H, s, CH₂); 6.98 (2H, d, J = 8.7Hz, aromatic-H's); 7.33 (2H, d, J = 8.7Hz, aromatic-H's); 7.35 (1H, s, aromatic-H); 7.55 (1H, d, J = 8.6Hz, aromatic-H); 7.76 (1H, dd, J = 1.6 and 8.6Hz, aromatic-H); 8.24 (1H, s, aromatic-H).

### EXAMPLE 20

### 2-[5-[5-(3-(4-Acetylaminobenzyl)-1,2,4-oxadiazol)yl]-1H-indol-3-yl]ethylamine. Bisoxalate.

Prepared as described for Example 6. The bis oxalate salt was prepared, mp 113-115°C; (Found: C, 53.67; H, 4.67; N, 13.46. C₂₁H₂₁N₅O₂. 2(C₂H₄O₂). 0.25 H₂O requires C, 53.62; H, 4.59; N, 13.51%); δ (360MHz, D₆-DMSO) 2.03 and 2.08 (total 3H, s, Me); 3.07 (4H, br s, 2 of CH₂); 3.92 and 4.08 (total 2H, s, CH₂); 6.53 and 7.27 (total 2H, d, J = 8.3Hz, aromatic-H's); 6.99 and 7.56 (total 2H, d, J = 8.4Hz, aromatic-H's); 7.41 (1H, s, aromatic-H); 7.53 (1H, d, J = 8.5Hz, aromatic-H); 7.80 (1H, dd, J = 1.5 and 8.5Hz, aromatic-H); 7.97 (2H, br s, NH₂); 8.35 (1H, s, aromatic-H); 9.94 (1H, s, NH); 11.54 (1H, s, NH).

### EXAMPLE 21

### 2-[5-[5-(3-(4-Methylsulphonylaminobenzyl)-1,2,4-oxadiazol)yl]-1H-indol-3-yl]ethylamine. Sesquioxalate

Prepared as described for Example 6. The sesquioxalate salt was prepared, mp 219-220°C; (Found: C, 50.91; H, 4.61; N, 12.59. C₂₀H₂₁N₅O₃. 1.5 (C₂H₂O₄) requires C, 50.55; H, 4.43; N, 12.81%); δ (360MHz, D₆-DMSO) 2.97 (3H, s, Me); 3.06 (4H, br s, 2 of CH₂); 4.10 (2H, s, CH₂); 7.18 (2H, d, J = 8.4Hz, aromatic-H's); 7.32 (2H, d, J = 8.4Hz, aromatic-H's); 7.42 (1H, s, aromatic-H); 7.55 (1H, d, J = 8.6Hz, aromatic-H); 8.36 (1H, s, aromatic-H); 9.70 (1H, br s, NH); 11.50 (1H, s, NH).

### EXAMPLE 22

### 2-[5-[5-(3-Phenethyl-1,2,4-oxadiazol)yl]-1H-indol-3-yl]ethylamine. Sesquioxalate

The sesquioxalate salt, mp 144-146°C; (Found: C, 58.05; H, 5.00; N, 11.67. C₂₀H₂₀N₄O. 1.6 (C₂H₂O₄). 0.2 H₂O requires C, 57.90; H, 5.20; N, 11.53%); m/e 333 (M⁺+1); δ (360MHz, CD₃OD) 3.04-3.20 (4H, m, 2 of CH₂); 3.26-3.33 (4H, m, 2 of CH₂); 7.15-7.28 (5H, m, aromatic-H's); 7.33 (1H, s, aromatic-H); 7.55 (1H, d, J = 8.49Hz, aromatic-H); 7.90 (1H, dd, J = 1.6 and 8.49Hz, aromatic-H); 8.42 (1H, s, aromatic-H).

### EXAMPLE 23

### 2-[5-[5-(3-Phenpropyl-1,2,4-oxadiazol)yl]-1H-indol-3-yl]ethylamine. Hydrogen Maleate

The hydrogen maleate salt, mp 150-151°C (isopropyl alcohol/ether); (Found: C, 63.65; H, 5.64; N, 11.87. C₂₁H₂₂N₄O. 1.17 (C₄H₄O₄) requires C, 63.93; H, 5.57; N, 11.60%); δ (360MHz, D₆-DMSO/D₂O) 2.02-2.18 (2H, m, CH₂); 2.65-2.84 (4H, m, 2 of CH₂); 3.14-3.24 (2H, m, CH₂); 3.28-3.40 (2H, m, CH₂); 7.16-7.44 (6H, m, aromatic-H); 7.56-7.68 (1H, m, aromatic-H); 7.74-7.86 (1H, m, aromatic-H); 8.24-8.35 (1H, m, aromatic-H).

### EXAMPLE 24

### 2-[5-[5-(3-Cyclopropyl-1,2,4-oxadiazol)yl]-1H-indol-3-yl]ethylamine. Hemisuccinate

The hemisuccinate salt was prepared, mp 205-207°C (isopropylalcohol/ether); (Found: C, 61.89; H, 5.91; N, 16.88. C₁₅H₁₆N₃O. 0.5 (C₄H₆O₄). 0.15 H₂O requires C, 61.86; H, 5.89; N, 16.97%); δ (360MHz, D₆-DMSO) 0.98-1.05 (2H, m, CH₂); 1.07-1.13 (2H, m, CH₂); 2.13-2.20 (1H, m, CH); 2.95 (4H, m, 2 of CH₂); 7.36 (1H, s, aromatic-H); 7.53 (1H, d, J = 8.6Hz, aromatic-H); 7.77 (1H, dd, J = 1.5 and 8.6Hz, aromatic-H); 8.30 (1H, s, aromatic-H); 11.39 (1H, br s, NH).

### EXAMPLE 25

### 2-[5-[5-(3-Ethyl-1,2,4-oxadiazol)yl]-1H-indol-3-yl]ethylamine. Bisoxalate Hemihydrate

The bisoxalate hemihydrate salt, mp 195-197°C; (Found: C, 48.34; H, 4.71; N, 12.41. C₁₄H₁₆N₄O. 2 (C₂H₂O₄). 0.5 H₂O requires C, 48.54; H, 4.75; N, 12.57%); m/e 257 (M⁺+1); δ (360MHz, D₆-DMSO) 1.30 (3H, t, J = 7.6Hz, Me); 2.78 (2H, q, J = 7.6Hz, CH₂); 3.08 (4H, br s, 2 of CH₂); 7.43 (1H, d, J = 1.8Hz, aromatic-H); 7.57 (1H, d, J = 8.5Hz, aromatic-H); 7.82 (1H, dd, J = 1.8 and 8.5Hz, aromatic-H); 7.96 (2H, br s, NH₂); 8.38 (1H, s, aromatic-H).

### EXAMPLE 26

### 2-[5-[5-(3-(4-Trifluoromethylbenzyl)-1,2,4-oxadiazol)yl]-1H-indol-3-yl]ethylamine. Bisoxalate

The bisoxalate salt, mp 125-127°C; (Found: C, 50.26; H, 4.07; N, 9.73. C₂₀H₁₇F₃N₄O.2 (C₂H₂O₄). 0.25 H₂O requires C, 50.09; H, 3.86; N, 9.73%); m/e 387 (M⁺+1); δ (360MHz, D₆-DMSO) 3.06 (4H, br s, 2 of CH₂); 4.29 (2H, s, CH₂); 7.43 (1H, s, aromatic-H); 7.56 (1H, d, J = 8.5Hz, aromatic-H); 7.60 (2H, d, J = 8.1Hz, aromatic-H's); 7.73 (2H, d, J = 8.1Hz, aromatic-H's); 7.81 (1H, d, J = 8.5Hz, aromatic-H); 7.91 (1H, br s, NH); 8.36 (1H s, aromatic-H).

### EXAMPLE 27

### N,N-Dimethyl-2-[5-[5-(3-(4-Acetylaminobenzyl)-1,2,4-oxadiazol)yl]-1H-indol-3-yl]ethylamine. Succinate Dihydrate

The title compound was prepared from N,N-dimethyl-2-(5-carboethoxy-1H-indol-3-yl)ethylamine and 4-acetylaminobenzyl amide oxime using the procedure described for Example 6. The succinate salt was prepared, mp 76-79°C; (Found: C, 58.05; H, 6.02; N, 12.52. C₂₃H₂₅N₅O₂. C₄H₆O₄. 2H₂O requires C,58.12; H, 6.32; N, 12.56%); m/e 404 (M⁺+1); δ (360MHz, D₆-DMSO) 2.02 (3H, s, Me); 2.44 (6H, s, N(Me)₂) 2.81 (2H, t, J = 7.2Hz, CH₂); 2.97 (2H, t, J = 7.2Hz, CH₂); 4.08 (2H, s, CH₂); 7.27 (2H, d, J = 8.5Hz, aromatic-H's); 7.36 (1H, s, aromatic-H); 7.52 (1H, d, J = 8.5Hz, aromatic-H); 7.53 (2H, d, J = 8.5Hz, aromatic-H's); 7.78 (1H, dd, J = 1.5 and 8.5Hz, aromatic-H); 8.32 (1H, s, aromatic-H); 9.90 (1H, s, NH); 11.37 (1H, s, NH).

### EXAMPLE 28

### N,N-Dimethyl-2-[5-[5-(3-(4-Methylsulphonyl aminobenzyl)-1,2,4-oxadiazol)yl]-1H-indol-3-yl]ethyl amine. Succinate Dihydrate

Prepared from N,N-dimethyl-2-(5-carboethoxy-1H-indol-3-yl)ethylamine and 4-methylsulphonylaminobenzyl amide oxime as described for Example 6. The succinate salt was prepared, mp 65-66°C; (Found: C, 52.99; H, 5.74; N, 11.86. C₂₂H₁₅N₅SO₃. C₄H₆O₄. 1.75H₂O requires C, 53.00; H, 5.90; N, 11.88%); m/e 440 (M⁺+1); δ (360MHz, D₆-DMSO) 2.50 (6H, s, N(Me)₂); 2.96 (3H, s, Me); 2.86-3.04 (4H, m, 2 of CH₂); 4.10 (2H, s, CH₂); 7.18 (2H, d, J = 8.3Hz, aromatic-H); 7.32 (2H, d, J = 8.3Hz, aromatic-H); 7.37 (1H, s, aromatic-H); 7.53 (1H, d, J = 8.4Hz, aromatic-H); 7.79 (1H, d, J = 8.4Hz, aromatic-H); 8.33 (1H, s, aromatic-H); 11.40 (2H, s, 2 of NH).

### EXAMPLE 29

### 2-[5-(5-(3-Naphth-2-yl-1,2,4-oxadiazol)ylmethyl)-1H-indol-3-yl]ethylamine. Sesquioxalate

Prepared from 2-(5-carboethoxymethyl-1H-indol-3-yl)ethylamine and 2-naphthyl amide oxime as described for Example 1. The sesquioxalate salt was prepared, mp 195-197°C (isopropylalcohol/ether); (Found: C, 61.07; H, 4.54; N, 11.15. C₂₃H₂₁N₄O. 1.6 (C₂H₂O₄) requires C, 61.28; H, 4.75; N, 10.91%); δ (360MHz, D₆-DMSO) 2.99 (2H, t, J = 7.3Hz, CH₂); 3.07 (2H, t, J = 7.3Hz, CH₂); 4.51 (2H, s, CH₂); 7.15 (1H, dd, J = 1.6 and 8.4Hz, aromatic-H); 7.26 (1H, d, J = 1.6Hz, aromatic-H); 7.38 (1H, d, J = 8.4Hz, aromatic-H); 7.59-7.64 (3H, m, aromatic-H's); 7.99-8.13 (4H, m, aromatic-H's); 8.60 (1H, s, aromatic-H).

### EXAMPLE 30

### N,N-Dimethyl-2-[5-[5-(3-Amino-1,2,4-oxadiazol)ylmethyl]-1H-indol-3-yl]ethylamine. Hemisuccinate Hydrate

Prepared from N,N-dimethyl-2-(5-carboethoxymethyl-1H-indol-3-yl)ethylamine and hydroxy guanidine sulphate as described for Example 6. The hemisuccinate salt was prepared, mp 150-153°C; (Found: C, 56.63; H, 6.62; N, 18.80. C₁₅H₁₉N₅O. 0.6 (C₄H₆O₄). 0.75 H₂O requires C, 56.53; H, 6.57; N, 18.94%); m/e 285 (M⁺); δ (360MHz, D₂O) 2.92 (6H, s, Me); 3.22 (2H, t, J = 7.4Hz, CH₂); 3.47 (2H, t, J = 7.4Hz, CH₂); 4.28 (2H, s, CH₂); 7.22 (1H, dd, J = 1.5 and 8.4Hz, aromatic-H); 7.35 (1H, s, aromatic-H); 7.52 (1H, d, J = 8.4Hz, aromatic-H); 7.62 (1H, s, aromatic-H).

### EXAMPLE 31

### 2-[5-[2-(5-(3-Amino-1,2,4-oxadiazol)yl)ethyl]-1H-indol-3-yl]ethylamine. Hydrogen Maleate Hydrate

The title compound was prepared from 2-[5-(2-(carboethoxy)ethyl)-1H-indol-3-yl]ethylamine and hydroxy guanidine sulphate using the procedure described for Example 6. The hydrogen maleate salt was prepared, mp 147-148°C (isopropylalcohol/ether); (Found: C, 53.89; H, 5.47; N, 17.67. c₁₄H₁₇N₅O. C₄H₄O₄. 0.75H₂O requires C, 53.93; H, 5.65; N, 17.47%); δ (360MHz, D₂O) 3.12 (2H, t, J = 6.9Hz, CH₂); 3.17 (4H, t, J = 4.1Hz, 2 of CH₂); 3.29 (2H, t, J = 6.9Hz, CH₂); 7.11 (1H, dd, J = 1.5 and 8.4Hz, aromatic-H); 7.27 (1H, s, aromatic-H); 7.40 (1H, s, aromatic-H); 7.44 (1H, d, J = 8.4Hz, aromatic-H).

### EXAMPLE 32

### 2-[5-[2-(5-(3-Dimethylamino-1,2,4-oxadiazol)yl)ethyl]-1H-indol-3-yl]ethylamine. Hemisuccinate

Prepared from 2-[5-(2-(carboethoxy)ethyl)-1H-indol-3-yl]ethylamine and dimethylamino amide oxime as described for Example 6. The hemisuccinate, mp 184-185°C; (Found: C, 59.83; H, 6.79; N, 18.41. C₁₆H₂₁N₅O. 0.62 (C₄H₆O₄) requires C, 59.57; H, 6.69; N, 18.79%); δ (360MHz, D₂O) 2.90 (6H, s, N(Me)₂); 3.12-3.20 (6H, m, 3 of CH₂); 3.30 (2H, t, J = 6.7Hz, CH₂); 7.09 (1H, dd, J = 1.6 and 8.4Hz, aromatic-H); 7.29 (1H, s, aromatic-H); 7.43 (1H, s, aromatic-H); 7.45 (1H, d, J = 8.4Hz, aromatic-H).

### EXAMPLE 33

### 2-[5-[3-(5-Methyl-1,2,4-oxadiazol)yl]-1H-indol-3-yl]ethylamine. Bisoxalate

### 1. 2-(5-Amide oxime-1H-indol-3-yl)ethylamine

Hydroxylamine hydrochloride (1.2g, 17.3mmol) was added to a stirred solution of sodium metal (0.4g, 17.5mmol) in methanol (10ml) followed by a solution of 2-(5-cyano-1H-indol-3-yl)ethylamine (1.25g, 6.8mmol), and the mixture refluxed for 16h. The mixture was filtered through hyflo filter aid and the solvent removed under vacuum and the residue chromatographed through silica gel (dichloromethane/ethanol/ammonia 30:8:1) to give the title product, mp 79-82°C; δ (360MHz, CD₃OD) 2.90-2.96 (4H, m, 2 of CH₂); 7.12 (1H, s, aromatic-H); 7.34 (1H, d, J = 8.5Hz, aromatic-H); 7.41 (1H, dd, J = 1.6 and 8.5Hz, aromatic-H); 7.87 (1H, s, aromatic-H).

### 2. 2-[5-[3-(5-Methyl-1,2,4-oxadiazol)yl]-1H-indol-3-yl]ethylamine. Bisoxalate

A solution of the preceding indolyl amide oxime (0.35g, 1.6mmol), sodium hydride (0.1g of an 80% dispersion in oil, 3.2mmol) and ethylacetate (0.5g, 5.7mmol), in ethanol (20ml) was heated under reflux for 2h. The solvent was removed under vacuum and the residue chromatographed through silica-gel eluting with dichloromethane/ethanol/ammonia (40:8:1) to give the title product (0.3g). The bisoxalate salt was prepared, mp 178-180°C; (Found: C, 48.68; H, 4.58; N, 13.04. C₁₃H₁₄N₄O. 2 (C₂H₂O₄) requires C, 48.35; N, 4.30; N, 13.27%); m/e 242 (M⁺); δ (360MHz, D₂O) 2.55 (3H, s, Me); 3.15 (2H, t, J = 7.2Hz, CH₂); 3.34 (2H, t, J = 7.2Hz, CH₂); 7.33 (1H, s, aromatic-H); 7.51 (1H, d, J = 8.4Hz, aromatic-H); 7.61 (1H, dd, J = 1.6 and 8.4Hz, aromatic-H); 7.97 (1H, d, J = 1.6Hz, aromatic-H).

### EXAMPLE 34

### 2-[5-[3-(5-Benzyl-1,2,4-oxadiazol)yl]-1H-indol-3-yl]ethylamine. Hydrogen Maleate

Prepared from 2-(5-amide oxime-1H-indol-3-yl)ethylamine and ethyl phenyl acetate as described for Example 33. The hydrogen maleate salt was prepared, mp 184-186°C; (Found: C, 64.10; H, 5.23; N, 13.25. C₁₉H₁₈N₄O. 0.9 (C₄H₄O₄) requires C, 64.19; H, 5.15; N, 13.25%); δ (360MHz, D₂O) 3.13 (2H, t, J = 7.3Hz, CH₂); 3.22 (2H, t, J = 7.3Hz, CH₂); 4.35 (2H, s, CH₂); 7.26 (1H, s, aromatic-H); 7.28-7.40 (5H, m, aromatic-H's); 7.47 (1H, d, J = 8.6Hz, aromatic-H); 7.82 (1H, dd, J = 1.3 and 8.6Hz, aromatic-H); 8.30 (1H, s, aromatic-H).

### EXAMPLE 35

### 2-[5-[3-(5-Methyl-1,2,4-oxadiazol)ylmethyl]-1H-indol-3-yl]ethylamine. Hydrogen Maleate

### 1. 2-(5-Acetamide oxime-1H-indol-3-yl)ethyl amine

Prepared from 2-(5-cyanomethyl-1H-indol-3yl)ethylamine and hydroxylamine as described in the preparation of Example 33; δ (360MHz, CD₃OD) 3.28-3.35 (4H, m, 2 of CH₂); 3.47 (2H, s, CH₂); 7.04 (1H, d, J = 8.4Hz, aromatic-H); 7.06 (1H, s, aromatic-H); 7.28 (1H, d, J = 8.4Hz, aromatic-H); 7.48 (1H, s, aromatic-H).

### 2. 2-[5-[3-(5-Methyl-1,2,4-oxadiazol)ylmethyl]-1H-indol-3yl]ethylamine. Hydrogen Maleate

Prepared from the preceding indolyl acetamide oxime and ethyl acetate using the general procedure. The hydrogen maleate salt was prepared, mp 145-149°C; (Found: C, 58.38; H, 5.70; N, 15.30. C₁₄H₁₆N₄O. C₄H₄O₄ requires C, 58.06; H, 5.41; N, 15.05%); m/e 256 (M⁺); δ (360MHz, D₂O) 2.55 (3H, s, Me); 3.16 (2H, t, J = 7.0Hz, CH₂); 3.34 (2H, t, J = 7.0Hz, CH₂); 4.18 (2H, s, CH₂); 7.18 (1H, d, J = 8.4Hz, aromatic-H); 7.32 (1H, s, aromatic-H); 7.50 (1H, d, J = 8.4Hz, aromatic-H); 7.60 (1H, s, aromatic-H).

### EXAMPLE 36

### 2-[5-[3-(5-Benzyl-1,2,4-oxadiazol)ylmethyl]-1H-indol-3-yl]ethylamine. Hydrogen Maleate

Prepared from 2-(5-acetamide oxime-1H-indol-3-yl)ethylamine and ethyl phenyl acetate using the general procedure. The hydrogen maleate salt, mp 143-144°C; (Found: C, 64.27; H, 5.56; N, 12.42. C₂₀H₂₀N₄O. C₄H₄O₄ requires C, 64.28; H, 5.39; N, 12.49%); δ (360MHz, D₂O) 3.11 (2H, t, J = 7.3Hz, CH₂); 3.28 (2H, t, J = 7.3Hz, CH₂); 4.16 (2H, s, CH₂); 4.27 (2H, s, CH₂); 7.13 (1H, dd, J = 1.5 and 8.4Hz, aromatic-H); 7.29 (1H, s, aromatic-H); 7.32-7.41 (5H, m, aromatic-H); 7.44 (1H, d, J = 8.4Hz, aromatic-H); 7.56 (1H, s, aromatic-H).

### EXAMPLE 37

### N,N-Dimethyl-2-[5-[3-(5-benzyl-1,2,4-oxadiazol)ylmethyl]-1H-indol-3-yl]ethylamine. Succinate

A solution of formaldehyde (0.85ml of a 35% solution in water) in methanol (10ml) was added dropwise to a stirred solution of 2[5-[3-(5-benzyl-1,2,4-oxadiazol)ylmethyl]-1H-indol-3-yl]ethylamine (0.4g, 1.2mmol), sodium cyanoborohydride (0.13g, 2.05mmol) and glacial acetic acid (0.34g), in methanol (15ml). The solution was stirred for 2.5h before basifying with a saturated solution of K₂CO₃ and extracting with ethyl acetate (3 x 50ml). The combined extracts were dried (MgSO₄), evaporated, and the residue chromatographed through silica gel eluting with dichloromethane/ethanol/ammonia 60:8:1 to give the title product (0.33g). The succinate salt was prepared, mp 195-196°C; (Found: C, 64.66; H, 6.37; N, 11.55. C₂₂H₂₄N₄O. 1.1 (C₄H₆O₄) requires C, 64.66; H, 6.29; N, 11.43%); m/e 360 (M⁺); δ (360MHz, D₂O) 2.89 (6H, s, N(Me)₂); 3.18 (2H, t, J = 7.4Hz, CH₂); 3.43 (2H, t, J = 7.4Hz, CH₂); 4.16 (2H, s, CH₂); 4.27 (2H, s, CH₂); 7.14 (1H, d, J = 8.4Hz, aromatic-H); 7.31-7.40 (6H, m, aromatic-H's); 7.44 (1H, d, J = 8.4Hz, aromatic-H); 7.56 (1H, s, aromatic-H).

Examples 38-48 were prepared from 2-(5-carboethoxy-1H-indol-3-yl)ethylamine and the appropriate amide oxime using the procedure described for Example 6, unless otherwise stated.

### EXAMPLE 38

### 2-[5-(5-(3-Methoxymethyl-1,2,4-oxadiazol)yl)-1H-indol-3-yl]ethylamine. Hemisuccinate

The hemisuccinate salt: mp 207-210°C (methanol/diethylether); (Found: C, 58.01; H, 5.85; N, 16.85. C₁₄H₁₆N₄O₂.0.5 (C₄H₆O₄) requires C, 58.00; H, 5.78; N, 16.91%); δ (360MHz, D₂O) 3.21 (2H, t, J = 7.2Hz, CH₂); 3.35 (2H, t, J = 7.2Hz, CH₂); 3.51 (3H, s, Me); 4.68 (2H, s, CH₂OMe); 7.43 (1H,s,Ar-H); 7.65 (1H,d,J = 8.6Hz, Ar-H); 7.90 (1H, dd, J = 8.6 and 1.6Hz, Ar-H); 8.41 (1H, d, J = 1.6Hz, Ar-H).

### EXAMPLE 39

### 2-[5-(5-(3-(4-N-Methylcarbamoyl benzyl)-1,2,4-oxadiazol)yl)-1H-indol-3-yl)]ethylamine. Succinate. Hydrate

The succinate salt mp 108-110°C; (Found: C, 59.12; H, 5.59; N, 14.05. C₂₁H₂₁N₅O₂. (C₄H₆O₄). 0.75H₂O requires C, 59.22; H, 5.66; N, 13.82%). δ (360MHz, D₆-DMSO) 2.77 (3H, d, J = 4.5Hz, CH₃); 3.01 (4H, br s, 2 of C H₂); 4.21 (2H, s, CH₂); 7.40 (1H, s, Ar-H); 7.43 (2H, d, J = 8.2Hz, Ar-H); 7.55 (1H, d, J = 8.6Hz, Ar-H); 7.79-7.81 (2H, d, J = 8.2Hz, Ar-H); 7.79-7.81 (1H, d, J = 8.6Hz, Ar-H); 8.35 (1H, s, Ar-H); 8.39 (1H, br q, J = 4.5Hz, NH).

### EXAMPLE 40

### 2-[5-(5-(3-(4-N-Methylcarbamoylphenyl)-1,2,4-oxadiazol)yl)-1H-indol-3-yl]ethylamine. Succinate. Hydrate

The succinate salt: mp 126-128°C; (Found: C, 58.31; H, 5.21; N, 14.22. C₂₀H₁₉N₅O₂. (C₄H₆O₄). 0.75H₂O requires C, 58.47; H, 5.41; N, 14.21%); δ (360MHz, D₆-DMSO) 2.83 (3H, d, J = 4.5Hz, CH₃); 3.06 (4H, br s, 2 of CH₂); 3.37 (2H, br s, NH₂); 7.44 (1H, s, Ar-H); 7.62 (1H, d, J = 8.5Hz, Ar-H); 7.94 (1H, dd, J = 1.6 and 8.5Hz, Ar-H); 8.04 (2H, d, J = 8.5Hz, Ar-H); 8.19 (2H, d, J = 8.5Hz, Ar-H); 8.49 (1H, s, Ar-H); 8.62 (1H, br q, J = 4.5Hz, NH).

### EXAMPLE 41

### 2-[5-(5-(3-(4-Methylaminosulphonylbenzyl)-1,2,4-oxadiazol)yl)-1H-indol-3-yl]ethylamine. Bissuccinate. Hydrate

Bissuccinate salt: mp 49-50°C (hygroscopic salt); (Found: C, 50.91; H, 5.47; N, 10.79. C₂₀H₂₁N₅SO₃. 2 (C₄H₆O₄). 0.75H₂O requires C, 50.87; H, 5.26; N, 10.59%). δ (360MHz, D₂O) 2.55 (3H, s, CH₃); 3.16 (2H, t, J = 7.1Hz, CH₂); 3.32 (2H, t, J = 7.1Hz, CH₂); 4.26 (2H, s, CH₂); 7.38 (1H, s, Ar-H); 7.58 (1H, d, J = 8.9Hz, Ar-H); 7.62 (2H, d, J = 8.4Hz, Ar-H); 7.79 (1H, dd, J = 1.6 and 8.9Hz, Ar-H); 7.84 (2H, d, J = 8.4Hz, Ar-H); 8.31 (1H, d, J = 1.6Hz, Ar-H).

### EXAMPLE 42

### 2-[5-(5-(3-(4-Dimethylaminosulphonylbenzyl)-1,2,4-oxadiazol)yl)-1H-indol-3-yl]ethylamine. Hydrochloride. Hydrate

The hydrochloride monhydrate salt: mp 144-145°C (MeOH)/Et₂O); (Found: C, 52.71; H, 5.50; N, 14.44. C₂₁H₂₄N₅SO₃Cl. 1H₂O requires C, 52.55; H, 5.46; N, 14.59%).

### EXAMPLE 43

### 2-[5-(5-(3-(3-Methylsulphonylaminobenzyl)-1,2,4-oxadiazol)yl)-1H-indol-3-yl]ethylamine. Hydrochloride. Dihydrate

The hydrochloride dihydrate salt: mp 241-242°C; (Found: C, 48.54; H, 5.05; N, 13.59. C₂₀H₂₁N₅SO₃. 1.2HCl. 2.4H₂O requires C, 48.19; H, 5.45; N, 14.05%).

### EXAMPLE 44

### 2-[5-(5-(3-(4-Carbamoylaminobenzyl)-1,2,4-oxadiazol)yl)-1H-indol-3-yl]ethylamine. Sesquioxalate. Hemihydrate

The sesquioxalate hemihydrate salt: mp 194-197°C; (Found: C, 52.8; H, 4.75; N, 16.42. C₂₀H₂₁N₆O₂. 1.5 (C₂H₂O₄). 0.5H₂O requires C, 53.1; H, 4.65; N, 16.15%).

### EXAMPLE 45

### 2-[5-(5-(3-Amino-1,2,4-oxadiazol)-yl)-1H-indol-3-yl]ethylamine. Bisoxalate. Hydrate

The bisoxalate salt: mp 160-164°C; (Found: C, 39.90; H, 4.29; N, 21.37. C₁₂H₁₃N₅O. 2(C₂H₂O₄). 0.9 (CH₅N₃O). 0.75H₂O requires C, 40.24; H, 4.60; N, 21.38%); δ (360MHz, D₂O) 3.15 (2H, t, J = 7.1Hz, CH₂); 3.34 (2H, t, J = 7.1Hz, CH₂); 7.33 (1H, s, Ar-H); 7.51 (1H, d, J = 8.5Hz, Ar-H); 7.69 (1H, dd, J = 1.5 and 8.5Hz, Ar-H); 8.12 (1H, d, J = 1.5Hz, Ar-H).

### EXAMPLE 46

### 2-[5-(5-(3-Acetylaminomethyl-1,2,4-oxadiazol)yl)-1H-indol-3-yl]ethylamine. Hemisuccinate. Monohydrate

The hemisuccinate monhydrate salt: mp 107-110°C; (Found: C, 54.47; H, 6.28; N, 17.81. C₁₅H₁₇N₅O₂. 0.5 (C₄H₆O₄). 1H₂O. 0.2 (iPA) requires C, 54.43; H, 6.13; N, 18.03%); δ (360MHz, D₂O) 2.14 (3H, s, CH₃); 3.14 (2H, t, J = 7.1Hz, CH₂); 3.34 (2H, t, J = 7.1Hz, CH₂); 4.51 (2H, s, CH₂); 7.33 (1H, s, Ar-H); 7.49 (1H, d, J = 8.7Hz, Ar-H); 7.68 (1H, dd, J = 1.5 and 8.7Hz, Ar-H); 8.09 (1H, d, J = 1.5Hz, Ar-H).

### EXAMPLE 47

### 2[5-(5-(3-(2-Acetylamino)ethyl-1,2,4-oxadiazol)yl)-1H-indol-3-yl]ethylamine. Oxalate. Hemihydrate

The oxalate hemihydrate salt: mp 188-189°C; (Found: C, 52.48; H, 5.56; N, 16.77. C₁₆H₁₉N₅O₂. C₂H₂O₄. 0.6H₂O requires C, 52.20; H, 5.40; N, 16.90%).

### EXAMPLE 48

### 2-[5-(5-(3-Aminomethyl-1,2,4-oxadiazol)yl)-1H-indol-3-yl]ethylamine. Succinate. Dihydrate

The succinate dihydrate salt: mp 125-130°C; (Found: C, 49.13; H, 6.06; N, 16.99. C₁₃H₁₅N₅O.2 (C₄H₆O₄). 2.2H₂O requires C, 49.19; H, 6.16; N, 16.87%).

Examples 49-54 were prepared from N,N-dimethyl-2-(5-carboethoxy-1H-indol-3yl)ethylamine and the appropriate amide oxime using the procedure described for Example 6.

### EXAMPLE 49

### N,N-Dimethyl-2-[5-(5-(3-amino-1,2,4-oxadiazol)yl)-1H-indol-3-yl]ethylamine. Bisoxalate. Monohydrate

The bisoxalate monhydrate salt: mp 156-158°C; (Found: C, 46.49; H, 4.66; N, 15.83. C₁₄H₁₇N₅O. 1.8 (C₂H₂O₄) 1H₂O requires C, 46.83; H, 5.04; N, 15.52%); δ (360MHz, D₂O) 2.93 (6H, s, 2 of CH₃); 3.18 (2H, t, J = 7.6Hz, CH₂); 3.46 (2H, t, J = 7.6Hz, CH₂); 7.33 (1H, s, Ar-H); 7.48 (1H, d, J = 8.7Hz, Ar-H); 7.65 (1H, dd, J = 8.7 and 1.5Hz, Ar-H); 8.04 (1H, d, J = 1.5Hz, Ar-H).

### EXAMPLE 50

### N,N-Dimethyl-2-[5-(5-(3-acetylaminomethyl-1,2,4-oxadiazol)yl)-1H-indol-3yl]ethylamine. Succinate. Hemihydrate

The succinate hemihydrate salt: mp 65-70°C (hygroscopic); (Found: C, 56.14; H, 6.03; N, 16.02. C₁₇H₂₁N₅O₂. 0.8 (C₄H₆O₄). 0.6H₂O requires C, 56.08; H, 6.29; N, 16.19%); δ (360MHz, D₂O) 2.16 (3H, s, CH₃); 2.96 (6H, s, 2 of CH₃); 3.15 (2H, t, J = 7.8Hz, CH₂); 3.45 (2H, t, J = 7.8Hz, CH₂); 4.50 (2H, s, CH₂); 7.31 (1H, s, Ar-H); 7.45 (1H, d, J = 8.6Hz, Ar-H); 7.61 (1H, dd, J = 8.6 and 1.5Hz, Ar-H); 7.96 (1H, d, J = 1.5Hz, Ar-H).

### EXAMPLE 51

### N,N-Dimethyl-2-[5-(5-(3-(2-acetylamino)ethyl-1,2,4-oxadiazol)yl)-1H-indol-3-yl]ethylamine. Sesquioxalate. Monohydrate

The sesquioxalate monohydrate salt: mp 35°C (hygroscopic); (Found: C, 51.76; H, 5.73; N, 14.17. C₁₈H₂₃N₅O₂. 1.4 (C₂H₂O₄). 0.9H₂O requires C, 51.65; H, 5.75; N, 14.47%).

### EXAMPLE 52

### N,N-Dimethyl-2[5-(5-(3-(4-carbamoylaminobenzyl)-1,2,4-oxadiazol)yl)-1H-indol-3-yl]ethylamine. Hydrochloride

The hydrochloride salt: mp 214-215°C; (Found: C, 57.70; H, 6.13; N, 17.34. C₂₂H₂₄N₆O₂. 1.25HCl. 1.0C₂H₅OH requires C, 58.10; H, 6.35; N, 16.94%).

### EXAMPLE 53

### N,N-dimethyl-2-[5-(5-(3-(2-t-butyloxycarbonyl amino)ethyl-1,2,4-oxadiazol)yl)-1H-indol-3-yl]ethylamine. Oxalate

The oxalate salt: mp 184-185°C; (Found: C, 55.97; H, 6.38; N, 14.18. C₂₁H₃₀N₅O₃. C₂H₂O₄. 0.3H₂O requires C, 55.82; H, 6.44; N. 14.15%).

### EXAMPLE 54

### N,N-Dimethyl-2-[5-(5-(3-(4-N-methylcarbamoylbenzyl)-1,2,4-oxadiazol)yl)-1H-indol-3-yl]ethylamine. Hemioxalate. Dihydrate

The hemioxalate dihydrate salt: mp 109-111°C; (Found: C, 59.88; H, 5.99; N, 14.24. C₂₃H₂₅N₅O₂. 0.5 (C₂H₂O₄). 1.9H₂O requires C, 59.71; H, 6.22; N, 14.51%).

### EXAMPLE 55

### N,N-Dimethyl-2-[5-(5-(3-(2-amino)ethyl-1,2,4-oxadiazol)yl)-1H-indol-3-yl]ethylamine. Sesquioxalate. Hemihydrate

Trifluoroacetic acid (25ml, 0.133mol) was added to a solution of N,N-dimethyl-2-[5-(5-(3-(2-t-butyloxycarbonylamino)ethyl-1,2,4-oxadiazol)yl)-1H-indol-3-yl]ethylamine (0.5g, 1.25mmol) in anhydrous CH₂Cl₂ (10ml) and the mixture stirred at 25°C for 1h. The solvent was removed under vacuum, aqueous K₂CO₃ (30ml) added, and extracted with EtOAc (6 x 200ml). The extracts were combined, dried, and evaporated to give the title-amine (0.36g, 96%). The sesquioxalate salt was prepared: mp 220-221°C; (Found: C, 50.81; H, 5.78; N, 15.49. C₁₆H₂₁N₅O. 1.6 (C₂H₂O₄) 0.5H₂O requires C, 50.97; H, 5.61; N, 15.48%); δ (360MHz, D₂O) 2.95 (6H, s, 2 of CH₃); 3.22-3.29 (4H, m, 2 of CH₂); 3.52 (4H, t, J = 7.2Hz,2 of CH₂); 7.42(1H, s, Ar-H); 7.61 (1H, d, J = 8.6Hz, Ar-H);7.86(1H,d,J =8.6Hz,Ar-H);8.32(1H,s,Ar-H).

### EXAMPLE 56

### N,N-dimethyl-2-[5-(5-(3 -(2-methylsulphonylamino)ethyl-1,2,4-oxadiazol)yl)-1H-indol-3-yl]ethylamine. Oxalate.1.5 Hydrate

Methane sulphonyl chloride (0.14ml, 1.81mmol) in CH₂Cl₂)(10ml) was added dropwise to a stirred solution of the preceding amine (Example 55;0.36g, 1.2mmol) in CH₂Cl₂ (10ml and pyridine (0.29ml, 3.6mmol), at -30°C. The solution was stirred for 1h, allowing to warm to room temperature. The solvent was removed under vacuum, and the residue purified by chromatography on silica-gel eluting with CH₂Cl₂/EtOH/NH₃ (90:8:1). The oxalate salt was prepared on the product obtained: mp <30°C (hygroscopic); (Found: C, 46.15; H, 5.71; N, 14.16. C₁₇H₂₃N₅SO₃. C₂H₂O₄. 1.5H₂O requires C, 46.39; H, 5.49; N, 14.12%). δ (360MHz, D₂O) 2.94 (6H, s, 2 of CH₃); 2.99 (2H, t, J = 6.5Hz, CH₂); 3.09 (3H, s, CH₃); 3.16-3.24 (2H, m, CH₂); 3.48 (2H, t, J = 7.4Hz, CH₂); 3.55 (2H, t, J = 6.5Hz, CH₂); 7.35 (1H, s, Ar-H); 7.52 (1H, d, J = 8.6Hz, Ar-H); 7.72 (1H, dd, J = 1.6 and 8.6Hz, Ar-H); 8.13 (1H, d, J = 1.6Hz, Ar-H).

### EXAMPLE 57

### N,N-dimethyl-2-[5-(5-(3-(2-carbamoylamino)ethyl-1,2,4-oxadiazol)yl)-1H-indol-3-yl]ethylamine. Sesquioxalate

Carbonyl diimidazole (0.26g, 1.6mmol) was added to a solution of Example 55 (0.4g, 1.53mmol) in dry THF (20ml), at 20°C. The solution was warmed to room temperature and stirred for 3h. NH₃(g) was then bubbled through the solution for 8h. The solvent was removed under vacuum and the residue chromatographed on silica-gel eluting with CH₂Cl₂/EtOH/NH₃ (60:8:1) to give the title-urea. The sesquioxalate salt was prepared: mp 81-82°C; (Found: C, 49.25; H, 5.44; N, 16.42. C₁₇H₂₂N₆O₂. 1.7 (C₂H₂O₄). 0.5 (MeOH) requires C, 49.08; H, 5.40; N, 16.43%).

### EXAMPLE 58

### N,N-Dimethyl-2-[5-(5-(3-(2-N-methyl carbamoyl amino)ethyl-1,2,4-oxadiazol)yl)-1H-indol-3yl]ethyl amine. Oxalate

To a solution of Example 55 (0.5g, 1.67mmol) in CH₂Cl₂ (30ml) was added dropwise a solution of methyl isocyanate (0.105g, 1.84mmol) in CH₂Cl₂ (10ml), at room temperature. The solution was stirred for 1h before removing the solvent under vacuum and preparing the oxalate salt of the product obtained: mp 185-188°C; (Found: C, 53.27; H, 5.92; N, 18.66. C₁₈H₂₄N₆O₂. C₂H₂O₄. 0.25H₂O requires C, 53.27; H, 5.92; N, 18.64%).

### EXAMPLE 59

### N,N-Dimethyl-2-[5-(5-(3-(2-methoxycarbonylamino)ethyl-1,2,4-oxadiazol)yl)-1H-indol-3-yl]ethylamine. Oxalate

To a solution of Example 55 (0.14g, 0.45mmol) in dry CH₂Cl₂ (7ml), at 0°C, was added triethylamine (0.60ml) and methylchloroformate (0.33ml). The reaction mixture was allowed to warm to room temperature and stir for 16h. Basic workup afforded a crude product which was purified by chromatography on silica-gel eluting with CH₂Cl₂MeOH/NH₃ (80:16:1). The oxalate salt was prepared from the product thus obtained: mp 175-181°C; (Found: C, 53.28; H, 5.46; N, 15.45. C₁₈H₂₃N₅O₃. C₂H₂O₄. 0.1H₂O requires C, 53.47; H, 5.65; N, 15.59%).

### EXAMPLE 60

### N,N-Dimethyl-2-[5-(5-(3-(2-ethoxycarbonylamino)ethyl-1,2,4-oxadiazol)yl)-1H-indol-3-yl]ethylamine. Oxalate

Prepared from the amine, Example 55, using ethylchloroformate as described for Example 59. The oxalate salt was prepared: mp 169-172°C; (Found: C, 54.09; H, 5.91; N, 14.94. C₁₉H₂₅N₅O₃. C₂H₂O₄. 0.2H₂O requires C, 54.23; H, 5.94; N, 15.06%).

### EXAMPLE 61

### N,N-Dimethyl-2-[5-(2-(5-(3-amino-1,2,4-oxadiazol)yl)ethyl)-1H-indol-3-yl]ethylamine. Oxalate

Prepared from N,N-dimethyl-2-(5(2-(carboethoxy)ethyl)-1H-indol-3-yl)ethylamine and hydroxyguanidine sulphate as described for Example 6. The oxalate salt was prepared: mp 164-167°C; (Found: C, 55.07; H, 5.74; N,17.81. C₁₆H₂₁N₅O.1.1 (C₂H₂O₄) requires C, 54.87; H, 5.87; N, 17.58%); δ (360MHz, D₂O) 2.89 (6H, s, 2 of CH₃); 3.21-3.14 (6H, m, 3 of CH₂); 3.42 (2H, t, J = 7.3Hz, CH₂); 7.12 (1H, dd, J = 1.6 and 8.4Hz, Ar-H); 7.30 (1H, s, Ar-H); 7.38 (1H, d, J = 1.6Hz, Ar-H); 7.45 (1H, d, J = 8.4Hz, Ar-H).

Examples 62-82 were prepared from N,N-dimethyl-2(5-carboethoxymethyl-1H-indol-3-yl)ethylamine and the appropriate amide oxime using the general NaOEt/EtOH procedure.

### EXAMPLE 62

### N,N-Dimethyl-2-[5-(5-(3-N-methylamino-1,2,4-oxadiazol)ylmethyl)-1H-indol-3-yl]ethylamine. Oxalate

The oxalate salt: mp 184°C (EtOH/Et₂O); (Found:C, 55.37; H, 6.17; N, 17.62. C₁₆H₂₁N₅O. 1.05 (C₂H₂O₄) requires C, 55.19; H, 5.91; N, 17.78%); δ (360MHz, D₆-DMSO) 2.66 (3H, d, J = 5.0Hz, NHMe); 2.78 (6H, s, 2 of CH₃); 3.03 (2H, m, CH₂); 3.23 (2H, m, CH₂); 4.15 (2H, s, CH₂); 6.54 (1H, q, J = 5.0Hz, NHMe); 7.02 (1H, d, J = 8.3Hz, Ar-H); 7.24 (1H, s, Ar-H); 7.32 (1H, d, J = 8.3Hz, Ar-H); 7.51 (1H, s, Ar-H); 10.98 (1H, s, indole NH).

### EXAMPLE 63

### N,N-Dimethyl-2-[5-(5-(3-(4-carbamoylbenzyl)-1,2,4-oxadiazol)ylmethyl)-1H-indol-3-yl]ethylamine Oxalate. Monohydrate

The oxalate monohydrate salt: mp 98-101°C; (Found: C, 58.95; H, 5.66; N, 13.78. C₂₃H₂₅N₅O₂. C₂H₂O₄. 0.9H₂O requires C, 58.90; H, 5.69; N, 13.74%); δ (360MHz, D₂O) 2.82 (6H, s, 2 of CH₃); 3.04 (2H, t, J = 7.5Hz, CH₂); 3.31 (2H, t, J = 7.5Hz, CH₂); 4.01 (2H, s, CH₂); 4.28 (2H s CH₂); 7.08 (1H, dd, J = 1.4 and 8.4Hz, Ar-H); 7.22 (2H, d, J = 8.2Hz, Ar-H); 7.24 (1H, s, Ar-H); 7.39 (1H, d, J = 8.4Hz, Ar-H); 7.48 (1H, d, J = 1.4Hz, Ar-H); 7.58 (2H, d, J = 8.2Hz, Ar-H).

### EXAMPLE 64

### N,N-Dimethyl-2-[5-(5-(3-(4-acetylaminobenzyl)-1,2,4-oxadiazol)ylmethyl)-1H-indol-3-yl]ethylamine. Oxalate. Hemihydrate

The oxalate hemihydrate salt: mp 98-102°C; (Found: C, 60.26; H, 5.72; N, 13.48. C₂₄H₂₇N₅O₂. C₂H₂O₄. 0.6H₂O requires C, 60.24; H, 5.87; N, 13.51%); δ (360MHz, D₂O) 2.11 (3H, s, CH₃); 2.77 (6H, s, 2 of CH₃); 2.99 (2H, t, J = 7.6Hz, CH₂); 3.25 (2H, t, J = 7.6Hz, CH₂); 3.89 (2H, s, CH₂); 4.23 (2H, s, CH₂); 7.06 (1H, dd, J = 1.5 and 8.4Hz, Ar-H); 7.10 (2H, d, J = 8.5Hz, Ar-H); 7.22 (2H, d, J = 8.5Hz, Ar-H); 7.23 (1H, s, Ar-H); 7.39 (1H, d, J = 8.4Hz, Ar-H); 7.41 (1H, s, Ar-H).

### EXAMPLE 65

### N,N-Dimethyl-2-[5(5-(3(4-methylaminosulphonylbenzyl)-1,2,4-oxadiazol)ylmethyl)-1H-indol-3-yl]ethylamine. Oxalate

The oxalate salt: mp 160-164°C; (Found: C, 54.89; H, 5.48; N, 12.76. C₂₃H₂₇N₅SO₃. C₂H₂O₄ requires C, 55.24; H, 5.38; N, 12.88%).

### EXAMPLE 66

### N,N-Dimethyl-2-[5-(5-(3-(4-carbamoylaminobenzyl)-1,2,4-oxadiazol)ylmethyl)-1H-indol-3-yl]ethylamine. Oxalate. Monohydrate

The oxalate monhydrate salt: mp 176-177°C; (Found: C, 57.10; H, 6.04; N, 15.97. C₂₃H₂₆N₆O₂. C₂H₂O₄. 1.0H₂O requires C, 57.03; H, 5.74; N, 15.96%).

### EXAMPLE 67

### N,N-Dimethyl-2-[5-(5-(3-(4-methylsulphonyl aminobenzyl-1,2,4-oxadiazol)ylmethyl)-1H-indol-3-yl]ethylamine. Oxalate

The oxalate salt: mp 156-159°C; (Found: C, 54.64; H, 5.35; N, 12.70; S, 6.13. C₂₃H₂₇N₅SO₃. C₂H₂O₄. 0.25H₂O requires C, 54.78; H, 5.43; N, 12.78; S,5.85%).

### EXAMPLE 68

### N,N-Dimethyl-2-[5-(5-(3-(4-N-methylcarbamoyl phenyl)-1,2,4-oxadiazol)ylmethyl)-1H-indol-3-yl]ethylamine. Oxalate. Hemihydrate

The oxalate hemihydrate salt: mp 205-207°C; (Found: C, 59.65; H, 5.71; N, 14.22. C₂₃H₂₅N₅O₂. C₂H₂O₄. 0.5H₂O requires C, 59.75; H, 5.62; N, 13.94%); δ (360MHz, D₂O) 2.83 (6H, s, 2 of CH₃); 2.89 (3H, s, CH₃); 3.01 (2H, t, J = 7.6Hz, CH₂); 3.29 (2H, t, J = 7.6Hz, CH₂); 4.14 (2H, s, CH₂); 6.98 (1H, d, J = 8.4Hz, Ar-H); 7.19 (1H, s, Ar-H); 7.34 (1H, d, J = 8.4Hz, Ar-H); 7.44 (1H, s, Ar-H); 7.60 (2H, d, J = 8.4Hz, Ar-H); 7.68 (2H, d, J = 8.4Hz, Ar-H).

### EXAMPLE 69

### N,N-Dimethyl-2-[5-(5-(3-acetylaminomethyl-1,2,4-oxadiazol)ylmethyl)-1H-indol-3-yl]ethylamine. Succinate. Hemihydrate

The succinate hemihydrate salt: mp 165°C; (Found: C, 56.94; H, 6.71; N, 14.57. C₁₅H₂₃N₅O₂. C₄H₆O₄ 0.4H₂O requires C, 56.62; H, 6.44; N, 15.01%); δ (360MHz, D₂O) 2.04 (3H, s, CH₃); 2.92 (6H, s, 2 of CH₃); 3.23 (2H, t, J = 7.3Hz, CH₂); 3.48 (2H, t, J = 7.3Hz, CH₂); 4.41 (2H, s, CH₂); 4.46 (2H, s, CH₂); 7.21 (1H, dd, J = 1.6 and 8.4Hz, Ar-H); 7.35 (1H, s, Ar-H); 7.51 (1H, d, J = 8.4Hz, Ar-H); 7.63 (1H, s, Ar-H).

### EXAMPLE 70

### N,N-Dimethyl-2-[5-(5-(3-methylsulphonylaminomethyl-1,2,4-oxadiazol)ylmethyl)-1H-indol-3-yl]ethylamine. Oxalate. Hemihydrate

The oxalate hemihydrate salt: mp 148-150°C; (Found: C, 47.90; H, 5.52; N, 14.37. C₁₇H₂₃N₅SO₃. C₂H₂O₄. 0.6H₂O requires C, 47.71; H, 5.52; N, 14.64%).

### EXAMPLE 71

### N,N-Dimethyl-2-[5-(5-(3-carbamoylmethyl-1-2,4-oxadiazol)ylmethyl)-1H-indol-3-yl]ethylamine. Oxalate

The oxalate salt: mp 210°C (dec.); (Found: C, 53.55; H, 5.38; N, 15.83. C₁₃H₂₁N₄O₂. 1.2 (C₂H₂O₄) requires C, 53.51; H, 5.42; N, 16.08%); δ (360MHz, D₂O) 2.90 (6H, s, 2 of CH₃); 3.21 (2H, t, J = 7.3Hz, CH₂); 3.47 (2H, t, J = 7.3Hz, CH₂); 3.80 (2H, s, CH₂); 4.43 (2H, s, CH₂); 7.22 (1H, dd, J = 1.6 and 8.4Hz Ar-H); 7.34 (1H, s, Ar-H); 7.51 (1H, d, J = 8.4Hz, Ar-H); 7.63 (1H, d, J = 1.6Hz, Ar-H).

### EXAMPLE 72

### N,N-Dimethyl-2-[5-(5-(3-(3-methylsulphonylamino benzyl)-1,2,4-oxadiazol)ylmethyl)-1H-indol-3-yl]ethylamine. Oxalate

The oxalate salt: mp 95-97°C (EtOH/Et₂O): (Found: C, 55.28; H, 5.58; N, 12.72%. C₂₃H₂₇N₅SO₃. C₂H₂O₄ requires C, 55.14; H, 5.55; N, 12.86%); δ (360MHz, D₂O) 2.86 (6H, s, NMe₂); 2.87 (3H, s, MeSO₂); 3.13 (2H, t, J = 7.3Hz, CH₂); 3.39 (2H, t, J = 7.3Hz, CH₂); 4.03 (2H, s, CH₂); 4.31 (2H, s, CH₂); 7.07-7.14 (4H, m, Ar-H); 7.29-7.34 (2H, m, Ar-H); 7.43 (1H, d, H = 8.4Hz, Ar-H); 7.52 (1H, s, Ar-H).

### EXAMPLE 73

### N,N-Dimethyl-2-[5-(5-(3-(3-acetylaminobenzyl)-1,2,4-oxadiazol)ylmethyl)-1H-indol-3yl]ethylamine. Oxalate

The oxalate salt: mp 92-96°C (EtOH/Et₂O); (Found: C, 61.72; H, 6.02; N, 13.60. C₂₄H₂₇N₅O₂. C₂H₂O₄ requires C, 61.53; H, 5.76; N, 13.80%).

### EXAMPLE 74

### N,N-Dimethyl-2-[5(5-(3-(4-carbamoylphenyl)-1,2,4-oxadiazol)ylmethyl)-1H-indol-3-yl]ethylamine. Oxalate

The oxalate salt: mp 217-219°C; (Found: C, 59.15; H, 5.38; N, 14.23. C₂₂H₂₃N₅O₂. 1.2 (C₂H₂O₄) requires C, 58.91; H, 5.15; N, 14.08%).

### EXAMPLE 75

### N,N-Dimethyl-2-[5(5-(3-(3-carbamoylphenyl)1,2,4-oxadiazol)ylmethyl)-1H-indol-3yl]ethylamine. Oxalate

The oxalate salt: mp 109-111°C; (Found: C, 59.46; H, 5.41; N, 14.40. C₂₂H₂₃N₅O₂ requires C, 59.50; H, 5.20; N, 14.33%).

### EXAMPLE 76

### N,N-Dimethyl-2-[5-(5-(3-(4-methylsulphonylamino phenyl)-1,2,4-oxadiazol)ylmethyl)-1H-indol-3-yl]ethylamine. Oxalate

The oxalate salt: mp 213-215°C; (Found: C, 55.03; H, 5.91; N, 12.44. C₂₂H₂₅N₅SO₃. C₂H₂O₄. 0.4 (Et₂O) requires C, 54.98; H, 5.59; N, 12.52%).

### EXAMPLE 77

### N,N-Dimethyl-2-[5-(5-(3-(4-methylaminosulphonylmethyl phenyl)-1,2,4-oxadiazol)ylmethyl)-1H-indol-3-yl]ethylamine. Oxalate

The oxalate salt: mp 190-192°C; (Found: C, 55.03; H, 5.77; N, 12.36. C₂₃H₂₇N₅SO₃. C₂H₂O₄. 0.5 (EtOH) requires C, 55.11; H, 5.69; N, 12.36%).

### EXAMPLE 78

### N,N-Dimethyl-2-[5-(5-(3-(3-methylaminosulphonylmethyl phenyl)-1,2,4-oxadiazol)ylmethyl)-1H-indol-3-yl]ethylamine. Oxalate. 0.3 Hydrate

The oxalate salt: mp 199-210°C; (Found: C, 54.54; H, 5.41; N, 12.65; S, 5.80. C₂₃H₂₇N₅SO₃. C₂H₂O₄. 0.3H₂O requires C, 54.69; H, 5.43; N, 12.76; S, 6.01%).; δ (360MHz, D₂O) 2.69 (3H, s, CH₃); 2.87 (6H, s, 2 of CH₃); 3.17 (2H t, J = 7.5Hz, CH₂); 3.43 (2H, t, J = 7.5Hz, CH₂); 4.42 (2H, s, CH₂); 4.48 (2H, s, CH₂); 7.23 (1H, d, J = 8.5Hz, Ar-H); 7.30 (1H, s, Ar-H); 7.49 (1H, d, J = 8.4Hz, Ar-H); 7.53-7.57 (2H, m, Ar-H); 7.63 (1H, s, Ar-H); 7.90-7.92 (2H, m, Ar-H).

### EXAMPLE 79

### N,N-Dimethyl-2-[5-(5-(3-(4-aminosulphonylmethyl phenyl)-1,2,4-oxadiazol)ylmethyl)-1H-indol-3yl]ethylamine. Oxalate. 1.5 Hydrate

The oxalate hydrate salt: mp 210-212°C; (Found: C, 51.72; H, 5.43; N, 12.49. C₂₂H₂₅N₅SO₃. C₂H₂O₄. 1.5H₂O requires C, 51.76; H, 5.34; N, 12.39%).

### EXAMPLE 80

### N,N-Dimethyl-2[5-(5-(3-(4-dimethylaminosulphonyl methylphenyl)-1,2,4-oxadiazol)ylmethyl)-1H-indol-3-yl]ethylamine. Oxalate. 0.25 Hydrate

The oxalate 0.25 hydrate salt: mp 208-210°C; (Found: C, 56.63; H, 5.74; N, 12.91. C₂₄H₂₉N₅SO₃. 0.75 (C₂H₂O₄) 0.25H₂O requires C, 56.76; H, 5.79; N, 12.98%).

### EXAMPLE 81

### N,N-Dimethyl-2-[5-(5-(3-t-butyloxycarbonylaminomethyl-1,2,4-oxadiazol)ylmethyl)-1H-indol-3-yl]ethylamine. Oxalate. 0.25 Hydrate

The oxalate 0.25 hydrate salt: mp 155-156°C; (Found: C, 54.64; H, 6.16; N, 13.34. C₂₁H₂₉N₅O₃. 1.25 (C₂H₂O₄). 0.25H₂O requires C, 54.64; H, 6.24; N, 13.56%); δ (360MHz, D₂O) 1.38 (9H, br s, 3 of CH₃); 2.91 (6H, s, 2 of CH₃); 3.21 (2H, t, J = 7.4Hz, CH₂); 3.47 (2H, t, J = 7.4Hz, CH₂); 4.31 (2H, br s, CH₂); 4.40 (2H, s, CH₂); 7.20 (1H, d, J = 8.4Hz, Ar-H); 7.34 (1H, s,Ar-H); 7.49 (1H, d, J = 8.4Hz, Ar-H); 7.63 (1H, s, Ar-H).

### EXAMPLE 82

### N-N-Dimethyl-2-[5-(5-(3-(2-t-butyloxycarbonyl amino)ethyl-1,2,4-oxadiazol)ylmethyl)-1H-indol-3-yl]ethylamine. Oxalate. 0.25 Hydrate

The oxalate 0.25 hydrate salt: mp 137-142°C; (Found: C, 56.64; H, 6.84; N, 13.69. C₂₂H₃₁N₅O₃. C₂H₂O₄. 0.2H₂O requires C, 56.84; H, 6.64; N, 13.81%).

### EXAMPLE 83

### N,N-Dimethyl-2-[5-(5-(3-aminomethyl-1,2,4-oxadiazol)ylmethyl)-1H-indol-3-yl]ethylamine. Oxalate

Prepared from Example 81 using the procedure described for Example 55. The oxalate salt mp: 109-110°C; m/e 300 (M⁺+1); δ (360MHz, D₂O) 2.92 (6H, s, 2 of CH₃); 3.24 (2H, t, J = 7.3Hz, CH₂); 3.50 (2H, t, J = 7.3Hz, CH₂); 4.37 (2H, s, CH₂); 4.48 (2H, s, CH₂); 7.23 (1H, d, J = 8.4Hz, Ar-H); 7.36 (1H, s, Ar-H); 7.53 (1H, d, J = 8.4Hz, Ar-H); 7.67 (1H s, Ar-H).

### EXAMPLE 84

### N,N-Dimethyl-2[5-(5-(3-methoxycarbonylaminoethyl-1,2,4-oxadiazol)ylmethyl)-1H-indol-3-yl]ethylamine. Oxalate

Prepared from Example 83 using the procedure described for Example 59. The oxalate salt was prepared: mp 132-133°C; (Found: C, 53.50; H, 5.62; N, 15.46. C₁₈H₂₃N₅O₃. C₂H₂O₄ requires C, 53.67; H, 5.63; N, 15.65%); δ (360MHz, D₂O) 2.90 (6H, s, 2 of CH₃); 3.21 (2H, t, J = 7.4Hz, CH₂); 3.46 (2H, t, J = 7.4Hz, CH₂); 3.66 (3H, s, CH₃); 4.29 (2H, s, CH₂); 4.40 (2H, s, CH₂); 7.19 (1H dd, J = 1.3 and 8.4Hz, Ar-H); 7.34 (1H, s, Ar-H); 7.50 (1H, d, J = 8.4Hz, Ar-H); 7.62 (1H s, Ar-H).

### EXAMPLE 85

### N,N-Dimethyl-2[5-(5-(3-N,N-dimethylaminomethyl-1-2,4-oxadiazol)ylmethyl)-1H-indol-3-yl]ethylamine. Succinate Hemihydrate

Prepared from Example 83 using the N-dimethylation procedure described for Example 2. The succinate hemihydrate salt was prepared: mp 135-137°C; (Found: C, 57.83; H, 7.19; N, 15.16. C₁₈H₂₅N₅O. 1.1 (C₄H₆O₄) 0.5H₂O requires C, 57.69; H, 7.05; N, 15.02%); δ (360MHz, D₂O) 2.86 (6H, s, 2 of CH₃); 2.94 (6H, s, 2 of CH₃); 3.26 (2H, t, J = 7.4Hz, CH₂); 3.51 (2H, t, J = 7.4Hz, CH₂); 4.38 (2H, s, CH₂); 4.51 (2H, s, CH₂); 7.25 (1H d, J = 8.4Hz, Ar-H); 7.38 (1H, s, Ar-H); 7.54 (1H, d, J = 8.4Hz, Ar-H); 7.70 (1H, s, Ar-H).

### EXAMPLE 86

### N,N-Dimethyl-2-[5-(5-(3-(2-methylsulphonylamino)ethyl-1,2,4-oxadiazol)ylmethyl)-1H-indol-3-yl]ethylamine. Oxalate

Prepared from Example 82 using the procedures described for the preparation of Examples 55 and 56. The oxalate salt was prepared: mp 163-164°C (isopropyl alcohol/ether); (Found: C, 49.72; H, 5.74; N, 14.37. C₁₈H₂₅N₅SO₃. C₂H₂O₄ requires C, 49.89; H, 5.65; N, 14.54%) δ (360MHz, D₂O) 2.90 (6H, s, 2 of CH₃); 2.92 (3H, s, CH₃); 2.96 (2H, t, J = 6.4Hz, CH₂); 3.21 (2H, t, J = 6.4Hz, CH₂); 3.44-3.49 (4H, m, 2 of CH₂); 4.40 (2H, s, CH₂); 7.21 (1H, dd, J = 1.4 and 8.5Hz, Ar-H); 7.34 (1H, s, Ar-H); 7.50 (1H, d, J = 8.5Hz, Ar-H); 7.62 (1H, s, Ar-H).

### EXAMPLE 87

### N,N-Dimethyl-2-[5-(5-(3-(2-ethoxycarbonylamino)ethyl-1,2,4-oxadiazol)ylmethyl)-1H-indol-3-yl]ethylamine. Oxalate

Prepared from Example 82 using the procedures described for the preparation of Examples 55 and 60. The oxalate salt was prepared: mp 120-124°C; (Found: C, 54.90; H, 6.29; N, 14.62. C₂₀H₂₇N₅O₃. C₂H₂O₄. 0.2H₂O requires C, 55.15; H, 6.19; N, 14.62%).

### EXAMPLE 88

### N,N-Dimethyl-2-[5-(5-(3-phenylcarboxamidomethyl-1,2,4-oxadiazol)ylmethyl)-1H-indol-3-yl]ethylamine. Succinate. Monohydrate

Benzoyl chloride (0.14ml) was added to a solution of Example 83 (0.35g, 1.2mmol) in THF (10ml) and pyridine (0.1ml), at -20°C. The mixture was allowed to warm to room temperature and stir for 16h before removing the solvents and chromatographing on silica-gel using CH₂Cl₂/EtOH/NH₃ (60:8:1) as eluant. The succinate salt was prepared: mp 72-74°C; (Found: C, 60.70; H, 6.14; N, 13.76. C₂₃H₂₅N₅O₂. 0.8 (C₄H₆O₄). 1.05H₂O requires C, 60.77; H, 6.22; N, 13.52%).

### EXAMPLE 89

### N,N-Dimethyl-2-[5-(5-(3-(2-phenylcarboxamido)ethyl-1,2,4-oxadiazol)ylmethyl)-1H-indol-3yl]ethylamine. Oxalate

Prepared from example 82 using the procedures described for the preparation of examples 55 and 88. the oxalate salt was prepared: mp 157-164°C; (Found: C, 61.56; H, 6.06; N, 13.59. C₂₄H₂₇N₅O₂. C₂H₂O₄ requires C, 61.53; H, 5.76; N, 13.80%).

### EXAMPLE 90

### N,N-Dimethyl-2-[5-(5-(3-(2-n-phenylcarbamoylamino) ethyl-1,2,4-oxadiazol)ylmethyl)-1h-indol-3-yl]ethylamine. Oxalate. 0.3 Hydrate

To a stirred solution of n,n-dimethyl-2-[5-(5-(3-(2-amino)ethyl-1,2,4-oxadiazol)ylmethyl)-1H-indol-3-yl]ethylamine (0.15g, 0.47mmol) in CH₂CL₂ (10ML) at 0°C was added phenyl isocyanate (56.0µl, 0.5mmol), dropwise. The solution was warmed to room temperature and stirred for 1H before removing the solvent under vacuum and purifying the residue by chromatography on silica-gel eluting with CH₂Cl₂/MeOH/NH₃ (40:8:1). The oxalate salt was prepared: mp 155-162°C; (Found: C, 59.10; H, 5.77; N, 15.67. C₂₄H₂₈N₆O₂. C₂H₂O₄. 0.3H₂O requires C, 59.15; H, 5.84; N, 15.92%).

### EXAMPLE 91

### N,N-Dimethyl-2-[5-(5-(3-(2-N-^{t}butylcarbamoylamino) ethyl-1,2,4-oxadiazol)ylmethyl)-1H-indol-3-yl]ethylamine. Oxalate. Hemihydrate

Prepared from Example 82 using the procedures described for the preparation of Examples 55 and 90, using ^{t} -butylisocyanate. The oxalate hemihydrate salt was prepared: mp 135-140°C; (Found: C, 56.21; H, 6.99; N, 16.27. C₂₂H₃₂N₆O₂. C₂H₂O₄. 0.5H₂O requires C, 56.35; H, 6.90; N, 16.43%).

### EXAMPLE 92

### N-Methyl-2-[5-(5-(3-amino-1,2,4-oxadiazol)ylmethyl)-1H-indol-3-yl]ethylamine. Hemisuccinate. Hemihydrate

### 1. N-Benzyl-2-[5-carboethoxymethyl-1H-indol-3-yl]ethylamine

To a solution of 2-[5-carboethoxymethyl-1H-indol-3-yl]ethylamine (2.8g, 11.37mmol) in EtOH (45ml) was added freshly distilled benzaldehyde (1.21g, 11.37mmol) and the resulting solution was stirred at room temperature for 22h. NaBH₄ (0.434g, 11.48mmol) was added portionwise over 10 min at room temperature and the resulting mixture was stirred for a further 0.5h before the solvent was removed under vacuum. The resulting residue was taken up into water (20ml) and acidified with 1N HCl (30ml). The mixture was then basified with 2N NaOH and extracted with EtOAc (4 x 70ml). The combined organic phases were washed with brine (50ml), dried and concentrated. Chromatography of the residue on silica-gel eluting with CH₂Cl₂/EtOH (90:10) gave the title-product (2.78g, 73%); δ (360MHz, CDCl₃) 1.25 (3H, t, J = 7.1Hz, CH₃); 2.98 (4H, s, 2 of CH₂); 3.68 (2H, s, CH₂); 3.81 (2H, s, CH₂); 4.14 (2H, q, J = 7.1Hz, CH₂); 6.98 (1H, d, J = 2.2Hz, Ar-H); 7.11 (1H, dd, J = 1.6 and 8.3Hz, Ar-H); 7.20-7.32 (6H, m, Ar-H); 7.49 (1H, d, J = 0.7Hz, Ar-H); 7.99 (1H, br s, indole N-H).

### 2. N-Methyl-N-benzyl-2-[5-carboethoxymethyl-1H-indol-3-yl]ethylamine.

To a stirred solution of the preceding amine (2.7g, 8.02mmol) in anhydrous DMF (80ml) was added K₂CO₃ (2.06g, 14.92mmol) followed by dimethylsulphate (0.82ml, 8.67mmol). The mixture was stirred at room temperature for 4h before adding H₂O (150ml) and extracting with EtOAc (2 x 125ml). The combined organic solutions were washed with brine (50ml), dried (Na₂SO₄) and concentrated. The residue was purified by flash chromatography on silica-gel eluting with CH₂Cl₂/EtOH (90:10). The product (1.7g, 61%) was obtained as a colourless oil; δ (250MHz, CDCl₃) 1.25 (3H, t, J = 7.1Hz, CH₃); 2.33 (3H, s, CH₃); 2.71-2.78 (2H, m, CH₂); 2.93-3.00 (2H, m, CH₂); 3.60 (2H, s, CH₂); 3.68 (2H, s, CH₂); 4.15 (2H, q, J = 7.1Hz, CH₂); 6.99 (1H, br s, Ar-H); 7.11 (1H, dd, J = 1.7 and 8.4Hz, Ar-H); 7.23-7.36 (6H, m, Ar-H); 7.41 (1H, s, Ar-H); 7.93 (1H, br s, indole N-H).

### 3. N-Methyl-2-[5-carboethoxymethyl-1H-indol-3-yl]ethylamine

A solution of the preceding benzylamine (1.6g) in ethanol (140ml) was hydrogenated at 1 atm over 10% Pd/C (1g) for 1h. The catalyst was removed by filtration, washed with EtOH (2 x 50ml) and the solvents were removed under vacuum to give the title -N-methylamine (1.12g); δ (250MHz, CDCl₃) 1.25 (3H, t, J = 7.1Hz, CH₃); 2.44 (3H, s, CH₃); 2.86-2.99 (4H, m, 2 of CH₂); 3.70 (2H, s, CH₂); 4.15 (2H, q, J = 7.1Hz, CH₂); 7.02 (1H, d, J = 2.0Hz, Ar-H); 7.12 (1H, dd, J = 1.6 and 8.4Hz, Ar-H); 7.30 (1H, d, J = 8.4Hz, Ar-H); 7.52 (1H, s, Ar-H); 8.08 (1H, br s, indole N-H).

### 4. N-Methyl-2-[5-(5-(3-amino-1,2,4-oxadiazol)yl methyl)-1H-indol-3-yl]ethylamine. Hemisuccinate. Hemihydrate

The title -compound was prepared from N-methyl-2[5-carboethoxymethyl-1H-indol-3-yl]ethylamine and hydroxyguanidine sulphate as described for Example 6. The hemisuccinate hemihydrate salt was prepared: mp 75-79°C (EtOH/Et₂O); (Found: C, 55.64; H, 6.62; N, 19.27%. C₁₄H₁₇N₅O. 0.65 (C₄H₆O₄). 0.13 (C₂H₆O). 0.6H₂O requires C, 55.50; H, 6.32; N, 19.19%); δ (360MHz, D₆-DMSO) 2.47 (3H, s, CH₃); 2.87-3.00 (4H, m, 2 of CH₂); 4.13 (2H, s, CH₂); 6.13 (2H, br s, NH₂); 7.01 (1H, dd, J = 1.5 and 8.3Hz, Ar-H); 7.19 (1H, d, J = 1.8Hz, Ar-H); 7.31 (1H, d, J = 8.3Hz, Ar-H); 7.48 (1H, s, Ar-H); 10.89 (1H, br s, indole N-H).

### EXAMPLE 93

### N,N-Dimethyl-2-[5-(5-(3-(4-^{t}-butyloxycarbonyl)piperazin-1,4-yl-1,2,4-oxadiazol)ylmethyl)-1H-indol-3-yl]ethylamine. Oxalate

Prepared from 4-^{t}-butyloxycarbonyl-piperazine amide oxime and N,N-dimethyl-2-(5-carboethoxymethyl-1H-indol-3-yl)ethylamine using the general procedure. The oxalate salt was prepared: mp 179-180°C; (Found: C, 56.48; H, 6.56; N, 14.87. C₂₄H₃₄N₆O₃. 1.2 (C₂H₂O₄) requires C, 56.36; H, 6.52; N, 14.94%); δ (360MHz, D₂O) 1.44 (9H, s, 3 of CH₃); 2.89 (6H, s, 2 of CH₃); 3.20 (2H, t, J = 7.3Hz, CH₂); 3.30-3.33 (4H, m, 2 of CH₂); 3.43-3.48 (6H, m, 3 of CH₂); 4.25 (2H, s, CH₂); 7.18 (1H, d, J = 8.3Hz, Ar-H); 7.33 (1H, s, Ar-H); 7.49 (1H, d, J = 8.3Hz, Ar-H); 7.60 (1H, s, Ar-H).

### EXAMPLE 94

### N-N-Dimethyl-2-[5-(5-(3-(4-methylsulphonyl)piperazin-1,4-yl-1,2,4-oxadiazol)ylmethyl)-1H-indol-3-yl]ethylamine. Oxalate

Prepared from Example 93 using the procedures described for Examples 55 and 56. The oxalate salt was prepared: mp 191-192°C; (Found: C, 50.56; H, 5.70; N, 15.78. C₂₀H₂₈N₆SO₃. C₂H₂O₄ requires C, 50.56; H, 5.79; N, 16.08%); δ (360MHz, D₂O) 2.89 (6H, s, 2 of CH₃); 2.96 (3H, s, CH₃); 3.17-3.25 (6H, m, 3 of CH₂); 3.40-3.49 (6H, m, 3 of CH₂); 4.24 (2H, s, CH₂); 7.16 (1H, d, J = 8.4Hz, Ar-H); 7.33 (1H, s, Ar-H); 7.48 (1H, d, J = 8.4Hz, Ar-H); 7.60 (1H, s, Ar-H).

### EXAMPLE 95

### N,N-Dimethyl-2-[5-(5-(3-(4-methoxycarbonyl)piperazin-1,4-yl-1,2,4-oxadiazol)ylmethyl)-1H-indol-3-yl]ethylamine. Oxalate. 0.2 Hydrate

Prepared from Example 93 using the procedures described for Examples 55 and 59. The oxalate salt was prepared: mp 204-205°C; (Found: C, 54.41; H, 5.81; N, 16.51. C₂₁H₂₈N₆O₃. C₂H₂O₄.0.2H₂O requires C, 54.58; H, 6.05; N, 16.60%).

### EXAMPLE 96

### N,N-Dimethyl-2-[5-(5-(3-(4-N-methylcarbamoyl) piperazin-1,4-yl-1,2,4-oxadiazol)ylmethyl)-1H-indole-3-yl]ethylamine. Oxalate. 0.4 Hydrate

Prepared from Example 93 using the procedures described for Examples 55 and 58. The oxalate salt was prepared: mp 193-194°C; (Found: C, 54.27; H, 6.24; N, 19.22. C₂₁H₂₉N₇O₂. 0.4H₂O requires C, 54.30; H, 6.30; N, 19.13%).

### EXAMPLE 97

### N,N-Dimethyl-2-[5-(5-(3-(4-acetyl)piperazin-1,4-yl-1,2,4-oxadiazol)ylmethyl)-1H-indol-3-yl]ethylamine. Oxalate. 0.3 Hydrate

The title-compound was prepared by N-acetylation with Ac₂O of the intermediate derived from Example 93 prepared using the procedure described for Example 55. The oxalate salt was prepared: mp 196-197°C; (Found: C, 56.07; H, 6.05; N, 16.91. C₂₁H₂₈N₆O₂. C₂H₂O₄. 0.3H₂O requires C, 56.16; H, 6.27; N, 17.08%).

### EXAMPLE 98

### N,N-Dimethyl-2-[5-(5-(3-(4-methylsulphonylaminomethyl) phenyl-1,2,4-oxadiazol)ylmethyl)-1H-indol-3-yl]ethylamine. Oxalate. Hemihydrate

The oxalate hemihydrate salt: mp 196-198°C; (Found: C, 54.16; H, 5.65; N, 12.51. C₂₃H₂₇N₅SO₃. C₂H₂O₄. 0.5H₂O requires C, 54.34; H, 5.47; N, 12.67%).

### EXAMPLE 99

### N,N-Dimethyl-2-[5-(5-(3-phenylsulphonylaminomethyl-1,2,4-oxadiazol)ylmethyl)-1H-indol-3-yl]ethylamine. Sesquioxalate

The sesquioxalate salt: mp 88-90°C; (Found: C, 52.16; H, 5.15; N, 12.26. C₂₂H₂₅N₅SO₃. 1.5 (C₂H₂O₄) requires C, 52.26; H, 4.91; N, 12.19%).

### EXAMPLE 100

### N,N-Dimethyl-2-[5-(5-(3-N-benzylamino-1,2,4-oxadiazol)ylmethyl)-1H-indol-3-yl]ethylamine. Oxalate. 0.25 Hydrate

Prepared from N-benzylamino amide oxime and N,N-dimethyl-2-(5-carboethoxymethyl-1H-indol-3-yl)ethylamine using the general NaOEt/EtOH procedure. The oxalate salt was prepared: mp 168-169°C; (Found: C, 61.20; H, 5.89; N, 14.93. C₂₂H₂₅N₅O. C₂H₂O₄. 0.25H₂O requires C, 61.33; H, 5.89; N, 14.90%).

### EXAMPLE 101

### N,N-Dimethyl-2-[5-(5-(3-pyrid-3-ylmethyl-1,2,4-oxadiazol)ylmethyl)-1H-indol-3-yl]ethylamine. Dihydrochloride. Monohydrate

Prepared from pyrid-3-ylmethylamideoxime and N,N-dimethyl-2-(5-carboethoxymethyl-1H-indol-3-yl)ethylamine using the general procedure. The dihydrochloride monohydrate salt: mp 150-152°C; (Found: C, 56.02; H, 6.01; N, 15.01. C₂₃H₂₃N₅O. 2HCl. 1H₂O. 0.1 (iPA) requires C, 55.81; H, 6.11; N, 15.28%).

### EXAMPLE 102

### N,N-Dimethyl-2-[5-(5-(3-(6-methoxy)pyrid-3-ylmethyl-1,2,4-oxadiazol)ylmethyl)-1H-indol-3-yl]ethylamine. Oxalate. 0.25 Hydrate.

The oxalate 0.25 hydrate salt: mp 146-148°C; (Found: C, 59.24; H, 5.70; N, 14.19. C₂₂H₂₅N₅O₂. C₂H₂O₄. 0.25H₂O requires C, 59.31; H, 5.70; N, 14.41%).

### EXAMPLE 103

### 2-[5-(5-(3-(4-Acetylaminobenzyl)-1,2,4-oxadiazol)ylmethyl)-1H-indol-3-yl]ethylamine. Oxalate

The oxalate salt: mp 140°C; (Found: C, 59.68; H, 5.68; N, 13.81. C₂₂H₂₃N₅O₂. C₂H₂O₄. 0.6 (C₂H₅OH) requires C, 59.65; H, 5.75; N, 13.85%).

### EXAMPLE 104

### 2-[5-(5-(3-(4-Methylsulphonylaminobenzyl)-1,2,4-oxadiazol)ylmethyl)-1H-indol-3-yl]ethylamine. Oxalate

The oxalate salt: mp 110-112°C, (Found: C, 52.48; H, 5.07; N, 12.77; S, 6.14. C₂₁H₂₃N₅SO₃. 1.25 (C₂H₂O₄). 0.3 (C₂H₅OH) requires C, 52.46; H, 5.01; N, 12.65; S, 5.79%).

Examples 105 and 106 were prepared by reaction of 2-[5-(2-(carboethoxy)ethyl)-1H-indol-3-yl]ethylamine with the appropriate amide oxime.

### EXAMPLE 105

### 2-[5-(2-(5-(3-(4-Acetylaminobenzyl)-1,2,4-oxadiazol)yl)ethyl)-1H-indol-3-yl]ethylamine. Oxalate

The oxalate salt: mp 121-127°C; (Found: C, 59.09; H, 5.56; N, 13.54. C₂₃H₂₅N₅O₂. 1.3 (C₂H₂O₄) requires C, 59.07; H, 5.34; N, 13.45%).

### EXAMPLE 106

### 2-[5-(2-(5-(3-(4-Methoxybenzyl)-1,2,4-oxadiazol)yl)ethyl)-1H-indol-3-yl]ethylamine. Sesquioxalate

The oxalate salt: mp 136-138°C; (Found: C, 58.70; H, 4.85; N, 11.00. C₂₂H₂₄N₄O₂. 1.5 (C₂H₂O₄) requires C, 58.94; H, 4.95; N, 11.00%).

### EXAMPLE 107

### N,N-Dimethyl-2-[5-(2-(5-Methyl-1,3-oxazol)yl)-1H-indol-3-yI]ethylamine. Sesquioxalate

### 1. 2-[5-Carboxy-1H-indol-3-yl]N,N-dimethylethylamine

A solution of 2-[5-Carboethoxy-1H-indol-3-yl]N,N-dimethylethylamine (1.4g, 5.4mmol) and lithium hydroxide (0.45g, 10.8mmol) in ethanol (40ml) was heated at 60°C for 8 hours, then stirred overnight at room temperature. The ethanol was removed in vacuo and the crude residue chromatographed (eluant 20:15:5:1 ether:ethanol:water:ammonia). The acid (0.94g, 75%) was isolated as a white solid, after precipitation with ether. δ (360MHz, D₆-DMSO) 2.86 (6H, s), 3.09 (2H, t, J = 7Hz), 3.33 (2H, t, J = 7Hz), 7.22 (1H, s), 7.46 (1H, d, J = 9Hz), 7.78 (1H, dd, 4 = 9 and 2Hz), 8.12 (1H, s).

### 2. 2-[5-Propynylcarboxamido-1H-indol-3-yl]N,N-dimethylethylamine

To a solution of 2-[5-carboxy-1H-indol-3-yl]N,N-dimethylethylamine (0.2g, 0.86mmol), 1-hydroxybenztriazole (0.14g, 1.0mmol), N-methyl morpholine (0.2ml, 1.7mmol) and propargylamine (71µl, 1.0mmol) in dichloromethane:dimethyl formamide (1:1) (25ml) at 0°C, was added 1-(3-dimethylaminopropyl)-3-ethyl-carbodiimide hydrochloride portionwise. The solution was stirred for 18 hours, then washed with water (1 x 50ml). The organic layer was separated, and the aqueous layer washed with more dichloromethane (4 x 20ml). The organic layers were combined and evaporated in vacuo. The crude residue was chromatographed (eluant 40:8:1 dichloromethane:ethanol:ammonia) to give the title compound (88mg, 38%). The aqueous phase was also evaporated and chromatographed, using 40:8:1 dichloromethane:ethanol:ammonia, to give the desired alkyne (100mg, 48%), slightly contaminated with the carbodiimide urea. δ (360MHz, CDCl₃) 2.33 (7H, m), 2.65 (2H, t, J = 7Hz), 2.92 (2H, t, J = 7Hz), 4.32 (2H, dd, J = 7 and 1Hz), 6.50 (1H, brt), 7.02 (1H, s), 7.24 (1H, d, J = 9Hz), 7.54 (1H, dd, J = 9 and 1Hz), 8.09 (1H, s), 8.79 (1H, brs).

### 3. N,N-Dimethyl-2-[5-(2-(5-methyl-1,3-oxazol)yl)-1H-indol-3-yl]ethylamine. Sesquioxalate

A solution of 2-[5-propynylcarboxamido-1H-indol-3-yl]N,N-dimethylethylamine (88mg, 0.33mmol) and mercuric acetate (7mg, 0.02mmol) in acetic acid (4ml) was refluxed for 3 hours. After this time the solution was cooled to ambient temperature and evaporated in vacuo. Saturated potassium carbonate solution (10ml) was added to the residue, and the mixture extracted with dichloromethane (5 x 20ml). The organic layers were combined, dried (MgSO₄) and evaporated. The residue was chromatographed (eluant 60:8:1 dichloromethane:ethanol:ammonia) to give the oxazole (50mg, 57%) as a pale yellow oil. The sesquioxalate salt was prepared: mp 164-166°C. (Found: C, 55.86; H, 5.52; N, 10.07, C₁₆H₁₉N₃O. 1.6 (C₂H₂O₄) requires C, 55.78, H, 5.41, N, 10.16%); δ (360MHz, D₂O) 2.49 (3H, s), 2.93 (6H, s), 3.25 (2H, t, J = 7Hz), 3.51 (2H, t, J = 7Hz), 7.30 (1H, s), 7.43 (1H, s), 7.61 (1H, d, J = 9Hz), 7.72 (1H, dd, J = 9 and 1Hz), 8.20 (1H, d, J = 1Hz). m/z (EI), 269 (M⁺), 225, 211, 181, 168, 155, 129, 115, 81, 69.

### EXAMPLE 108

### N,N-Dimethyl-2-[5-(2-(2-(5-methyl-1,3-oxazol)yl)ethyl)-1H-indol-3-yl]ethylamine. Tartrate

This was prepared according to the three step procedure described in the previous example using 2-[5-(2-(carboethoxy)ethyl)-1H-indol-3-yl]N,N-dimethylethylamine. mp 55-60°C. Formula: C₁₈H₂₃N₃O. (C₄H₆O₆). 0.6H₂O. Analysis: Calc: C, 57.66; H, 6.64; N, 9.17. Found: C, 57.94; H, 7.22; N, 8.82 δ (360MHz, D₂O) δ 2.06 (3H, s), 2.92 (6H, s), 3.15 (6H, m), 3.44 (2H, t, J = 7Hz), 4.39 (2H, s), 6.62 (1H, s), 7.09 (1H, dd, J = 8 and 2Hz), 7.30 (1H, s), 7.38 (1H, s), 7.43 (1H, d, J = 8Hz).

### EXAMPLE 109

### 4-[5-(3-Amino-1,2,4-oxadiazol-5-yl)-1H-indol-3-yl]N-methylpiperidine. Oxalate

A mixture of indole-5-carboxylic acid (1.0g, 6.2mmol), 1-methyl-4-piperidone (1.4ml, 11.2mmol) and potassium hydroxide (30ml of a 2M solution) was heated at reflux for 5 hours. The solution was then stirred overnight at room temperature after which time the solvent was evaporated in vacuo. The residue was then chromatographed (eluant 20:15:5:1 ether:ethanol:water:ammonia), to give 4-[5-carboxy-1H-indol-3-yl]N-methylpiperid-3-ene (1.0g, 63%).

To a stirred solution of methanol:ethanol (2:1, 200ml) at 0°C, was added, dropwise, thionyl chloride (1.1ml, 15mmol) under an atmosphere of nitrogen. The acid (1.0g, 3.9mmol) was added portionwise at 0°C, then the solution was allowed to warm to room temperature and stirred overnight. The solution was then heated at reflux for 2 hours, then allowed to cool to ambient temperature. The mixture was evaporated in vacuo to give the hydrochloride salts of the corresponding methyl:ethyl esters (2:1) (0.97g, 80%).

The methyl:ethyl (2:1) esters of 4-[5-carboxy-1H-indol-3-yl]N-methylpiperid-3-ene hydrochloride (0.5g, 1.6mmol) in ethanol (50ml) were hydrogenated at 30 p.s.i. for four hours in the presence of palladium on carbon (600mg). After this time the catalyst was filtered off and the ethanol evaporated in vacuo. The crude residue was chromatographed using 80:8:1 dichloromethane:ethanol:ammonia, to give the methyl:ethyl (2:1) esters of 4-[5-carboxy-indol-3-yl]N-methylpiperidine (255mg, 58%), as a viscous oil.

Sodium metal (0.19g, 8.5mmol) was added to a stirred suspension of hydroxyguanidine sulphate (0.57g, 2.1mmol) in ethanol (10ml). After 30 minutes a solution of the above esters (255mg, 0.91mmol) in ethanol (5ml) was added, and the mixture heated at reflux for 72 hours. The mixture was then cooled to ambient temperature, the solvent removed in vacuo, and the residue chromatographed (eluant 40:8:1 dichloromethane:ethanol:ammonia). The desired product, 4-[5-(3-amino-1,2,4-oxadiazol-5-yl)-1H-indol-3yl]N-methylpiperidine (12mg, 4.5%) was isolated as a viscous oil. Column fractions containing product and starting material were combined, evaporated and subjected to preparative thin layer chromatography (eluant 40:8:1 dichloromethane:ethanol:ammonia) to give the desired amino oxadiazole (10mg, 3.5%) as a viscous oil. mp 186-188°C Formula: C₁₆H₁₉N₅O. 1.2 (CO₂H)₂. 0.8H₂O. Analysis: Found: C, 52.39; H, 5.55; N, 17.09 Calc. C, 52.64; H, 5.52; N, 16.68; δ (360MHz, D₂O) δ 1.87 (2H, m), 2.22 (2H, m), 2.94 (4H, m), 3.13 (2H, m), 3.63 (2H, m), 7.18 (1H, s), 7.38 (1H, d, J = 9Hz), 7.53 (1H, dd, J = 9 and 1Hz), 7.94 (1H, d, J = 1Hz). m/z (FAB) 298 (M+1), 185, 93, 75.

### EXAMPLE 110

### 4-[5-(3-Amino-1,2,4-oxadiazol-5-ylmethyl)-1H-indol-3-yl]N-methylpiperidine. Oxalate

### Step 1: 4-[5-Carbomethoxymethyl-1H-indol-3-yl]N-methylpiperidine

A solution of 1-methyl-4 (formylmethyl)piperidine (2.3g, 16mmol) and 4-(ethoxycarbonylmethyl)phenyl hydrazine hydrochloride (3.7g, 16mmol) in methanol:water (20:1) (25ml) was stirred at room temperature for 1 hour. Polyphosphoric acid (7g) was then added and the mixture heated at reflux for 5 hours, under nitrogen. The mixture was then cooled to ambient temperature, basified with saturated sodium bicarbonate to pH9, and extracted with dichloromethane (2 x 20ml). The organic phases were combined, dried (MgSO₄) and evaporated to give a brown residue. This was chromatographed (eluant 50:8:1 dichloromethane:ethanol:ammonia) to give 4-[5-carbomethoxymethyl-1H-indol-3-yl]N-methylpiperidine (1.8g, 39%) as a yellow solid. mp 105-107°C. δ (360MHz, CDCl₃) δ 1.85 (2H, m), 2.16 (4H, m), 2.36 (3H, s), 2.81 (1H, m), 3.00 (2H, m), 3.70 (3H, s), 3.73 (2H, s), 6.97 (1H, d, J = 2Hz), 7.10 (1H, dd, J = 8 and Hz), 7.31 (1H, d, J = 8Hz), 7.53 (1H, s), 7.97 (1H, brs).

### Step 2: 4-[5-(3-Amino-1,2,4-oxadiazol-5-ylmethyl)-1H-indol-3-yl]N-methyl piperidine. Oxalate

Sodium (0.4g, 17mmol) was dissolved in ethanol (30ml), under an atmosphere of nitrogen and to the stirred solution was added hydroxyguanidine sulphate (1.53g, 5.8mmol). After stirring for 20 minutes at room temperature, the ester (0.5g, 1.7mmol) was added portionwise, and the mixture heated at reflux for 1.5 hours. The solution was cooled to room temperature, filtered, and the filtrate evaporated in vacuo. The residue was then columned, using 50:8:1 dichloromethane:ethanol:ammonia, to give the title amino oxadiazole (313mg, 60%). The oxalate salt was prepared: mp 116-120°C. Formula: C₁₇H₂₁N₅O. 1.2 (CO₂H)₂. 0.2H₂O. 0.34 (C₄H₁₀O). Analysis: Calc: C, 55.76; H, 6.12; N, 15.63. Found: C, 55.63; H, 6.31; N, 15.86. δ (360MHz, D₆-DMSO) δ 1.95 (2H, m), 2.10 (2H, m), 2.76 (3H, s), 3.05 (3H, m), 3.42 (2H, m), 4.13 (2H, s), 6.13 (2H, s), 7.00 (1H, d, J = 8Hz), 7.15 (1H, s), 7.31 (1H, d, J = 8Hz), 7.54 (1H, s), 10.90 (1H, s). m/z (EI) 311 (M⁺), 271, 156, 97, 70.

### EXAMPLE 111

### 4-[5-(3-(4-Methylsulphonylaminobenzyl)-1,2,4-oxadiazol-5-ylmethyl)-1H-indol-3-yl]N-methylpiperidine. Oxalate

mp 122-124°C; δ (360MHz, D₆-DMSO) 1.91 (2H, m), 2.08 (2H, m), 2.76 (3H, s), 3.01 (6H, m), 3.42 (2H, m), 3.99 (2H, s), 4.32 (2H, s), 7.02 (1H, dd, J = 8 and 2Hz), 7.14 (3H, m), 7.23 (2H, d, J = 9Hz), 7.31 (1H, d, J = 8Hz), 7.56 (1H, s), 10.92 (1H, s).

### EXAMPLE 112

### 4-[5-(3-(3-Pyridyl)-1,2,4-oxadiazol-5-ylmethyl)-1H-indol-3-yl]N-methylpiperidine. Oxalate

mp 94-96°C. δ (360MHz, D₆-DMSO) δ 1.92 (2H, m), 2.10 (2H, m), 2.80 (3H, s), 3.05 (3H, m), 3.45 (2H, m), 4.11 (2H, s), 4.32 (2H, s), 7.00 (1H, d,J = 8Hz), 7.16 (1H, s), 7.31 (1H, d, J = 9Hz), 7.35 (1H, m), 7.56 (1H, s), 7.70 (1H, d, J = 8Hz), 8.46 (1H, m), 8.52 (1H, s), 10.93 (1H, s).

### EXAMPLE 113

### N,N-Dimethyl-2-[5-(5-(3-pyridyl-4-yl-methyl-1,2,4-oxadiazol)ylmethyl)-1H-indol-3-yl]ethylamine. Oxalate. Monohydrate

The oxalate monohydrate salt: mp <50°C (hygroscopic); (Found: C, 58.52; H, 5.71; N, 14.60. C₂₁H₂₃N₅O. 1.05 (C₂H₂O₄).1H₂O requires C, 58.53; H, 5.76; N, 14.78%).

### EXAMPLE 114

### N,N-Dimethyl-2-[5-(5-(3-(4-^{t}-butyloxycarbonyl)ethylene-1,4-amino-1,2,4-oxadiazol)ylmethyl)-1H-indol-3-yl]ethylamine. Oxalate

The oxalate salt: mp 120-122°C; (Found: C, 55.42; H, 6.59; N, 15.91. C₂₂H₃₂N₆O₃. C₂H₂O₄ requires C, 55.59; H, 6.61; N, 16.21%).

### EXAMPLE 115

### 2-[5-(5-(3-(Carboxamido)-1,2,4-oxadiazol)ylmethyl)-1H-indol-3-yl]ethylamine. Hydrogen Oxalate

### Step 1: 2-[5-(5-(3-Ethoxycarbonyl)-1,2,4-oxadiazol)ylmethyl)-1H-indol-3-yl]-N-(tert-butyloxycarbonyl)ethylamine

To a solution of 2-[5-Carboxymethyl-1H-indol-3-yl]-N-(tert-butyloxycarbonyl)ethylamine (3g, 9.4mmol) over 4A molecular sieves (3g), in tetrahydrofuran (100ml) was added triethylamine (2.62ml, 18.8mmol). The solution was stirred under nitrogen at room temperature for 1 hour and then cooled to -10°C. Isobutylchloroformate (2.45ml, 18.8mmol) was added and after stirring at -10°C for 15 minutes, a solution of (ethoxycarbonyl) formamide oxime (1.87g, 14.2mmol) in tetrahydrofuran (10ml) was added. The reaction mixture was allowed to warm to room temperature and stirred for 2.5hours. The suspension was filtered through Hyflo and the filtrate evaporated in vacuo to give a yellow solid. This was dissolved in 1,4-dioxane (25ml) and heated to reflux over 4A molecular sieves (3g) under nitrogen for 2 days. After filtering through Hyflo, the filtrate was evaporated in vacuo and chromatographed on silica (gradient elution 3:1 petroleum ether:ethyl acetate then 1:1 petroleum ether: ethyl acetate) to give the desired ester oxadiazole as a yellow gum (1.56g, 40%). δ (360MHz, CDCl₃) 1.42 (3H, t, J = 7.1Hz), 1.43 (9H, s), 2.92 (2H, t, J = 6.8Hz), 3.44 (2H, m), 4.40 (2H, s), 4.48 (2H, q, J = 7.1Hz), 7.04 (1H, s), 7.15 (1H, dd, J = 1.6, 8.3Hz), 7.32 (1H, d, J = 8.3Hz), 7.54 (1H, s), 8.13 (1H, s). m/z (EI), 414 (M⁺), 358, 297, 212, 143, 115, 91.

### Step 2: 2-[5-(5-(3-(Carboxamido)-1,2,4-oxadiazol)ylmethyl)-1H-indol-3-yl]ethylamine

A solution of the ester oxadiazole (0. 19g, 0.46mmol) in ethanol (30ml) was cooled to 0°C (ice/water bath) and then ammonia gas was bubbled through for 15 minutes. The solvent was evaporated in vacuo to give 2-[5-(5-(3-(Carboxamido)-1,2,4-oxadiazol)ylmethyl)-1H-indol-3-yl]-N-(tert-butyloxycarbonyl)ethylamine as a yellow gum (0.17g). The crude residue was dissolved in dry dichloromethane (20ml) under nitrogen, cooled to 0°C and trifluoroacetic acid (1ml, 13.0mmol) was added. The solution was allowed to warm to room temperature and stirred under nitrogen for 3 hours. The solvent was evaporated in vacuo and the residue was azeotroped with toluene (2 x 5ml) to give a pale orange gum. This was chromatographed on silica ( eluant 20:15:5:1, ether:ethanol:water:ammonia) to give the desired amide oxadiazole as a beige gum (95mg, 72%).

The oxalate salt was prepared: m.p. 184-186°C. Formula: C₁₄H₁₅N₅O₂. 0.8 (CO₂H)₂. 0.2 (CH₃OH). Analysis: Calc: C, 52.17; H, 4.82; N, 19.25. Found: C, 51.99; H, 5.09; N, 19.25. δ (360MHz, D₆-DMSO) 2.90 (2H, t, J = 6.8Hz), 2.99 (2H, t, J = 6.8Hz), 4.44 (2H, s), 7.06 (1H, dd, J = 1.4, 8.3Hz), 7.23 (1H, s), 7.34 (1H, d, J = 8.3Hz), 7.51 (1H, s), 8.04 (1H, br s), 8.24 (1H, br s), 10.96 (1H, br s). m/z (FAB) 286 (M+1).

Examples 116-118 were prepared using the procedure described for Example 115, Step 2, using the appropriate amine.

### EXAMPLE 116

### 2-[5-(5-(3-(N-Methylcarboxamido)-1,2,4-oxadiazol)ylmethyl)-1H-indol-3-yl]ethylamine. Hydrogen Oxalate

m.p. 113-115°C; Formula: C₁₅H₁₇N₅O₂ (CO₂H)₂.0.5 (H₂O). 0.15 (Et₂O). Analysis: Calc: C, 51.62; H, 5.29; N, 17.10. Found: C, 51.58; H, 5.15; N, 17.07. δ (360MHz, D₆-DMSO) 2.76 (3H, d, J = 4.7Hz), 2.96 (2H, t, J = 7.1Hz), 3.06 (2H, t, J = 7.1Hz), 4.45 (2H, s), 7.07 (1H, dd, J = 1.4, 8.3Hz), 7.25 (1H, s), 7.35 (1H, d, J = 8.3Hz), 7.51 (1H, s), 8.85 (1H, br d, J = 4.5Hz), 11.01 (1H, br s). m/z (FAB) 300 (M+1).

### EXAMPLE 117

### 2-[5-(5-(3-(N-Pyrrolidinylcarboxamido)-1,2,4-oxadiazol)ylmethyl)-1H-indol-3-yl]ethylamine. Hydrogen Oxalate

m.p. 182-185°C δ (360MHz, D₆-DMSO) 1.86 (4H, m), 2.95 (2H, t, J = 7.4Hz), 3.05 (2H, t, J = 7.4Hz), 3.47 (2H, t, J = 6.9Hz), 3.54 (2H, t, J = 6.9Hz), 4.45 (2H, s), 7.08 (1H, d, J = 7.0Hz), 7.25 (1H, s), 7.35 (1H, d, J = 8.3Hz), 7.53 (1H, s), 11.0 (1H, br s).

### EXAMPLE 118

### 2-[5-(5-(3-(N-Azetidinylcarboxamido)-1,2,4-oxadiazol)ylmethyl)-1H-indol-3-yl]ethylamine. Hydrogen Oxalate

m.p. 114-117°C; δ (360MHz, D₆-DMSO) 2.28 (2H, quin, J = 7.8Hz), 2.95 (2H, t, J = 7.1Hz), 3.05 (2H, t, J = 7.1Hz), 4.06 (2H, t, J = 7.8Hz), 4.40 (2H, t, J = 7.8Hz), 4.44 (2H, s), 7.07 (1H, dd, J = 1.6, 8.3Hz), 7.25 (1H, s), 7.35 (1H, d, J = 8.3Hz), 7.52 (1H, s), 11.00 (1H, br s).

### EXAMPLE 119

### N-N-Dimethyl-2-[5-(5-(3-(4-phenylsulphonyl)piperazin-1,4-yl-1,2,4-oxadiazol)ylmethyl)-1H-indol-3-yl]ethylamine. Oxalate. 0.3 Hydrate

Prepared from Example 93 using the procedures described for Examples 55 and 56. The oxalate salt was prepared: mp 210-211°C; (Found: C, 54.15; H, 5.26; N, 13.81. C₂₅H₃₀N₆SO₃. 1.2 (C₂H₂O₄). 0.3H₂O requires C, 54.12; H, 5.47; N, 13.82%).

### EXAMPLE 120

### N,N-Dimethyl-2-[5-(5-(3-Pyrrolidinyloxy carbonylamino)ethyl-1,2,4-oxadiazol)ylmethyl)-1H-indol-3-yl]ethylamine. Oxalate. Hemihydrate

Prepared from Example 82 using the procedures described for the preparation of Examples 55 and 57 using pyrrolidine. The oxalate hemihydrate salt was prepared: mp 132-135°C; (Found: C, 56.63; H, 6.23; N, 16.38. C₂₂H₃₀N₆O. C₂H₂O₄. 0.5H₂O requires C, 56.57; H, 6.53; N, 16.49%).

### EXAMPLE 121

### N,N-Dimethyl-2-[5-(5-(3-(4-Methylsulphonyl)ethylene-1,4-diamino-1,2,4-oxadiazol)ylmethyl)-1H-indol-3-yl]ethylamine. Oxalate. Hemihydrate.

The oxalate hemihydrate salt: mp 178-181°C; (Found: C, 47.43; H, 5.49; N, 16.82. C₁₈H₂₆N₆SO₃. C₂H₂O₄. 0.5H₂O requires C, 47.52; H, 5.78; N, 16.62%).

### EXAMPLE 122

### N,N-Dimethyl-2-[5-(5-(3-amino-1,2,4-thiadiazol)ylmethyl)-1H-indol-3-yl]ethylamine

### 1. N,N-Dimethyl-2-[5-(4-methoxybenzyl)oxycarbonylmethyl-1H-indol-3-yl]ethylamine

To a cooled (-70°C) and stirred solution of 4-methoxybenzyl alcohol (6.3g, 45.6mmol) in dry THF (50ml) was added dropwise n-butyllithium (1.6M in hexanes; 20ml) over 0.2h. After a further 5 min at -70°C, a solution of N,N-dimethyl-2-(5-carbomethoxymethyl-1H-indol-3-yl)ethylamine (2.5g, 9.6mmol) in THF (20ml) was added dropwise over 5 min and the resulting solution allowed to warm to RT and stir for 1h. Solvents were removed under vacuum and the residue dissolved in dry toluene (100ml) and concentrated again. Water (50ml) was added to the residue and extracted into Et₂O (2 x 150ml). The combined organic solutions were washed once with brine (50ml), dried (Na₂SO₄) and concentrated. Flash chromatography of the remaining oil (CH₂Cl₂/MeOH/NH₃; 90:10:1; silica) gave the title-compound (3.1g, 89%); δ (360MHz, CDCl₃) 2.33 (6H, s, NMe₂); 2.59-2.65 (2H, m, CH₂); 2.87-2.94 (2H, m, CH₂); 3.74 (2H, s, Ar-CH₂); 3.80 (3H, s, OMe); 5.07 (2H, s Ar-CH₂-O); 6.83-6.88 (2H, m, Ar-H); 6.99 (1H, d, J = 2.5Hz, Ar-H); 7.10 (1H, dd, J = 1.7 and 8.4Hz, Ar-H); 7.23-7.29 (3H, m, Ar-H); 7.48 (1H, s, Ar-H); 8.04 (1H, brs, indole-NH).

### 2. N,N-Dimethyl-2-[5-(4-methoxybenzyl)oxy carbonylmethyl-1-tert-butoxycarbonyl-indol-3-yl]ethylamine

To a solution of the preceding ester (3.4g, 9.27mmol) in dry CH₃CN (25ml) was added di-tert-butyl dicarbonate (2.63g, 12.06mmol) followed by 4-DAMP (0.11g). After stirring at RT for 1h, solvents were removed under vacuum and the residue purified by flash chromatography (silica, CH₂Cl₂/MeOH; 95:5) to give the title-product (3.33g, 77%); δ (360MHz, CDCl₃) 1.66 (9H, s, 3 of CH₃); 2.32 (6H, s, N(Me)₂); 2.58-2.65 (2H, m, CH₂); 2.80-2.88 (2H, m, CH₂); 3.74 (2H, s, Ar-CH₂-CO); 3.80 (3H, s, OMe); 5.07 (2H, s,Ar-CH₂-O); 6.84-6.89 (2H, m, Ar-H); 7.18-7.28 (3H, m, Ar-H); 7.38 (1H, s, Ar-H); 7.41 (1H, d, J = 1.2Hz, Ar-H); 8.03 (1H, br d, J = 8.1Hz, Ar-H).

### 3. N,N-Dimethyl-2-[5-(5-(3-amino-1,2,4-thiadiazol)ylmethyl)-1H-indol-3-yl]ethylamine

To a solution of the preceding ester (0.3g, 0.64mmol) in dry DMF (4ml) was added NaH (64mg of a 60% dispersion in oil) and the mixture stirred at RT for 15 min before adding a solution of 3-amino-5-chloro-1,2,4-thiadiazole (0.17g, 1.27mmol) in dry DMF (1ml). After 1h, water (50ml) was added and products were extracted into CH₂Cl₂ (2 x 70ml). The residue obtained on removal of solvents was chromatographed on silica-gel eluting with CH₂Cl₂/MeOH (10%) to give 64mg (17%) of a white foam; δ (250MHz, CDCl₃) 1.65 (9H, s, 3 of CH₃); 2.32 (6H, s, N(Me)₂); 2.57-2.64 (2H, m, CH₂); 2.78-2.84 (2H, m, CH₂); 3.78 (3H, s, OMe); 4.83 (2H, s, NH₂); 5.08 (1H, d, J = 11.9Hz, Ar-CH₂-O); 5.19 (1H, d, J = 11.9Hz, Ar-CH₂-O); 5.35 (1H, s, Ar-CH-CO); 6.79-6.84 (2H, m, Ar-H); 7.16-7.21 (2H, m, Ar-H); 7.28 (1H, dd, J = 1.9 and 8.7Hz, Ar-H); 7.41 (1H, s, Ar-H); 7.50 (1H, d, J = 1.7Hz, Ar-H); 8.06 (1H, d, J = 8.3Hz, Ar-H).

A solution of the preceding product (5mg) in CH₂Cl₂ (0.8ml), H₂O (30µl) and TFA (130µl) was stirred at RT for 1h. Solvents were removed under vacuum and the remaining residue was dissolved in dry toluene (1.5ml) and MeOH (0.3ml) and concentrated again. The residue was dissolved in MeOH and refluxed for 0.5min. The solvent was removed under vacuum and the residue purified by preparative thick layer chromatography (silica-gel, CH₂Cl₂/MeOH/NH₃; 80:20:1.5) to give N,N-dimethyl-2-[5-(5-(3-amino-1,2,4-thiadiazol)ylmethyl)-1H-indol-3-yl]ethylamine (1mg); δ (250MHz, CDCl₃) 2.50 (6H, s, N(Me)₂); 2.80-2.88 (2H, m, CH₂); 3.05-3.10 (2H, m, CH₂); 4.36 (2H, s, CH₂); 4.88 (2H, br s, NH₂); 7.09 (1H, d, J = 2.5Hz, Ar-H); 7.13 (1H, dd, J = 1.7 and 8.4Hz, Ar-H); 7.34 (1H, d, J = 8.4Hz, Ar-H); 7.55 (1H, s, Ar-H); 8.08 (1H, br s, indole-NH).

### EXAMPLE 123

### Tablet Preparation

Tablets containing 1.0, 2.0, 25.0, 26.0, 50.0 and 100.0mg, respectively of:
N,N-Dimethyl-2-[5-[5-(3-Amino-1,2,4-oxadiazol)ylmethyl]-1H-indol-3-yl]ethylamine. Hemisuccinate Hydrate
2-[5-[3-(5-Benzyl-1,2,4-oxadiazol)yl]-1H-indol-3-yl]ethylamine. Hydrogen Maleate
N,N-Dimethyl-2-[5-(2-(5-Methyl-1,3-oxazol)yl)-1H-indol-3-yl]ethylamine. Sesquioxalate
4-[5-(3-(4-Methylsulphonylaminobenzyl)-1,2,4-oxadiazol-5-ylmethyl)-1H-indol-3-yl]N-methylpiperidine. Oxalate

| TABLE FOR DOSES CONTAINING FROM 1-25MG OF THE ACTIVE COMPOUND | | | |
|---|---|---|---|
| | Amount-mg | | |
| Active Compound | 1.0 | 2.0 | 25.0 |
| Microcrystalline cellulose | 49.25 | 48.75 | 37.25 |
| Modified food corn starch | 49.25 | 48.75 | 37.25 |
| Magnesium stearate | 0.50 | 0.50 | 0.50 |

| TABLE FOR DOSES CONTAINING FROM 26-100MG OF THE ACTIVE COMPOUND | | | |
|---|---|---|---|
| | Amount-mg | | |
| Active Compound | 26.0 | 50.0 | 100.0 |
| Microcrystalline cellulose | 52.0 | 100.0 | 200.0 |
| Modified food corn starch | 2.21 | 4.25 | 8.5 |
| Magnesium stearate | 0.39 | 0.75 | 1.5 |

All of the active compound, cellulose, and a portion of the corn starch are mixed and granulated to 10% corn starch paste. The resulting granulation is sieved, dried and blended with the remainder of the corn starch and the magnesium stearate. The resulting granulation is then compressed into tablets containing 1.0mg, 2.0mg, 25.0mg, 26.0mg, 50.0mg and 100mg of the active ingredient per tablet.

## Claims (Claims for the following Contracting State(s): AT, BE, CH, DE, DK, FR, GB, IT, LI, LU, NL, SE)

1. A compound of formula I, or a salt thereof: 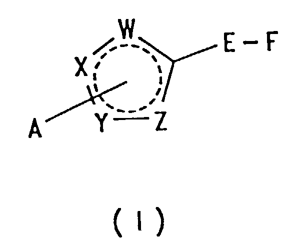 wherein the broken circle represents two non-adjacent double bonds in any position in the five-membered ring;
the five-membered ring containing the substituents W to Z represents a 1,2,4-oxadiazole, 1,3,4-oxadiazole, 1,2,4-thiadiazole, 1,3,4-thiadiazole, 1,3-oxazole or 1,3-thiazole ring;
A represents methyl, methoxymethyl, aminomethyl, dimethylaminomethyl, acetylaminomethyl, benzoylaminomethyl, t-butoxy-carbonylaminomethyl, methylsulphonylaminomethyl, phenylsulphonylaminomethyl, aminocarbonylmethyl, ethyl, aminoethyl, acetylaminoethyl, benzoylaminoethyl, methoxycarbonylaminoethyl, ethoxycarbonylaminoethyl, t-butoxycarbonylaminoethyl, methylsulphonylaminoethyl, aminocarbonylaminoethyl, methylaminocarbonylaminoethyl, t-butylaminocarbonylaminoethyl, phenylaminocarbonyl-aminoethyl, pyrrolidylcarbonylaminoethyl, cyclopropyl, phenyl, methylsulphonylaminophenyl, aminocarbonylphenyl, methylaminocarbonylphenyl, methylsulphonylaminomethylphenyl, aminosulphonylmethylphenyl, methylaminosulphonyl-methylphenyl, dimethylaminosulphonylmethylphenyl, naphthyl, benzyl, diphenylmethyl, trifluoromethylbenzyl, methoxybenzyl, acetylaminobenzyl, methylsulphonylamino-benzyl, aminocarbonylaminobenzyl, aminocarbonylbenzyl, methylaminocarbonylbenzyl, methylsulphonylbenzyl, methylaminosulphonylbenzyl, phenethyl, phenylpropyl, acetylpiperazinyl, methoxycarbonylpiperazinyl, t-butoxycarbonylpiperazinyl, methylaminocarbonyl-piperazinyl, methylsulphonylpiperazinyl, phenylsulphonyl-piperazinyl, pyridylmethyl, methoxypyridylmethyl, amino, methylamino, benzylamino, dimethylamino, t-butoxy-carbonylaminoethylamino, methylsulphonylaminoethylamino, aminocarbonyl, methylaminocarbonyl, azetidinylcarbonyl or pyrrolidylcarbonyl;
E represents a bond or a straight or branched alkylene chain containing from 1 to 4 carbon atoms;
F represents a group of formula 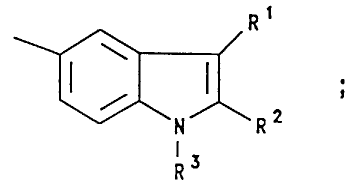
R¹ represents aminoethyl, N-methylaminoethyl, N,N-dimethylaminoethyl or 1-methyl-4-piperidyl; and
R² and R³ independently represent hydrogen or C₁₋₆ alkyl.

2. A compound as claimed in Claim 1 represented by formula IIA, and salts thereof: 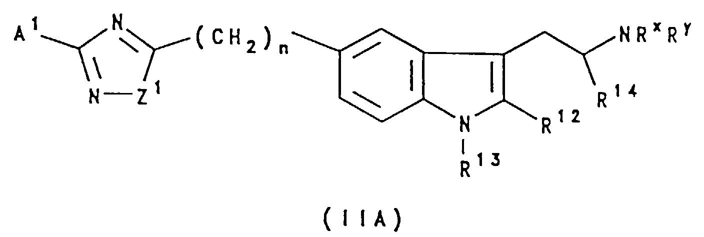 wherein
Z¹ represents oxygen or sulphur;
n is zero, 1, 2 or 3;
A¹ corresponds to the group A as defined in Claim 1;
R¹², R¹³ and R¹⁴ each represents hydrogen; and
R^{x} and R^{y} independently represent hydrogen or methyl.

3. A compound as claimed in Claim 1 represented by formula IIB, and salts thereof: 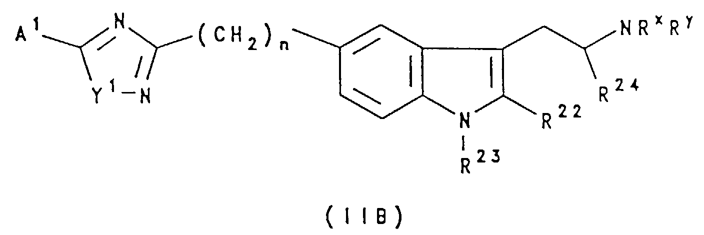 wherein
Y¹ represents oxygen or sulphur;
n is zero, 1, 2 or 3;
A¹ is as defined in Claim 2;
R²², R²³ and R²⁴ each represents hydrogen; and
R^{x} and R^{y} independently represent hydrogen or methyl.

4. A compound as claimed in Claim 1 represented by formula IIC, and salts thereof: 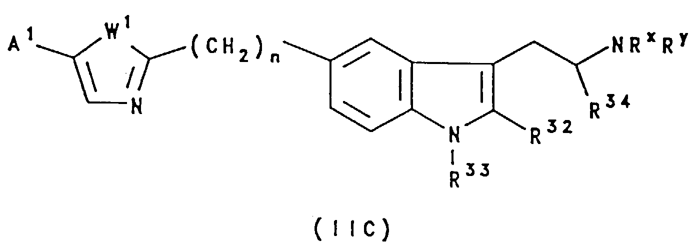 wherein
W¹ represents oxygen or sulphur;
n is zero, 1, 2 or 3;
A¹ is as defined in Claim 2;
R³², R³³ and R³⁴ each represents hydrogen; and
R^{x} and R^{y} independently represent hydrogen or methyl.

5. A compound as claimed in Claim 1 represented by formula IID, and salts thereof: 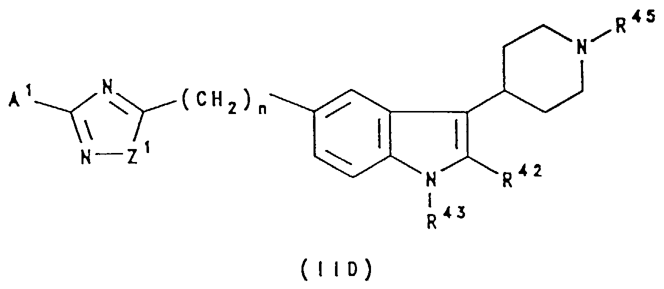 wherein
Z¹ represents oxygen or sulphur;
n is zero, 1, 2 or 3;
A¹ is as defined in Claim 2;
R⁴² and R⁴³ each represents hydrogen; and
R⁴⁵ represents methyl.

6. A compound as claimed in Claim 1 selected from:
2-[5-(3-benzyl-1,2,4-oxadiazol-5-yl)-1H-indol-3-yl]ethylamine;
2-[5-(3-methyl-1,2,4-oxadiazol-5-yl)-1H-indol-3-yl]ethylamine;
N,N-dimethyl-2-[5-(3-benzyl-1,2,4-oxadiazol-5-yl)-1H-indol-3-yl]ethylamine;
2-[5-(3-benzyl-1,2,4-oxadiazol-5-ylmethyl)-1H-indol-3-yl]ethylamine;
2-[5-(3-methyl-1,2,4-oxadiazol-5-ylmethyl)-1H-indol-3-yl]ethylamine;
2-[5-(3-amino-1,2,4-oxadiazol-5-ylmethyl)-1H-indol-3-yl]ethylamine;
2-[5-(3-phenyl-1,2,4-oxadiazol-5-ylmethyl)-1H-indol-3-yl]ethylamine;
2-[5-[3-(2-methoxybenzyl)-1,2,4-oxadiazol-5-ylmethyl]-1H-indol-3-yl]ethylamine;
N,N-dimethyl-2-[5-(3-benzyl-1,2,4-oxadiazol-5-ylmethyl)-1H-indol-3-yl]ethylamine;
2-[5-[2-(3-benzyl-1,2,4-oxadiazol-5-yl)ethyl]-1H-indol-3-yl]ethylamine;
N,N-dimethyl-2-[5-(3-methyl-1,2,4-oxadiazol-5-ylmethyl)-1H-indol-3-yl]ethylamine;
2-[5-(3-diphenylmethyl-1,2,4-oxadiazol-5-yl)-1H-indol-3-yl]ethylamine;
2-[5-(3-phenyl-1,2,4-oxadiazol-5-yl)-1H-indol-3-yl]ethylamine;
2-[5-[3-(2-methoxybenzyl)-1,2,4-oxadiazol-5-yl]-1H-indol-3-yl]ethylamine;
2-[5-[3-(3-benzyl-1,2,4-oxadiazol-5-yl)propyl]-1H-indol-3-yl]ethylamine;
2-[5-(3-phenethyl-1,2,4-oxadiazol-5-yl)-1H-indol-3-yl]ethylamine;
2-[5-(5-benzyl-1,2,4-oxadiazol-3-yl)-1H-indol-3-yl]ethylamine;
2-[5-(5-benzyl-1,2,4-oxadiazol-3-ylmethyl)-1H-indol-3-yl]ethylamine;
N,N-dimethyl-2-[5-[3-(2-methoxybenzyl)-1,2,4-oxadiazol-5-yl]-1H-indol-3-yl]ethylamine;
N,N-dimethyl-2-[5-[2-(3-benzyl-1,2,4-oxadiazol-5-yl)ethyl]-1H-indol-3-yl]ethylamine;
2-[5-[3-(1-naphthyl)-1,2,4-oxadiazol-5-ylmethyl]-1H-indol-3-yl]ethylamine;
2-[5-[3-(3-methyl-1,2,4-oxadiazol-5-yl)propyl]-1H-indol-3-yl]ethylamine;
2-[5-[3-(3-cyclopropyl-1,2,4-oxadiazol-5-yl)propyl]-1H-indol-3-yl]ethylamine;
2-[5-[3-(3-methoxybenzyl)-1,2,4-oxadiazol-5-yl]-1H-indol-3-yl]ethylamine;
2-[5-[3-(4-methoxybenzyl)-1,2,4-oxadiazol-5-yl]-1H-indol-3-yl]ethylamine;
2-[5-[3-(4-acetylaminobenzyl)-1,2,4-oxadiazol-5-yl]-1H-indol-3-yl]ethylamine;
2-[5-[3-(4-methylsulphonylaminobenzyl)-1,2,4-oxadiazol-5-yl]-1H-indol-3-yl]ethylamine;
2-[5-[3-(3-phenylpropyl)-1,2,4-oxadiazol-5-yl]-1H-indol-3-yl]ethylamine;
2-[5-(3-cyclopropyl-1,2,4-oxadiazol-5-yl)-1H-indol-3-yl]ethylamine;
2-[5-(3-ethyl-1,2,4-oxadiazol-5-yl)-1H-indol-3-yl]ethylamine;
2-[5-[3-(4-trifluoromethylbenzyl)-1,2,4-oxadiazol-5-yl]-1H-indol-3-yl]ethylamine;
N,N-dimethyl-2-[5-[3-(4-acetylaminobenzyl)-1,2,4-oxadiazol-5-yl]-1H-indol-3-yl]ethylamine;
N,N-dimethyl-2-[5-[3-(4-methylsulphonylaminobenzyl)-1,2,4-oxadiazol-5-yl]-1H-indol-3-yl]ethylamine;
N,N-dimethyl-2-[5-(3-amino-1,2,4-oxadiazol-5-ylmethyl)-1H-indol-3-yl]ethylamine;
2-[5-[2-(3-amino-1,2,4-oxadiazol-5-yl)ethyl]-1H-indol-3-yl]ethylamine;
2-[5-[2-(3-dimethylamino-1,2,4-oxadiazol-5-yl)ethyl]-1H-indol-3-yl]ethylamine;
2-[5-(5-methyl-1,2,4-oxadiazol-3-yl)-1H-indol-3-yl]ethylamine;
2-[5-(5-methyl-1,2,4-oxadiazol-3-ylmethyl)-1H-indol-3-yl]ethylamine;
N,N-dimethyl-2-[5-(5-benzyl-1,2,4-oxadiazol-3-ylmethyl)-1H-indol-3-yl]ethylamine;
2-[5-(3-methoxymethyl-1,2,4-oxadiazol-5-yl)-1H-indol-3-yl]ethylamine;
2-[5-[3-(4-methylaminocarbonylbenzyl)-1,2,4-oxadiazol-5-yl]-1H-indol-3-yl]ethylamine;
2-[5-[3-(4-methylaminocarbonylphenyl)-1,2,4-oxadiazol-5-yl]-1H-indol-3-yl]ethylamine;
2-[5-[3-(4-methylaminosulphonylbenzyl)-1,2,4-oxadiazol-5-yl]-1H-indol-3-yl]ethylamine;
2-[5-[3-(4-methylsulphonylbenzyl)-1,2,4-oxadiazol-5-yl]-1H-indol-3-yl]ethylamine;
2-[5-[3-(3-methylsulphonylaminobenzyl)-1,2,4-oxadiazol-5-yl]-1H-indol-3-yl]ethylamine;
2-[5-[3-(4-aminocarbonylaminobenzyl)-1,2,4-oxadiazol-5-yl]-1H-indol-3-yl]ethylamine;
2-[5-(3-amino-1,2,4-oxadiazol-5-yl)-1H-indol-3-yl]ethylamine;
2-[5-(3-acetylaminomethyl-1,2,4-oxadiazol-5-yl)-1H-indol-3-yl]ethylamine;
2-[5-[3-(2-acetylaminoethyl)-1,2,4-oxadiazol-5-yl]-1H-indol-3-yl]ethylamine;
2-[5-(3-aminomethyl-1,2,4-oxadiazol-5-yl)-1H-indol-3-yl]ethylamine;
N,N-dimethyl-2-[5-(3-amino-1,2,4-oxadiazol-5-yl)-1H-indol-3-yl]ethylamine;
N,N-dimethyl-2-[5-(3-acetylaminomethyl-1,2,4-oxadiazol-5-yl)-1H-indol-3-yl]ethylamine;
N,N-dimethyl-2-[5-[3-(2-acetylaminoethyl)-1,2,4-oxadiazol-5-yl]-1H-indol-3-yl]ethylamine;
N,N-dimethyl-2-[5-[3-(4-aminocarbonylaminobenzyl)-1,2,4-oxadiazol-5-yl]-1H-indol-3-yl]ethylamine;
N,N-dimethyl-2-[5-[3-(2-(t-butoxycarbonylamino)ethyl)-1,2,4-oxadiazol-5-yl]-1H-indol-3-yl]ethylamine;
N,N-dimethyl-2-[5-[3-(4-methylaminocarbonylbenzyl)-1,2,4-oxadiazol-5-yl]-1H-indol-3-yl]ethylamine;
N,N-dimethyl-2-[5-[3-(2-aminoethyl)-1,2,4-oxadiazol-5-yl]-1H-indol-3-yl]ethylamine;
N,N-dimethyl-2-[5-[3-(2-methylsulphonylaminoethyl)-1,2,4-oxadiazol-5-yl]-1H-indol-3-yl]ethylamine;
N,N-dimethyl-2-[5-[3-(2-aminocarbonylaminoethyl)-1,2,4-oxadiazol-5-yl]-1H-indol-3-yl]ethylamine;
N,N-dimethyl-2-[5-[3-(2-methylaminocarbonylaminoethyl)-1,2,4-oxadiazol-5-yl]-1H-indol-3-yl]ethylamine;
N,N-dimethyl-2-[5-[3-(2-methylaminocarbonylaminoethyl)-1,2,4-oxadiazol-5-yl]-1H-indol-3-yl]ethylamine;
N,N-dimethyl-2-[5-[3-(2-methoxycarbonylaminoethyl)-1,2,4-oxadiazol-5-yl]-1H-indol-3-yl]ethylamine;
N,N-dimethyl-2-[5-[3-(2-ethoxycarbonylaminoethyl)1,2,4-oxadiazol-5-yl]-1H-indol-3-yl]ethylamine;
N,N-dimethyl-2-[5-[2-(3-amino-1,2,4-oxadiazol-5-yl)ethyl]-1H-indol-3-yl]ethylamine;
N,N-dimethyl-2-[5-(3-methylamino-1,2,4-oxadiazol-5-ylmethyl)-1H-indol-3-yl]ethylamine;
N,N-dimethyl-2-[5-[3-(4-aminocarbonylbenzyl)-1,2,4-oxadiazol-5-ylmethyl]-1H-indol-3-yl]ethylamine;
N,N-dimethyl-2-[5-[3-(4-acetylaminobenzyl)-1,2,4-oxadiazol-5-ylmethyl]-1H-indol-3-yl]ethylamine;
N,N-dimethyl-2-[5-[3-(4-methylaminosulphonylbenzyl)-1,2,4-oxadiazol-5-ylmethyl]-1H-indol-3-yl]ethylamine;
N,N-dimethyl-2-[5-[3-(4-aminocarbonylaminobenzyl)-1,2,4-oxadiazol-5-ylmethyl]-1H-indol-3-yl]ethylamine;
N,N-dimethyl-2-[5-[3-(4-methylsulphonylaminobenzyl)-1,2,4-oxadiazol-5-ylmethyl]-1H-indol-3-yl]ethylamine;
N,N-dimethyl-2-[5-[3-(4-methylaminocarbonylphenyl)-1,2,4-oxadiazol-5-ylmethyl]-1H-indol-3-yl]ethylamine;
N,N-dimethyl-2-[5-(3-acetylaminomethyl-1,2,4-oxadiazol-5-ylmethyl)-1H-indol-3-yl]ethylamine;
N,N-dimethyl-2-[5-(3-methylsulphonylaminomethyl-1,2,4-oxadiazol-5-ylmethyl)-1H-indol-3-yl]ethylamine;
N,N-dimethyl-2-[5-(3-aminocarbonylmethyl-1,2,4-oxadiazol-5-ylmethyl)-1H-indol-3-yl]ethylamine;
N,N-dimethyl-2-[5-[3-(3-methylsulphonylaminobenzyl)-1,2,4-oxadiazol-5-ylmethyl]-1H-indol-3-yl]ethylamine;
N,N-dimethyl-2-[5-[3-(3-acetylaminobenzyl)-1,2,4-oxadiazol-5-ylmethyl]-1H-indol-3-yl]ethylamine;
N,N-dimethyl-2-[5-[3-(4-aminocarbonylphenyl)-1,2,4-oxadiazol-5-ylmethyl]-1H-indol-3-yl]ethylamine;
N,N-dimethyl-2-[5-[3-(3-aminocarbonylphenyl)-1,2,4-oxadiazol-5-ylmethyl]-1H-indol-3-yl]ethylamine;
N,N-dimethyl-2-[5-[3-(4-methylsulphonylaminophenyl)-1,2,4-oxadiazol-5-ylmethyl]-1H-indol-3-yl]ethylamine;
N,N-dimethyl-2-[5-[3-(4-methylaminosulphonylmethylphenyl)-1,2,4-oxadiazol-5-ylmethyl]-1H-indol-3-yl]ethylamine;
N,N-dimethyl-2-[5-[3-(3-methylaminosulphonylmethylphenyl)-1,2,4-oxadiazol-5-ylmethyl]-1H-indol-3-yl]ethylamine;
N,N-dimethyl-2-[5-[3-(4-aminosulphonylmethylphenyl)-1,2,4-oxadiazol-5-ylmethyl]-1H-indol-3-yl]ethylamine;
N,N-dimethyl-2-[5-[3-(4-dimethylaminosulphonylmethylphenyl)-1,2,4-oxadiazol-5-ylmethyl]-1H-indol-3-yl]ethylamine;
N,N-dimethyl-2-[5-[3-(t-butoxycarbonylamino)methyl-1,2,4-oxadiazol-5-ylmethyl]-1H-indol-3-yl]ethylamine;
N,N-dimethyl-2-[5-[3-(2-(t-butoxycarbonylamino)ethyl)-1,2,4-oxadiazol-5-ylmethyl]-1H-indol-3-yl]ethylamine;
N,N-dimethyl-2-[5-(3-aminomethyl-1,2,4-oxadiazol-5-ylmethyl)-1H-indol-3-yl]ethylamine;
N,N-dimethyl-2-[5-(3-methoxycarbonylaminomethyl-1,2,4-oxadiazol-5-ylmethyl)-1H-indol-3-yl]ethylamine;
N,N-dimethyl-2-[5-(3-dimethylaminomethyl-1,2,4-oxadiazol-5-ylmethyl)-1H-indol-3-yl]ethylamine;
N,N-dimethyl-2-[5-[3-(2-methylsulphonylaminoethyl)-1,2,4-oxadiazol-5-ylmethyl]-1H-indol-3-yl]ethylamine;
N,N-dimethyl-2-[5-[3-(2-ethoxycarbonylaminoethyl)-1,2,4-oxadiazol-5-ylmethyl]-1H-indol-3-yl]ethylamine;
N,N-dimethyl-2-[5-(3-benzoylaminomethyl-1,2,4-oxadiazol-5-ylmethyl)-1H-indol-3-yl]ethylamine;
N,N-dimethyl-2-[5-[3-(2-benzoylaminoethyl)-1,2,4-oxadiazol-5-ylmethyl]-1H-indol-3-yl]ethylamine;
N,N-dimethyl-2-[5-[3-(2-phenylaminocarbonylaminoethyl)-1,2,4-oxadiazol-5-ylmethyl]-1H-indol-3-yl]ethylamine;
N,N-dimethyl-2-[5-[3-(2-(t-butylaminocarbonyl-amino)ethyl)-1,2,4-oxadiazol-5-ylmethyl]-1H-indol-3-yl]ethylamine;
N-methyl-2-[5-(3-amino-1,2,4-oxadiazol-5-ylmethyl)-1H-indol-3-yl]ethylamine;
N,N-dimethyl-2-[5-[3-(4-(t-butoxycarbonyl)piperazin-1-yl)-1,2,4-oxadiazol-5-ylmethyl]-1H-indol-3-yl]ethylamine;
N,N-dimethyl-2-[5-[3-(4-methylsulphonylpiperazin-1-yl)-1,2,4-oxadiazol-5-ylmethyl]-1H-indol-3-yl]ethylamine;
N,N-dimethyl-2-[5-[3-(4-methoxycarbonylpiperazin-1-yl)-1,2,4-oxadiazol-5-ylmethyl]-1H-indol-3-yl]ethylamine;
N,N-dimethyl-2-[5-[3-(4-methylaminocarbonylpiperazin-1-yl)-1,2,4-oxadiazol-5-ylmethyl]-1H-indol-3-yl]ethylamine;
N,N-dimethyl-2-[5-[3-(4-acetylpiperazin-1-yl)-1,2,4-oxadiazol-5-ylmethyl]-1H-indol-3-yl]ethylamine;
N,N-dimethyl-2-[5-[3-(4-methylsulphonylaminomethylphenyl)-1,2,4-oxadiazol-5-yl-methyl]-1H-indol-3-yl]ethylamine;
N,N-dimethyl-2-[5-(3-phenylsulphonylaminomethyl-1,2,4-oxadiazol-5-ylmethyl)-1H-indol-3-yl]ethylamine;
N,N-dimethyl-2-[5-(3-benzylamino-1,2,4-oxadiazol-5-ylmethyl)-1H-indol-3-yl]ethylamine;
N,N-dimethyl-2-[5-[3-(3-pyridyl)methyl-1,2,4-oxadiazol-5-ylmethyl]-1H-indol-3-yl]ethylamine;
N,N-dimethyl-2-[5-[3-(2-methoxypyrid-5-yl)methyl-1,2,4-oxadiazol-5-ylmethyl]-1H-indol-3-yl]ethylamine;
2-[5-[3-(4-acetylaminobenzyl)-1,2,4-oxadiazol-5-ylmethyl]-1H-indol-3-yl]ethylamine;
2-[5-[3-(4-methylsulphonylaminobenzyl)-1,2,4-oxadiazol-5-ylmethyl]-1H-indol-3-yl]ethylamine;
2-[5-[2-[3-(4-acetylaminobenzyl)-1,2,4-oxadiazol-5-yl]ethyl]-1H-indol-3-yl]ethylamine;
2-[5-[2-[3-(4-methoxybenzyl)-1,2,4-oxadiazol-5-yl]ethyl]-1H-indol-3-yl]ethylamine;
N,N-dimethyl-2-[5-(5-methyl-1,3-oxazol-2-yl)-1H-indol-3-yl]ethylamine;
N,N-dimethyl-2-[5-[2-(5-methyl-1,3-oxazol-2-yl)ethyl]-1H-indol-3-yl]ethylamine;
1-methyl-4-[5-(3-amino-1,2,4-oxadiazol-5-yl)-1H-indol-3-yl]piperidine;
1-methyl-4-[5-(3-amino-1,2,4-oxadiazol-5-ylmethyl)-1H-indol-3-yl]piperidine;
1-methyl-4-[5-[3-(4-methylsulphonylaminobenzyl)-1,2,4-oxadiazol-5-ylmethyl]-1H-indol-3-yl]piperidine;
1-methyl-4-[5-[3-(3-pyridyl)methyl-1,2,4-oxadiazol-5-ylmethyl]-1H-indol-3-yl]piperidine;
N,N-dimethyl-2-[5-[3-(4-pyridyl)methyl-1,2,4-oxadiazol-5-ylmethyl]-1H-indol-3-yl]ethylamine;
N,N-dimethyl-2-[5-[3-(2-(t-butoxycarbonylamino)ethyl)-amino-1,2,4-oxadiazol-5-ylmethyl]-1H-indol-3-yl]ethylamine;
2 -[5-(3-aminocarbonyl-1,2,4-oxadiazol-5-ylmethyl)-1H-indol-3-yl]ethylamine;
2-[5-(3-methylaminocarbonyl-1,2,4-oxadiazol-5-ylmethyl)-1H-indol-3-yl]ethylamine;
2-[5-[3-(pyrrolid-1-yl)carbonyl-1,2,4-oxadiazol-5-ylmethyl]-1H-indol-3-yl]ethylamine;
2-[5-[3-(azetidin-1-yl)carbonyl-1,2,4-oxadiazol-5-ylmethyl]1H-indol-3-yl]ethylamine;
N,N-dimethyl-2-[5-[3-(4-phenylsulphonylpiperazin-1-yl)-1,2,4-oxadiazol-5-ylmethyl]-1H-indol-3-yl]ethylamine;
N,N-dimethyl-2-[5-[3-(2-(pyrrolid-1-ylcarbonyl-amino)ethyl)-1,2,4-oxadiazol-5-ylmethyl]-1H-indol-3-yl]ethylamine;
N,N-dimethyl-2-[5-[3-(2-methylsulphonylaminoethyl)amino-1,2,4-oxadiazol-5-ylmethyl]-1H-indol-3-yl]ethylamine;
N,N-dimethyl-2-[5-(3-amino-1,4-thiadiazol-5-ylmethyl)-1H-indol-3-yl]ethylamine;
and salts thereof.

7. A pharmaceutical composition comprising a compound as claimed in any one of the preceding Claims in association with a pharmaceutically acceptable carrier or excipient.

8. A compound as claimed in any one of Claims 1 to 6 for use in therapy.

9. The use of a compound as claimed in any one of Claims 1 to 6 for the manufacture of a medicament for the treatment and/or prevention of clinical conditions for which a selective agonist of 5-HT₁-like receptors is indicated.

10. A process for the preparation of a compound as claimed in any one of Claims 1 to 6 which comprises:
(A) reacting a reactive derivative of a carboxylic acid of formula R^{c}-CO₂H with a compound either of formula III or of formula IV, or a salt thereof: 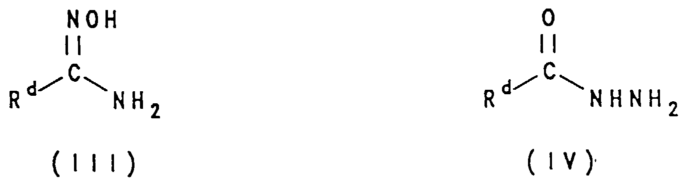 wherein one of R^{c} and R^{d} is a group of formula A, and the other is a group of formula -E-F, as defined in Claim 1; or
(B) cyclisation of a compound of formula XI: 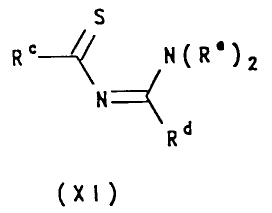 wherein R^{c} and R^{d} are as defined above, and R^{e} is hydrogen or an alkyl group; or
(C) cycloaddition of a nitrile sulphide R^{c}-C≡N⁺-S. with a nitrile of formula R^{d}-CN where R^{c} and R^{d} are as defined above; or
(D) dehydration of a thiosemicarbazide of formula R^{c}CSNHNHCONR^{s}R^{t}, where R^{c} is as defined above and R^{s} and R^{t} are hydrogen or an alkyl group; followed by attachment of the Rd group by conventional means; or
(E) reaction of an amide or thioamide of formula XII with a α-haloketone of formula XIII: 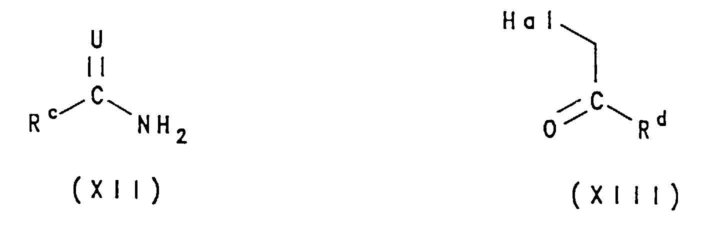 wherein U is oxygen or sulphur, Hal represents halogen, and R^{c} and R^{d} are as defined above; or
(F) reacting a compound of formula XV: 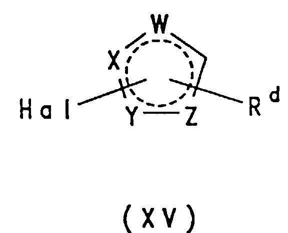 with a reagent which provides an anion -R^{c}, where W, X, Y and Z are as defined in Claim 1, R^{c} and R^{d} are as previously defined and Hal represents halogen; or
(G) reacting a compound of formula XVI: 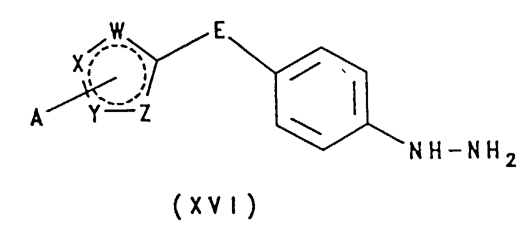
wherein W, X, Y, Z, A and E are as defined in Claim 1; with a compound of formula VII or a carbonyl-protected form thereof: 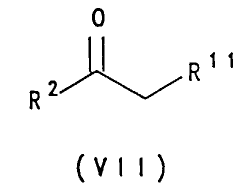 wherein R² is as defined in Claim 1 and R¹¹ corresponds to the group R¹ as defined in Claim 1 or represents a group of formula -CH₂.CHR⁴D, in which R⁴ is hydrogen and D represents a readily displaceable group; followed, where required, by N-alkylation by standard methods to introduce the moiety R³ as defined in Claim 1.

## Claims (Claims for the following Contracting State(s): ES, GR)

1. A process for the preparation of a compound of formula I, or a salt thereof: 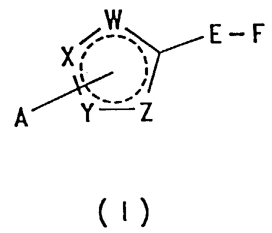 wherein the broken circle represents two non-adjacent double bonds in any position in the five-membered ring;
the five-membered ring containing the substituents W to Z represents a 1,2,4-oxadiazole, 1,3,4-oxadiazole, 1,2,4-thiadiazole, 1,3,4-thiadiazole, 1,3-oxazole or 1,3-thiazole ring;
A represents methyl, methoxymethyl, aminomethyl, dimethylaminomethyl, acetylaminomethyl, benzoylaminomethyl, t-butoxy-carbonylaminomethyl, methylsulphonylaminomethyl, phenylsulphonylaminomethyl, aminocarbonylmethyl, ethyl, aminoethyl, acetylaminoethyl, benzoylaminoethyl, methoxycarbonylaminoethyl, ethoxycarbonylaminoethyl, t-butoxycarbonylaminoethyl, methylsulphonylaminoethyl, aminocarbonylaminoethyl, methylaminocarbonylaminoethyl, t-butylaminocarbonylaminoethyl, phenylaminocarbonylaminoethyl, pyrrolidylcarbonylaminoethyl, cyclopropyl, phenyl, methylsulphonylaminophenyl, aminocarbonylphenyl, methylaminocarbonylphenyl, methylsulphonylaminomethylphenyl, aminosulphonylmethylphenyl, methylaminosulphonylmethylphenyl, dimethylaminosulphonylmethylphenyl, naphthyl, benzyl, diphenylmethyl, trifluoromethylbenzyl, methoxybenzyl, acetylaminobenzyl, methylsulphonylaminobenzyl, aminocarbonylaminobenzyl, aminocarbonylbenzyl, methylaminocarbonylbenzyl, methylsulphonylbenzyl, methylaminosulphonylbenzyl, phenethyl, phenylpropyl, acetylpiperazinyl, methoxycarbonylpiperazinyl, t-butoxycarbonylpiperazinyl, methylaminocarbonylpiperazinyl, methylsulphonylpiperazinyl, phenylsulphonylpiperazinyl, pyridylmethyl, methoxypyridylmethyl, amino, methylamino, benzylamino, dimethylamino, t-butoxycarbonylaminoethylamino, methylsulphonylaminoethylamino, aminocarbonyl, methylaminocarbonyl, azetidinylcarbonyl or pyrrolidylcarbonyl;
E represents a bond or a straight or branched alkylene chain containing from 1 to 4 carbon atoms;
F represents a group of formula 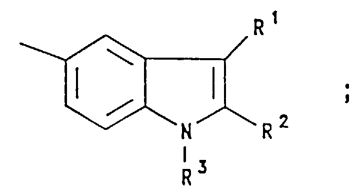
R¹ represents aminoethyl, N-methylaminoethyl, N,N-dimethylaminoethyl or 1-methyl-4-piperidyl; and
R² and R³ independently represent hydrogen or C₁₋₆ alkyl;
which process comprises:
(A) reacting a reactive derivative of a carboxylic acid of formula R^{c}-CO₂H with a compound either of formula III or of formula IV, or a salt thereof: 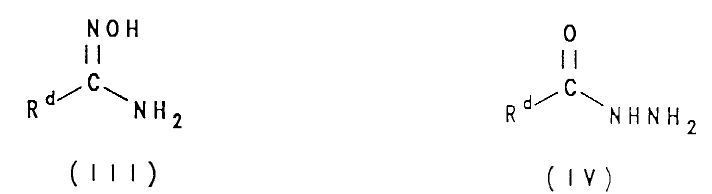 wherein one of R^{c} and R^{d} is a group of formula A, and the other is a group of formula -E-F, as defined above; or
(B) cyclisation of a compound of formula XI: 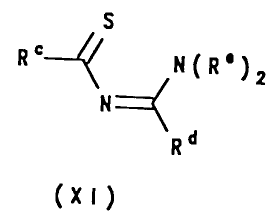 wherein R^{c} and R^{d} are as defined above, and R^{e} is hydrogen or an alkyl group; or
(C) cycloaddition of a nitrile sulphide R^{c}-C≡N⁺-S⁻ with a nitrile of formula R^{d}-CN where R^{c} and R^{d} are as defined above; or
(D) dehydration of a thiosemicarbazide of formula R^{c}CSNHNHCONR^{s}R^{t}, where R^{c} is as defined above and R^{s} and R^{t} are hydrogen or an alkyl group; followed by attachment of the R^{d} group by conventional means; or
(E) reaction of an amide or thioamide of formula XII with a α-haloketone of formula XIII: 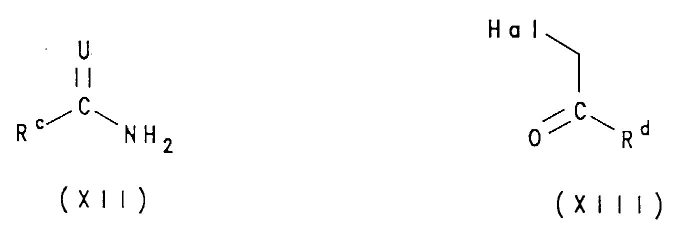 wherein U is oxygen or sulphur, Hal represents halogen, and R^{c} and R^{d} are as defined above; or
(F) reacting a compound of formula XV: 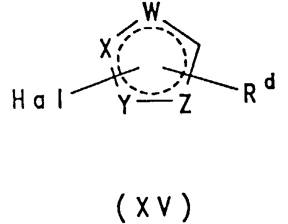 with a reagent which provides an anion ⁻R^{c}, where W, X, Y, Z, R^{c} and R^{d} are as defined above and Hal represents halogen; or
(G) reacting a compound of formula XVI: 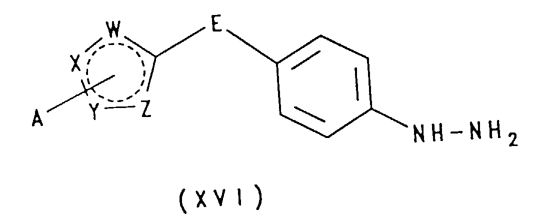
wherein W, X, Y, Z, A and E are as defined above; with a compound of formula VII or a carbonyl-protected form thereof: 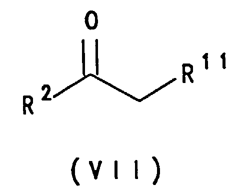 wherein R² is as defined above and R¹¹ corresponds to the group R¹ as defined above or represents a group of formula -CN₂.CHR⁴D, in which R⁴ is hydrogen and D represents a readily displaceable group; followed, where required, by N-alkylation by standard methods to introduce the moiety R³ as defined above.

2. A process as claimed in Claim 1 for the preparation of a compound represented by formula IIA, and salts thereof: 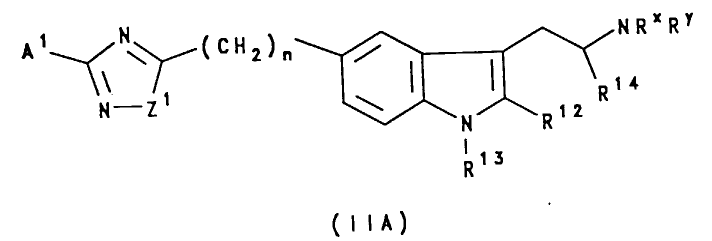 wherein
Z¹ represents oxygen or sulphur;
n is zero, 1, 2 or 3;
A¹ corresponds to the group A as defined in claim 1;
R¹², R¹³ and R¹⁴ each represents hydrogen; and
R^{x} and R^{y} independently represent hydrogen or methyl.

3. A process as claimed in Claim 1 for the preparation of a compound represented by formula IIB, and salts thereof: 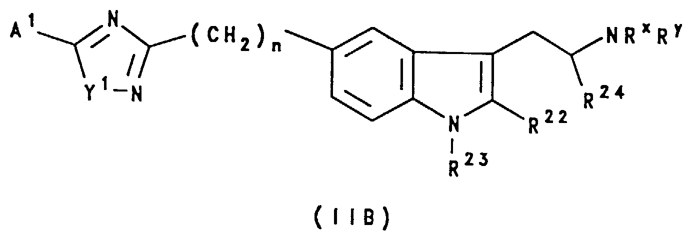 wherein
Y¹ represents oxygen or sulphur;
n is zero, 1, 2 or 3;
A¹ is as defined in Claim 2;
R²², R²³ and R²⁴ each represents hydrogen; and
R^{x} and R^{y} independently represent hydrogen or methyl.

4. A process as claimed in Claim 1 for the preparation of a compound represented by formula IIC, and salts thereof: 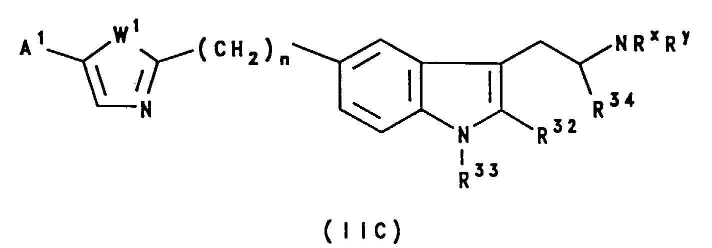 wherein
W¹ represents oxygen or sulphur;
n is zero, 1, 2 or 3;
A¹ is as defined in Claim 2;
R³², R³³ and R³⁴ each represents hydrogen; and
R^{x} and R^{y} independently represent hydrogen or methyl.

5. A process as claimed in Claim 1 for the preparation of a compound represented by formula IID, and salts thereof: 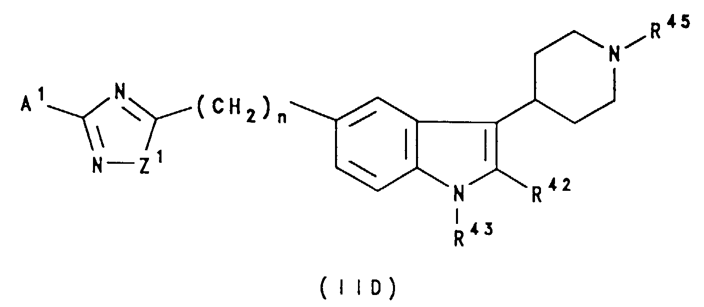 wherein
Z¹ represents oxygen or sulphur;
n is zero, 1, 2 or 3;
A¹ is as defined in Claim 2;
R⁴², and R⁴³ each represents hydrogen; and
R⁴⁵ represents methyl.

6. A process as claimed in Claim 1 for the preparation of a compound selected from:
2-[5-(3-benzyl-1,2,4-oxadiazol-5-yl)-1H-indol-3-yl]ethylamine;
2-[5-(3-methyl-1,2,4-oxadiazol-5-yl)-1H-indol-3-yl]ethylamine;
N,N-dimethyl-2-[5-(3-benzyl-1,2,4-oxadiazol-5-yl)-1H-indol-3-yl]ethylamine;
2-[5-(3-benzyl-1,2,4-oxadiazol-5-ylmethyl)-1H-indol-3-yl]ethylamine;
2-[5-(3-methyl-1,2,4-oxadiazol-5-ylmethyl)-1H-indol-3-yl]ethylamine;
2-[5-(3-amino-1,2,4-oxadiazol-5-ylmethyl)-1H-indol-3-yl]ethylamine;
2-[5-(3-phenyl-1,2,4-oxadiazol-5-ylmethyl)-1H-indol-3-yl]ethylamine;
2-[5-[3-(2-methoxybenzyl)-1,2,4-oxadiazol-5-ylmethyl]-1H-indol-3-yl]ethylamine;
N,N-dimethyl-2-[5-(3-benzyl-1,2,4-oxadiazol-5-ylmethyl)-1H-indol-3-yl]ethylamine;
2-[5-[2-(3-benzyl-1,2,4-oxadiazol-5-yl)ethyl]-1H-indol-3-yl]ethylamine;
N,N-dimethyl-2-[5-(3-methyl-1,2,4-oxadiazol-5-ylmethyl)-1H-indol-3-yl]ethylamine;
2-[5-(3-diphenylmethyl-1,2,4-oxadiazol-5-yl)-1H-indol-3-yl]ethylamine;
2-[5-(3-phenyl-1,2,4-oxadiazol-5-yl)-1H-indol-3-yl]ethylamine;
2-[5-[3-(2-methoxybenzyl)-1,2,4-oxadiazol-5-yl]-1H-indol-3-yl]ethylamine;
2-[5-[3-(3-benzyl-1,2,4-oxadiazol-5-yl)propyl]-1H-indol-3-yl]ethylamine;
2-[5-(3-phenethyl-1,2,4-oxadiazol-5-yl)-1H-indol-3-yl]ethylamine;
2-[5-(5-benzyl-1,2,4-oxadiazol-3-yl)-1H-indol-3-yl]ethylamine;
2-[5-(5-benzyl-1,2,4-oxadiazol-3-ylmethyl)-1H-indol-3-yl]ethylamine;
N,N-dimethyl-2-[5-[3-(2-methoxybenzyl)-1,2,4-oxadiazol-5-yl]-1H-indol-3-yl]ethylamine;
N,N-dimethyl-2-[5-[2-(3-benzyl-1,2,4-oxadiazol-5-yl)ethyl]-1H-indol-3-yl]ethylamine;
2-[5-[3-(1-naphthyl)-1,2,4-oxadiazol-5-ylmethyl]-1H-indol-3-yl]ethylamine;
2-[5-[3-(3-methyl-1,2,4-oxadiazol-5-yl)propyl]-1H-indol-3-yl]ethylamine;
2-[5-[3-(3-cyclopropyl-1,2,4-oxadiazol-5-yl)propyl]-1H-indol-3-yl]ethylamine;
2-[5-[3-(3-methoxybenzyl)-1,2,4-oxadiazol-5-yl]-1H-indol-3-yl]ethylamine;
2-[5-[3-(4-methoxybenzyl)-1,2,4-oxadiazol-5-yl]-1H-indol-3-yl]ethylamine;
2-[5-[3-(4-acetylaminobenzyl)-1,2,4-oxadiazol-5-yl]-1H-indol-3-yl]ethylamine;
2-[5-[3-(4-methylsulphonylaminobenzyl)-1,2,4-oxadiazol-5-yl]-1H-indol-3-yl]ethylamine;
2-[5-[3-(3-phenylpropyl)-1,2,4-oxadiazol-5-yl]-1H-indol-3-yl]ethylamine;
2-[5-(3-cyclopropyl-1,2,4-oxadiazol-5-yl)-1H-indol-3-yl]ethylamine;
2-[5-(3-ethyl-1,2,4-oxadiazol-5-yl)-1H-indol-3-yl]ethylamine;
2-[5-[3-(4-trifluoromethylbenzyl)-1,2,4-oxadiazol-5-yl]-1H-indol-3-yl]ethylamine;
N,N-dimethyl-2-[5-[3-(4-acetylaminobenzyl)-1,2,4-oxadiazol-5-yl]-1H-indol-3-yl]ethylamine;
N,N-dimethyl-2-[5-[3-(4-methylsulphonylaminobenzyl)-1,2,4-oxadiazol-5-yl]-1H-indol-3-yl]ethylamine;
N,N-dimethyl-2-[5-(3-amino-1,2,4-oxadiazol-5-ylmethyl)-1H-indol-3-yl]ethylamine;
2-[5-[2-(3-amino-1,2,4-oxadiazol-5-yl)ethyl]-1H-indol-3-yl]ethylamine;
2-[5-[2-(3-dimethylamino-1,2,4-oxadiazol-5-yl)ethyl]-1H-indol-3-yl]ethylamine;
2-[5-(5-methyl-1,2,4-oxadiazol-3-yl)-1H-indol-3-yl]ethylamine;
2-[5-(5-methyl-1,2,4-oxadiazol-3-ylmethyl)-1H-indol-3-yl]ethylamine;
N,N-dimethyl-2-[5-(5-benzyl-1,2,4-oxadiazol-3-ylmethyl)-1H-indol-3-yl]ethylamine;
2-[5-(3-methoxymethyl-1,2,4-oxadiazol-5-yl)-1H-indol-3-yl]ethylamine;
2-[5-[3-(4-methylaminocarbonylbenzyl)-1,2,4-oxadiazol-5-yl]-1H-indol-3-yl]ethylamine;
2-[5-[3-(4-methylaminocarbonylphenyl)-1,2,4-oxadiazol-5-yl]-1H-indol-3-yl]ethylamine;
2-[5-[3-(4-methylaminosulphonylbenzyl)-1,2,4-oxadiazol-5-yl]-1H-indol-3-yl]ethylamine;
2-[5-[3-(4-methylsulphonylbenzyl)-1,2,4-oxadiazol-5-yl]-1H-indol-3-yl]ethylamine;
2-[5-[3-(3-methylsulphonylaminobenzyl)-1,2,4-oxadiazol-5-yl]-1H-indol-3-yl]ethylamine;
2-[5-[3-(4-aminocarbonylaminobenzyl)-1,2,4-oxadiazol-5-yl]-1H-indol-3-yl]ethylamine;
2-[5-(3-amino-1,2,4-oxadiazol-5-yl)-1H-indol-3-yl]ethylamine;
2-[5-(3-acetylaminomethyl-1,2,4-oxadiazol-5-yl)-1H-indol-3-yl]ethylamine;
2-[5-[3-(2-acetylaminoethyl)-1,2,4-oxadiazol-5-yl]-1H-indol-3-yl]ethylamine;
2-[5-(3-aminomethyl-1,2,4-oxadiazol-5-yl)-1H-indol-3-yl]ethylamine;
N,N-dimethyl-2-[5-(3-amino-1,2,4-oxadiazol-5-yl)-1H-indol-3-yl]ethylamine;
N,N-dimethyl-2-[5-(3-acetylaminomethyl-1,2,4-oxadiazol-5-yl)-1H-indol-3-yl]ethylamine;
N,N-dimethyl-2-[5-[3-(2-acetylaminoethyl)-1,2,4-oxadiazol-5-yl]-1H-indol-3-yl]ethylamine;
N,N-dimethyl-2-[5-[3-(4-aminocarbonylaminobenzyl)-1,2,4-oxadiazol-5-yl]-1H-indol-3-yl]ethylamine;
N,N-dimethyl-2-[5-[3-(2-(t-butoxycarbonylamino)ethyl)-1,2,4-oxadiazol-5-yl]-1H-indol-3-yl]ethylamine;
N,N-dimethyl-2-[5-[3-(4-methylaminocarbonylbenzyl)-1,2,4-oxadiazol-5-yl]-1H-indol-3-yl]ethylamine;
N,N-dimethyl-2-[5-[3-(2-aminoethyl)-1,2,4-oxadiazol-5-yl]-1H-indol-3-yl]ethylamine;
N,N-dimethyl-2-[5-[3-(2-methylsulphonylaminoethyl)-1,2,4-oxadiazol-5-yl]-1H-indol-3-yl]ethylamine;
N,N-dimethyl-2-[5-[3-(2-aminocarbonylaminoethyl)-1,2,4-oxadiazol-5-yl]-1H-indol-3-yl]ethylamine;
N,N-dimethyl-2-[5-[3-(2-methylaminocarbonylaminoethyl)-1,2,4-oxadiazol-5-yl]-1H-indol-3-yl]ethylamine;
N,N-dimethyl-2-[5-[3-(2-methylaminocarbonylaminoethyl)-1,2,4-oxadiazol-5-yl]-1H-indol-3-yl]ethylamine;
N,N-dimethyl-2-[5-[3-(2-methoxycarbonylaminoethyl)-1,2,4-oxadiazol-5-yl]-1H-indol-3-yl]ethylamine;
N,N-dimethyl-2-[5-[3-(2-ethoxycarbonylaminoethyl)1,2,4-oxadiazol-5-yl]-1H-indol-3-yl]ethylamine;
N,N-dimethyl-2-[5-[2-(3-amino-1,2,4-oxadiazol-5-yl)ethyl]-1H-indol-3-yl]ethylamine;
N,N-dimethyl-2-[5-(3-methylamino-1,2,4-oxadiazol-5-ylmethyl)-1H-indol-3-yl]ethylamine;
N,N-dimethyl-2-[5-[3-(4-aminocarbonylbenzyl)-1,2,4-oxadiazol-5-ylmethyl]-1H-indol-3-yl]ethylamine;
N,N-dimethyl-2-[5-[3-(4-acetylaminobenzyl)-1,2,4-oxadiazol-5-ylmethyl]-1H-indol-3-yl]ethylamine;
N,N-dimethyl-2-[5-[3-(4-methylaminosulphonylbenzyl)-1,2,4-oxadiazol-5-ylmethyl]-1H-indol-3-yl]ethylamine;
N,N-dimethyl-2-[5-[3-(4-aminocarbonylaminobenzyl)-1,2,4-oxadiazol-5-ylmethyl]-1H-indol-3-yl]ethylamine;
N,N-dimethyl-2-[5-[3-(4-methylsulphonylaminobenzyl)-1,2,4-oxadiazol-5-ylmethyl]-1H-indol-3-yl]ethylamine;
N,N-dimethyl-2-[5-[3-(4-methylaminocarbonylphenyl)-1,2,4-oxadiazol-5-ylmethyl]-1H-indol-3-yl]ethylamine;
N,N-dimethyl-2-[5-(3-acetylaminomethyl-1,2,4-oxadiazol-5-ylmethyl)-1H-indol-3-yl]ethylamine;
N,N-dimethyl-2-[5-(3-methylsulphonylaminomethyl-1,2,4-oxadiazol-5-ylmethyl)-1H-indol-3-yl]ethylamine;
N,N-dimethyl-2-[5-(3-aminocarbonylmethyl-1,2,4-oxadiazol-5-ylmethyl)-1H-indol-3-yl]ethylamine;
N,N-dimethyl-2-[5-[3-(3-methylsulphonylaminobenzyl)-1,2,4-oxadiazol-5-ylmethyl]-1H-indol-3-yl]ethylamine;
N,N-dimethyl-2-[5-[3-(3-acetylaminobenzyl)-1,2,4-oxadiazol-5-ylmethyl]-1H-indol-3-yl]ethylamine;
N,N-dimethyl-2-[5-[3-(4-aminocarbonylphenyl)-1,2,4-oxadiazol-5-ylmethyl]-1H-indol-3-yl]ethylamine;
N,N-dimethyl-2-[5-[3-(3-aminocarbonylphenyl)-1,2,4-oxadiazol-5-ylmethyl]-1H-indol-3-yl]ethylamine;
N,N-dimethyl-2-[5-[3-(4-methylsulphonylaminophenyl)-1,2,4-oxadiazol-5-ylmethyl]-1H-indol-3-yl]ethylamine;
N,N-dimethyl-2-[5-[3-(4-methylaminosulphonylmethylphenyl)-1,2,4-oxadiazol-5-ylmethyl]-1H-indol-3-yl]ethylamine;
N,N-dimethyl-2-[5-[3-(3-methylaminosulphonylmethylphenyl)-1,2,4-oxadiazol-5-ylmethyl]-1H-indol-3-yl]ethylamine;
N,N-dimethyl-2-[5-[3-(4-aminosulphonylmethylphenyl)-1,2,4-oxadiazol-5-ylmethyl]-1H-indol-3-yl]ethylamine;
N,N-dimethyl-2-[5-[3-(4-dimethylaminosulphonylmethylphenyl)-1,2,4-oxadiazol-5-ylmethyl]-1H-indol-3-yl]ethylamine;
N,N-dimethyl-2-[5-[3-(t-butoxycarbonylamino)methyl-1,2,4-oxadiazol-5-ylmethyl]-1H-indol-3-yl]ethylamine;
N,N-dimethyl-2-[5-[3-(2-(t-butoxycarbonylamino)ethyl)-1,2,4-oxadiazol-5-ylmethyl]-1H-indol-3-yl]ethylamine;
N,N-dimethyl-2-[5-(3-aminomethyl-1,2,4-oxadiazol-5-ylmethyl)-1H-indol-3-yl]ethylamine;
N,N-dimethyl-2-[5-(3-methoxycarbonylaminomethyl-1,2,4-oxadiazol-5-ylmethyl)-1H-indol-3-yl]ethylamine;
N,N-dimethyl-2-[5-(3-dimethylaminomethyl-1,2,4-oxadiazol-5-ylmethyl)-1H-indol-3-yl]ethylamine;
N,N-dimethyl-2-[5-[3-(2-methylsulphonylaminoethyl)-1,2,4-oxadiazol-5-ylmethyl]-1H-indol-3-yl]ethylamine;
N,N-dimethyl-2-[5-[3-(2-ethoxycarbonylaminoethyl)-1,2,4-oxadiazol-5-ylmethyl]-1H-indol-3-yl]ethylamine;
N,N-dimethyl-2-[5-(3-benzoylaminomethyl-5-ylmethyl)-1H-indol-3-yl]ethylamine;
N,N-dimethyl-2-[5-[3-(2-benzoylaminoethyl)-1,2,4-oxadiazol-5-ylmethyl]-1H-indol-3-yl]ethylamine;
N,N-dimethyl-2-[5-[3-(2-phenylaminocarbonylaminoethyl)-1,2,4-oxadiazol-5-ylmethyl]-1H-indol-3-yl]ethylamine;
N,N-dimethyl-2-[5-[3-(2-(t-butylaminocarbonylamino)ethyl)-1,2,4-oxadiazol-5-ylmethyl]-1H-indol-3-yl]ethylamine;
N-methyl-2-[5-(3-amino-1,2,4-oxadiazol-5-ylmethyl)-1H-indol-3-yl]ethylamine;
N,N-dimethyl-2-[5-[3-(4-(t-butoxycarbonyl)piperazin-1-yl)-1,2,4-oxadiazol-5-ylmethyl]-1H-indol-3-yl]ethylamine;
N,N-dimethyl-2-[5-[3-(4-methylsulphonylpiperazin-1-yl)-1,2,4-oxadiazol-5-ylmethyl]-1H-indol-3-yl]ethylamine;
N,N-dimethyl-2-[5-[3-(4-methoxycarbonylpiperazin-1-yl)-1,2,4-oxadiazol-5-ylmethyl]-1H-indol-3-yl]ethylamine;
N,N-dimethyl-2-[5-[3-(4-methylaminocarbonylpiperazin-1-yl)-1,2,4-oxadiazol-5-ylmethyl]-1H-indol-3-yl]ethylamine;
N,N-dimethyl-2-[5-[3-(4-acetylpiperazin-1-yl)-1,2,4-oxadiazol-5-ylmethyl]-1H-indol-3-yl]ethylamine;
N,N-dimethyl-2-[5-[3-(4-methylsulphonylaminomethylphenyl)-1,2,4-oxadiazol-5-yl-methyl]-1H-indol-3-yl]ethylamine;
N,N-dimethyl-2-[5-(3-phenylsulphonylaminomethyl-1,2,4-oxadiazol-5-ylmethyl)-1H-indol-3-yl]ethylamine;
N,N-dimethyl-2-[5-(3-benzylamino-1,2,4-oxadiazol-5-ylmethyl)-1H-indol-3-yl]ethylamine;.
N,N-dimethyl-2-[5-[3-(3-pyridyl)methyl-1,2,4-oxadiazol-5-ylmethyl]-1H-indol-3-yl]ethylamine;
N,N-dimethyl-2-[5-[3-(2-methoxypyrid-5-yl)methyl-1,2,4-oxadiazol-5-ylmethyl]-1H-indol-3-yl]ethylamine;
2-[5-[3-(4-acetylaminobenzyl)-1,2,4-oxadiazol-5-ylmethyl]-1H-indol-3-yl]ethylamine;
2-[5-[3-(4-methylsulphonylaminobenzyl)-1,2,4-oxadiazol-5-ylmethyl]-1H-indol-3-yl]ethylamine;
2-[5-[2-[3-(4-acetylaminobenzyl)-1,2,4-oxadiazol-5-yl]ethyl]-1H-indol-3-yl]ethylamine;
2-[5-[2-[3-(4-methoxybenzyl)-1,2,4-oxadiazol-5-yl]ethyl]-1H-indol-3-yl]ethylamine;
N,N-dimethyl-2-[5-(5-methyl-1,3-oxazol-2-yl)-1H-indol-3-yl]ethylamine;
N,N-dimethyl-2-[5-[2-(5-methyl-1,3-oxazol-2-yl)ethyl]-1H-indol-3-yl]ethylamine;
1-methyl-4-[5-(3-amino-1,2,4-oxadiazol-5-yl)-1H-indol-3-yl]piperidine;
1-methyl-4-[5-(3-amino-1,2,4-oxadiazol-5-ylmethyl)-1H-indol-3-yl]piperidine;
1-methyl-4-[5-[3-(4-methylsulphonylaminobenzyl)-1,2,4-oxadiazol-5-ylmethyl]-1H-indol-3-yl]piperidine;
1-methyl-4-[5-[3-(3-pyridyl)methyl-1,2,4-oxadiazol-5-ylmethyl]-1H-indol-3-yl]piperidine;
N,N-dimethyl-2-[5-[3-(4-pyridyl)methyl-1,2,4-oxadiazol-5-ylmethyl]-1H-indol-3-yl]ethylamine;
N,N-dimethyl-2-[5-[3-(2-(t-butoxycarbonylamino)ethyl)amino-1,2,4-oxadiazol-5-ylmethyl]-1H-indol-3-yl]ethylamine;
2-[5-(3-aminocarbonyl-1,2,4-oxadiazol-5-ylmethyl)-1H-indol-3-yl]ethylamine;
2-[5-(3-methylaminocarbonyl-1,2,4-oxadiazol-5-ylmethyl)-1H-indol-3-yl]ethylamine;
2-[5-[3-(pyrrolid-1-yl)carbonyl-1,2,4-oxadiazol-5-ylmethyl]-1H-indol-3-yl]ethylamine;
2-[5-[3-(azetidin-1-yl)carbonyl-1,2,4-oxadiazol-5-ylmethyl]1H-indol-3-yl]ethylamine;
N,N-dimethyl-2-[5-[3-(4-phenylsulphonylpiperazin-1-yl)-1,2,4-oxadiazol-5-ylmethyl]-1H-indol-3-yl]ethylamine;
N,N-dimethyl-2-[5-[3-(2-(pyrrolid-1-ylcarbonylamino)ethyl)-1,2,4-oxadiazol-5-ylmethyl]-1H-indol-3-yl]ethylamine;
N,N-dimethyl-2-[5-[3-(2-methylsulphonylaminoethyl)amino-1,2,4-oxadiazol-5-ylmethyl]-1H-indol-3-yl]ethylamine;
N,N-dimethyl-2-[5-(3-amino-1,4-thiadiazol-5-ylmethyl)-1H-indol-3-yl]ethylamine;
and salts thereof.

7. A process for the preparation of a pharmaceutical composition which comprises mixing a compound prepared as claimed in any one of the preceding Claims with a pharmaceutically acceptable carrier or excipient.

8. The use of a compound prepared as claimed in any one of Claims 1 to 6 for the manufacture of a medicament for the treatment and/or prevention of clinical conditions for which a selective agonist of 5-HT₁-like receptors is indicated.

## Patentansprüche (Patentansprüche für folgende(n) Vertragsstaat(en): AT, BE, CH, DE, DK, FR, GB, IT, LI, LU, NL, SE)

1. Eine Verbindung der Formel I oder ein Salz davon: 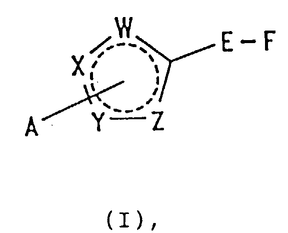 worin
der gestrichelte Kreis zwei nichtbenachbarte Doppelbindungen in irgendeiner Stellung in dem fünfgliedrigen Ring bedeutet,
der fünfgliedrige Ring, der die Substituenten W bis Z enthält, einen 1,2,4-Oxadiazol-, 1,3,4-Oxadiazol-, 1,2,4-Thiadiazol-, 1,3,4-Thiadiazol-, 1,3-Oxazol- oder 1,3-Thiazolring bedeutet,
A Methyl, Methoxymethyl, Aminomethyl, Dimethylaminomethyl, Acetylaminomethyl, Benzoylaminomethyl, t-Butoxycarbonylaminomethyl, Methylsulfonylaminomethyl, Phenylsulfonylaminomethyl, Aminocarbonylmethyl, Ethyl, Aminoethyl, Acetylaminoethyl, Benzoylaminoethyl, Methoxycarbonylaminoethyl, Ethoxycarbonylaminoethyl, t-Butoxycarbonylaminoethyl, Methylsulfonylaminoethyl, Aminocarbonylaminoethyl, Methylaminocarbonylaminoethyl, t-Butylaminocarbonylaminoethyl, Phenylaminocarbonylaminoethyl, Pyrrolidylcarbonylaminoethyl, Cyclopropyl, Phenyl, Methylsulfonylaminophenyl, Aminocarbonylphenyl, Methylaminocarbonylphenyl, Methylsulfonylaminomethylphenyl, Aminosulfonylmethylphenyl, Methylaminosulfonylmethylphenyl, Dimethylaminosulfonylmethylphenyl, Naphthyl, Benzyl, Diphenylmethyl, Trifluormethylbenzyl, Methoxybenzyl, Acetylaminobenzyl, Methylsulfonylaminobenzyl, Aminocarbonylaminobenzyl, Aminocarbonylbenzyl, Methylaminocarbonylbenzyl, Methylsulfonylbenzyl, Methylaminosulfonylbenzyl, Phenethyl, Phenylpropyl, Acetylpiperazinyl, Methoxycarbonylpiperazinyl, t-Butoxycarbonylpiperazinyl, Methylaminocarbonylpiperazinyl, Methylsulfonylpiperazinyl, Phenylsulfonylpiperazinyl, Pyridylmethyl, Methoxypyridylmethyl, Amino, Methylamino, Benzylamino, Dimethylamino, t-Butoxycarbonylaminoethylamino, Methylsulfonylaminoethylamino, Aminocarbonyl, Methylaminocarbonyl, Azetidinylcarbonyl oder Pyrrolidylcarbonyl bedeutet,
E eine Bindung oder eine gerade oder verzweigte Alkylenkette mit 1 bis 4 Kohlenstoffatomen bedeutet,
F eine Gruppe der Formel 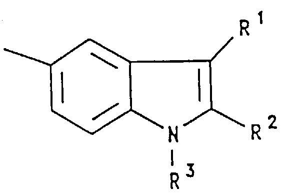 bedeutet,
R¹ Aminoethyl, N-Methylaminoethyl, N,N-Dimethylaminoethyl oder 1-Methyl-4-piperidyl bedeutet, und
R² und R³ unabhängig voneinander Wasserstoff oder C₁₋₆-Alkyl bedeuten.

2. Eine Verbindung wie in Anspruch 1 beansprucht, dargestellt durch Formel IIA, und Salze davon: 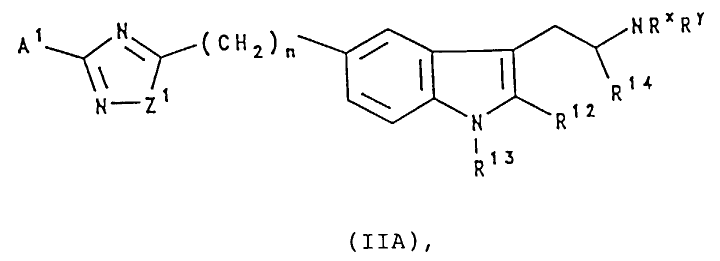 worin
Z¹ Sauerstoff oder Schwefel bedeutet,
n null, 1, 2 oder 3 ist,
A¹ der wie in Anspruch 1 definierten Gruppe A entspricht,
R¹², R¹³ und R¹⁴ jeweils Wasserstoff bedeuten, und
R^{x} und R^{y} unabhängig voneinander Wasserstoff oder Methyl bedeuten.

3. Eine Verbindung wie in Anspruch 1 beansprucht, dargestellt durch Formel IIB, und Salze davon: 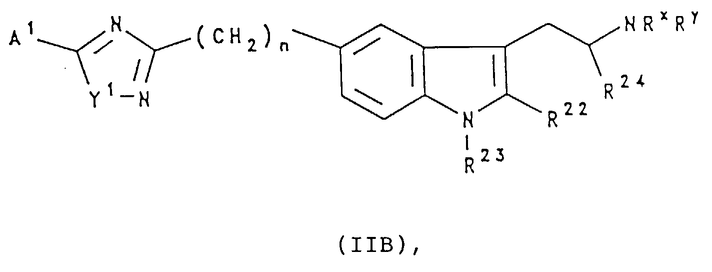 worin
Y¹ Sauerstoff oder Schwefel bedeutet,
n null, 1, 2 oder 3 ist,
A¹ wie in Anspruch 2 definiert ist,
R²², R²³ und R²⁴ jeweils Wasserstoff bedeuten, und
R^{x} und R^{y} unabhängig voneinander Wasserstoff oder Methyl bedeuten.

4. Eine Verbindung wie in Anspruch 1 beansprucht, dargestellt durch Formel IIC, und Salze davon: 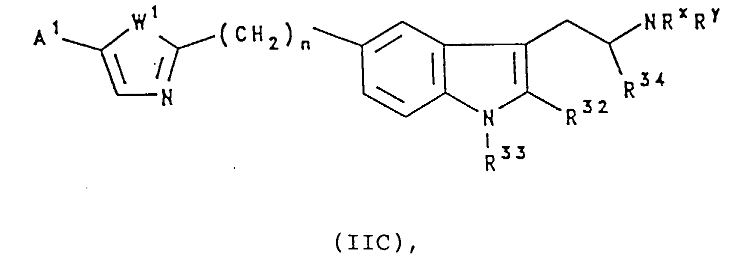 worin
W¹ Sauerstoff oder Schwefel bedeutet,
n null, 1, 2 oder 3 ist,
A¹ wie in Anspruch 2 definiert ist,
R³², R³³ und R³⁴ jeweils Wasserstoff bedeuten, und
R^{x} und R^{y} unabhängig voneinander Wasserstoff oder Methyl bedeuten.

5. Eine Verbindung wie in Anspruch 1 beansprucht, dargestellt durch Formel IID, und Salze davon: 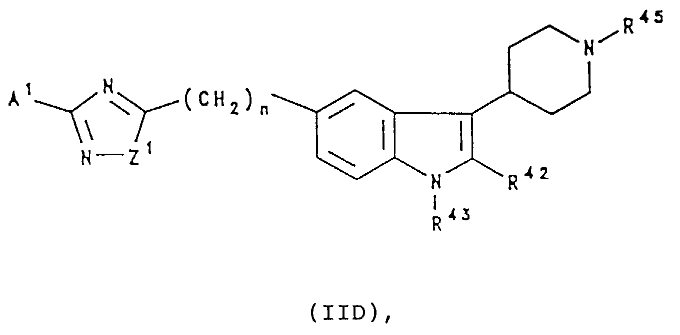 worin
Z¹ Sauerstoff oder Schwefel bedeutet,
n null, 1, 2 oder 3 ist,
A¹ wie in Anspruch 2 definiert ist,
R⁴² und R⁴³ jeweils Wasserstoff bedeuten, und
R⁴⁵ Methyl bedeutet.

6. Eine Verbindung wie in Anspruch 1 beansprucht, ausgewählt aus:
2-[5-(3-Benzyl-1,2,4-oxadiazol-5-yl)-1H-indol-3-yl]ethylamin,
2-[5-(3-Methyl-1,2,4-oxadiazol-5-yl)-1H-indol-3-yl]ethylamin,
N,N-Dimethyl-2-[5-(3-benzyl-1,2,4-oxadiazol-5-yl)-1H-indol-3-yl]ethylamin,
2-[5-(3-Benzyl-1,2,4-oxadiazol-5-ylmethyl)-1H-indol-3-yl]-ethylamin,
2-[5-(3-Methyl-1,2,4-oxadiazol-5-ylmethyl)-1H-indol-3-yl]-ethylamin,
2-[5-(3-Amino-1,2,4-oxadiazol-5-ylmethyl)-1H-indol-3-yl]ethylamin,
2-[5-(3-Phenyl-1,2,4-oxadiazol-5-ylmethyl)-1H-indol-3-yl]ethylamin,
2-[5-[3-(2-Methoxybenzyl)-1,2,4-oxadiazol-5-ylmethyl]-1H-indol-3-yl]ethylamin,
N,N-Dimethyl-2-[5-(3-benzyl-1,2,4-oxadiazol-5-ylmethyl)-1H-indol-3-yl]ethylamin,
2-[5-[2-(3-Benzyl-1,2,4-oxadiazol-5-yl)ethyl]-1H-indol-3-yl]-ethylamin,
N,N-Dimethyl-2-[5-(3-methyl-1,2,4-oxadiazol-5-ylmethyl)-1H-indol-3-yl]ethylamin,
2-[5-(3-Diphenylmethyl-1,2,4-oxadiazol-5-yl)-1H-indol-3-yl]-ethylamin,
2-[5-[3-Phenyl-1,2,4-oxadiazol-5-yl)-1H-indol-3-yl]ethylamin,
2-[5-[3-(2-Methoxybenzyl)-1,2,4-oxadiazol-5-yl]-1H-indol-3-yl]-ethylamin,
2-[5-[3-(3-Benzyl-1,2,4-oxadiazol-5-yl)propyl]-1H-indol-3-yl]ethylamin,
2-[5-(3-Phenethyl-1,2,4-oxadiazol-5-yl)-1H-indol-3-yl]ethylamin,
2-[5-(5-Benzyl-1,2,4-oxadiazol-3-yl)-1H-indol-3-yl]ethylamin,
2-[5-(5-Benzyl-1,2,4-oxadiazol-3-ylmethyl)-1H-indol-3-yl]ethylamin,
N,N-Dimethyl-2-[5-[3-(2-methoxybenzyl) -1,2,4-oxadiazol-5-yl]-1H-indol-3-yl]ethylamin,
N,N-Dimethyl-2-[5-[2-(3-benzyl-1,2,4-oxadiazol-5-yl)ethyl]-1H-indol-3-yl]ethylamin,
2-[5-[3-(1-Naphthyl)-1,2,4-oxadiazol-5-ylmethyl]-1H-indol-3-yl]ethylamin,
2-[5-[3-(3-Methyl-1,2,4-oxadiazol-5-yl)propyl]-1H-indol-3-yl]-ethylamin,
2-[5-[3-(3-Cyclopropyl-1,2,4-oxadiazol-5-yl)propyl]-1H-indol-3-yl]ethylamin,
2-[5-[3-(3-Methoxybenzyl)-1,2,4-oxadiazol-5-yl]-1H-indol-3-yl]-ethylamin,
2-[5-[3-(4-Methoxybenzyl)-1,2,4-oxadiazol-5-yl]-1H-indol-3-yl]-ethylamin,
2-[5-[3-(4-Acetylaminobenzyl)-1,2,4-oxadiazol-5-yl]-1H-indol-3-yl]ethylamin,
2-[5-[3-(4-Methylsulfonylaminobenzyl)-1,2,4-oxadiazol-5-yl]-1H-indol-3-yl]ethylamin,
2-[5-[3-(3-Phenylpropyl)-1,2,4-oxadiazol-5-yl]-1H-indol-3-yl]-ethylamin,
2-[5-(3-Cyclopropyl-1,2,4-oxadiazol-5-yl)-1H-indol-3-yl]ethylamin,
2-[5-(3-Ethyl-1,2,4-oxadiazol-5-yl)-1H-indol-3-yl]ethylamin,
2-[5-[3-(4-Trifluormethylbenzyl)-1,2,4-oxadiazol-5-yl]-1H-indol-3-yl]ethylamin,
N,N-Dimethyl-2-[5-[3-(4-acetylaminobenzyl)-1,2,4-oxadiazol-5-yl]-1H-indol-3-yl]ethylamin,
N,N-Dimethyl-2-[5-[3-(4-methylsulfonylaminobenzyl)-1,2,4-oxadiazol-5-yl]-1H-indol-3-yl]ethylamin,
N,N-Dimethyl-2-[5-(3-amino-1,2,4-oxadiazol-5-ylmethyl)-1H-indol-3-yl]ethylamin,
2-[5-[2-(3-Amino-1,2,4-oxadiazol-5-yl)ethyl]-1H-indol-3-yl]-ethylamin,
2-[5-[2-(3-Dimethylamino-1,2,4-oxadiazol-5-yl)ethyl]-1H-indol-3-yl]ethylamin,
2-[5-(5-Methyl-1,2,4-oxadiazol-3-yl)-1H-indol-3-yl]ethylamin,
2-[5-(5-Methyl-1,2,4-oxadiazol-3-ylmethyl)-1H-indol-3-yl]-ethylamin,
N,N-Dimethyl-2-[5-(5-benzyl-1,2,4-oxadiazol-3-ylmethyl)-1H-indol-3-yl]ethylamin,
2-[5-(3-Methoxymethyl-1,2,4-oxadiazol-5-yl)-1H-indol-3-yl]-ethylamin,
2-[5-[3-(4-Methylaminocarbonylbenzyl)-1,2,4-oxadiazol-5-yl]-1H-indol-3-yl]ethylamin,
2-[5-[3-(4-Methylaminocarbonylphenyl)-1,2,4-oxadiazol-5-yl]-1H-indol-3-yl]ethylamin,
2-[5-[3-(4-Methylaminosulfonylbenzyl)-1,2,4-oxadiazol-5-yl]-1H-indol-3-yl]ethylamin,
2-[5-[3-(4-Methylsulfonylbenzyl)-1,2,4-oxadiazol-5-yl]-1H-indol-3-yl]ethylamin,
2-[5-[3-(3-Methylsulfonylaminobenzyl)-1,2,4-oxadiazol-5-yl]-1H-indol-3-yl]ethylamin,
2-[5-[3-(4-Aminocarbonylaminobenzyl)-1,2,4-oxadiazol-5-yl]-1H-indol-3-yl]ethylamin,
2-[5-(3-Amino-1,2,4-oxadiazol-5-yl)-1H-indol-3-yl]ethylamin,
2-[5-(3-Acetylaminomethyl-1,2,4-oxadiazol-5-yl)-1H-indol-3-yl]-ethylamin,
2-[5-[3-(2-Acetylaminoethyl)-1,2,4-oxadiazol-5-yl]-1H-indol-3-yl]ethylamin,
2-[5-(3-Aminomethyl-1,2,4-oxadiazol-5-yl)-1H-indol-3-yl]ethylamin,
N,N-Dimethyl-2-[5-(3-amino-1,2,4-oxadiazol-5-yl)-1H-indol-3-yl]ethylamin,
N,N-Dimethyl-2-[5-(3-acetylaminomethyl-1,2,4-oxadiazol-5-yl)-1H-indol-3-yl]ethylamin,
N,N-Dimethyl-2-[5-[3-(2-acetylaminoethyl)-1,2,4-oxadiazol-5-yl]-1H-indol-3-yl]ethylamin,
N,N-Dimethyl-2-[5-[3-(4-aminocarbonylaminobenzyl)-1,2,4-oxadiazol-5-yl]-1H-indol-3-yl]ethylamin,
N,N-Dimethyl-2-[5-[3-(2-(t-butoxycarbonylamino)ethyl)-1,2,4-oxadiazol-5-yl]-1H-indol-3-yl]ethylamin,
N,N-Dimethyl-2-[5-[3-(4-methylaminocarbonylbenzyl)-1,2,4-oxadiazol-5-yl]-1H-indol-3-yl]ethylamin,
N,N-Dimethyl-2-[5-[3-(2-aminoethyl)-1,2,4-oxadiazol-5-yl]-1H-indol-3-yl]ethylamin,
N,N-Dimethyl-2-[5-[3-(2-methylsulfonylaminoethyl)-1,2,4-oxadiazol-5-yl]-1H-indol-3-yl]ethylamin,
N,N-Dimethyl-2-[5-[3-(2-aminocarbonylaminoethyl)-1,2,4-oxadiazol-5-yl]-1H-indol-3-yl]ethylamin,
N,N-Dimethyl-2-[5-[3-(2-methylaminocarbonylaminoethyl)-1,2,4-oxadiazol-5-yl]-1H-indol-3-yl]ethylamin,
N,N-Dimethyl-2-[5-[3-(2-methylaminocarbonylaminoethyl)-1,2,4-oxadiazol-5-yl]-1H-indol-3-yl]ethylamin,
N,N-Dimethyl-2-[5-[3-(2-methoxycarbonylaminoethyl)-1,2,4-oxadiazol-5-yl]-1H-indol-3-yl]ethylamin,
N,N-Dimethyl-2-[5-[3-(2-ethoxycarbonylaminoethyl)-1,2,4-oxadiazol-5-yl]-1H-indol-3-yl]ethylamin,
N,N-Dimethyl-2-[5-[2-(3-amino-1,2,4-oxadiazol-5-yl)ethyl]-1H-indol-3-yl]ethylamin,
N,N-Dimethyl-2-[5-(3-methylamino-1,2,4-oxadiazol-5-ylmethyl)-1H-indol-3-yl]ethylamin,
N,N-Dimethyl-2-[5-[3-(4-aminocarbonylbenzyl)-1,2,4-oxadiazol-5-ylmethyl]-1H-indol-3-yl]ethylamin,
N,N-Dimethyl-2-[5-[3-(4-acetylaminobenzyl)-1,2,4-oxadiazol-5-ylmethyl]-1H-indol-3-yl]ethylamin,
N,N-Dimethyl-2-[5-[3-(4-methylaminosulfonylbenzyl)-1,2,4-oxadiazol-5-ylmethyl]-1H-indol-3-yl]ethylamin,
N,N-Dimethyl-2-[5-[3-(4-aminocarbonylaminobenzyl)-1,2,4-oxadiazol-5-ylmethyl]-1H-indol-3-yl]ethylamin,
N,N-Dimethyl-2-[5-[3-(4-methylsulfonylaminobenzyl)-1,2,4-oxadiazol-5-ylmethyl]-1H-indol-3-yl]ethylamin,
N,N-Dimethyl-2-[5-[3-(4-methylaminocarbonylphenyl)-1,2,4-oxadiazol-5-ylmethyl]-1H-indol-3-yl]ethylamin,
N,N-Dimethyl-2-[5-(3-acetylaminomethyl-1,2,4-oxadiazol-5-yl-methyl)-1H-indol-3-yl]ethylamin,
N,N-Dimethyl-2-[5-(3-methylsulfonylaminomethyl-1,2,4-oxadiazol-5-ylmethyl)-1H-indol-3-yl]ethylamin,
N,N-Dimethyl-2-[5-(3-aminocarbonylmethyl-1,2,4-oxadiazol-5-yl-methyl)-1H-indol-3-yl]ethylamin,
N,N-Dimethyl-2-[5-[3-(3-methylsulfonylaminobenzyl)-1,2,4-oxadiazol-5-ylmethyl]-1H-indol-3-yl]ethylamin,
N,N-Dimethyl-2-[5-[3-(3-acetylaminobenzyl)-1,2,4-oxadiazol-5-ylmethyl]-1H-indol-3-yl]ethylamin,
N,N-Dimethyl-2-[5-[3-(4-aminocarbonylphenyl)-1,2,4-oxadiazol-5-ylmethyl]-1H-indol-3-yl]ethylamin,
N,N-Dimethyl-2-[5-[3-(3-aminocarbonylphenyl)-1,2,4-oxadiazol-5-ylmethyl]-1H-indol-3-yl]ethylamin,
N,N-Dimethyl-2-[5-[3-(4-methylsulfonylaminophenyl)-1,2,4-oxadiazol-5-ylmethyl]-1H-indol-3-yl]ethylamin,
N,N-Dimethyl-2-[5-[3-(4-methylaminosulfonylmethylphenyl)-1,2,4-oxadiazol-5-ylmethyl]-1H-indol-3-yl]ethylamin,
N,N-Dimethyl-2-[5-[3-(3-methylaminosulfonylmethylphenyl)-1,2,4-oxadiazol-5-ylmethyl]-1H-indol-3-yl]ethylamin,
N,N-Dimethyl-2-[5-[3-(4-aminosulfonylmethylphenyl)-1,2,4-oxadiazol-5-ylmethyl]-1H-indol-3-yl]ethylamin,
N,N-Dimethyl-2-[5-[3-(4-dimethylaminosulfonylmethylphenyl)-1,2,4-oxadiazol-5-ylmethyl]-1H-indol-3-yl]ethylamin,
N,N-Dimethyl-2-[5-[3-(t-butoxycarbonylamino)methyl-1,2,4-oxadiazol-5-ylmethyl]-1H-indol-3-yl]ethylamin,
N,N-Dimethyl-2-[5-[3-(2-(t-butoxycarbonylamino)ethyl)-1,2,4-oxadiazol-5-ylmethyl]-1H-indol-3-yl]ethylamin,
N,N-Dimethyl-2-[5-(3-aminomethyl-1,2,4-oxadiazol-5-ylmethyl)-1H-indol-3-yl]ethylamin,
N,N-Dimethyl-2-[5-(3-methoxycarbonylaminomethyl-1,2,4-oxadiazol-5-ylmethyl)-1H-indol-3-yl]ethylamin,
N,N-Dimethyl-2-[5-(3-dimethylaminomethyl-1,2,4-oxadiazol-5-yl-methyl)-1H-indol-3-yl]ethylamin,
N,N-Dimethyl-2-[5-[3-(2-methylsulfonylaminoethyl)-1,2,4-oxadiazol-5-ylmethyl]-1H-indol-3-yl]ethylamin,
N,N-Dimethyl-2-[5-[3-(2-ethoxycarbonylaminoethyl)-1,2,4-oxadiazol-5-ylmethyl]-1H-indol-3-yl]ethylamin,
N,N-Dimethyl-2-[5-(3-benzoylaminomethyl-1,2,4-oxadiazol-5-yl-methyl)-1H-indol-3-yl]ethylamin,
N,N-Dimethyl-2-[5-[3-(2-benzoylaminoethyl)-1,2,4-oxadiazol-5-ylmethyl]-1H-indol-3-yl]ethylamin,
N,N-Dimethyl-2-[5-[3-(2-phenylaminocarbonylaminoethyl)-1,2,4-oxadiazol-5-ylmethyl]-1H-indol-3-yl]ethylamin,
N,N-Dimethyl-2-[5-[3-(2-(t-butylaminocarbonylamino)ethyl)-1,2,4-oxadiazol-5-ylmethyl]-1H-indol-3-yl]ethylamin,
N-Methyl-2-[5-(3-amino-1,2,4-oxadiazol-5-ylmethyl)-1H-indol-3-yl]ethylamin,
N,N-Dimethyl-2-[5-[3-(4-(t-butoxycarbonyl)piperazin-1-yl)-1,2,4-oxadiazol-5-ylmethyl]-1H-indol-3-yl]ethylamin,
N,N-Dimethyl-2-[5-[3-(4-methylsulfonylpiperazin-1-yl)-1,2,4-oxadiazol-5-ylmethyl]-1H-indol-3-yl]ethylamin,
N,N-Dimethyl-2-[5-[3-(4-methoxycarbonylpiperazin-1-yl)-1,2,4-oxadiazol-5-ylmethyl]-1H-indol-3-yl]ethylamin,
N,N-Dimethyl-2-[5-[3-(4-methylaminocarbonylpiperazin-1-yl)-1,2,4-oxadiazol-5-ylmethyl]-1H-indol-3-yl]ethylamin,
N,N-Dimethyl-2-[5-[3-(4-acetylpiperazin-1-yl)-1,2,4-oxadiazol-5-ylmethyl]-1H-indol-3-yl]ethylamin,
N,N-Dimethyl-2-[5-[3-(4-methylsulfonylaminomethylphenyl)-1,2,4-oxadiazol-5-yl-methyl]-1H-indol-3-yl]ethylamin,
N,N-Dimethyl-2-[5-(3-phenylsulfonylaminomethyl-1,2,4-oxadiazol-5-ylmethyl)-1H-indol-3-yl]ethylamin,
N,N-Dimethyl-2-[5-(3-benzylamino-1,2,4-oxadiazol-5-ylmethyl)-1H-indol-3-yl]ethylamin,
N,N-Dimethyl-2-[5-[3-(3-pyridyl)methyl-1,2,4-oxadiazol-5-yl-methyl]-1H-indol-3-yl]ethylamin,
N,N-Dimethyl-2-[5-[3-(2-methoxypyrid-5-yl)methyl-1,2,4-oxadiazol-5-ylmethyl]-1H-indol-3-yl]ethylamin,
2-[5-[3-(4-Acetylaminobenzyl)-1,2,4-oxadiazol-5-ylmethyl]-1H-indol-3-yl]ethylamin,
2-[5-[3-(4-Methylsulfonylaminobenzyl)-1,2,4-oxadiazol-5-yl-methyl]-1H-indol-3-yl]ethylamin,
2-[5-[2-[3-(4-Acetylaminobenzyl)-1,2,4-oxadiazol-5-yl]ethyl]-1H-indol-3-yl]ethylamin,
2-[5-[2-[3-(4-Methoxybenzyl)-1,2,4-oxadiazol-5-yl]ethyl]-1H-indol-3-yl]ethylamin,
N,N-Dimethyl-2-[5-(5-methyl-1,3-oxazol-2-yl)-1H-indol-3-yl]-ethylamin,
N,N-Dimethyl-2-[5-[2-(5-methyl-1,3-oxazol-2-yl)ethyl]-1H-indol-3-yl]ethylamin,
1-Methyl-4-[5-(3-amino-1,2,4-oxadiazol-5-yl)-1H-indol-3-yl]-piperidin,
1-Methyl-4-[5-(3-amino-1,2,4-oxadiazol-5-ylmethyl)-1H-indol-3-yl]piperidin,
1-Methyl-4-[5-[3-(4-methylsulfonylaminobenzyl)-1,2,4-oxadiazol-5-ylmethyl]-1H-indol-3-yl]piperidin,
1-Methyl-4-[5-[3-(3-pyridyl)methyl-1,2,4-oxadiazol-5-ylmethyl]-1H-indol-3-yl]piperidin,
N,N-Dimethyl-2-[5-[3-(4-pyridyl)methyl-1,2,4-oxadiazol-5-yl-methyl]-1H-indol-3-yl]ethylamin,
N,N-Dimethyl-2-[5-[3-(2-(t-butoxycarbonylamino)ethyl)amino-1,2,4-oxadiazol-5-ylmethyl]-1H-indol-3-yl]ethylamin,
2-[5-(3-Aminocarbonyl-1,2,4-oxadiazol-5-ylmethyl)-1H-indol-3-yl]ethylamin,
2-[5-(3-Methylaminocarbonyl-1,2,4-oxadiazol-5-ylmethyl)-1H-indol-3-yl]ethylamin,
2-[5-[3-(Pyrrolid-1-yl)carbonyl-1,2,4-oxadiazol-5-ylmethyl]-1H-indol-3-yl]ethylamin,
2-[5-[3-(Azetidin-1-yl)carbonyl-1,2,4-oxadiazol-5-ylmethyl]-1H-indol-3-yl]ethylamin,
N,N-Dimethyl-2-[5-[3-(4-phenylsulfonylpiperazin-1-yl)-1,2,4-oxadiazol-5-ylmethyl]-1H-indol-3-yl]ethylamin,
N,N-Dimethyl-2-[5-[3-(2-(pyrrolid-1-ylcarbonylamino)ethyl)-1,2,4-oxadiazol-5-ylmethyl]-1H-indol-3-yl]ethylamin,
N,N-Dimethyl-2-[5-[3-(2-methylsulfonylaminoethyl)amino-1,2,4-oxadiazol-5-ylmethyl]-1H-indol-3-yl]ethylamin,
N,N-Dimethyl-2-[5-(3-amino-1,4-thiadiazol-5-ylmethyl)-1H-indol-3-yl]ethylamin
und Salzen davon.

7. Eine pharmazeutische Zusammensetzungen, die eine wie in irgendeinem der vorhergehenden Ansprüche beanspruchte Verbindung in Verbindung mit einem pharmazeutisch annehmbaren Träger oder Hilfsstoff enthält.

8. Eine Verbindung wie in irgendeinem der Ansprüche 1 bis 6 beansprucht zur Verwendung in der Therapie.

9. Die Verwendung einer wie in irgendeinem der Ansprüche 1 bis 6 beanspruchten Verbindung zur Herstellung eines Medikaments zur Behandlung und/oder Prävention von klinischen Zuständen, für die ein selektiver Agonist von 5-HT₁-ähnlichen Rezeptoren indiziert ist.

10. Ein Verfahren zur Herstellung einer wie in irgendeinem der Ansprüche 1 bis 6 beanspruchten Verbindung, das umfaßt:
(A) Umsetzung eines reaktiven Derivats einer Carbonsäure der Formel R^{c}-CO₂H mit einer Verbindung entweder der Formel III oder der Formel IV oder einem Salz davon: 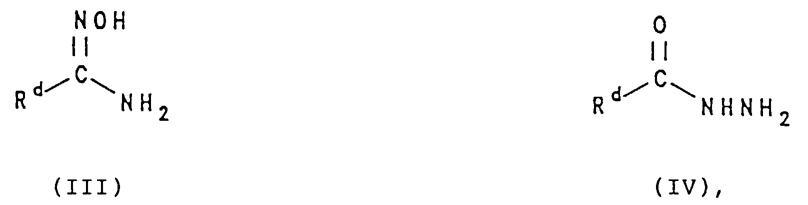 worin einer der Reste R^{c} und R^{d} eine Gruppe der Formel A ist, und der andere eine Gruppe der Formel -E-F, wie sie in Anspruch 1 definiert ist, ist, oder
(B) Cyclisierung einer Verbindung der Formel XI: 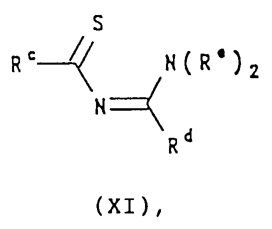 worin R^{c} und R^{d} wie oben definiert sind, und R^{e} Wasserstoff oder eine Alkylgruppe ist, oder
(C) Cycloaddition eines Nitrilsulfids R^{c}-C≡N⁺-S⁻ mit einem Nitril der Formel R^{d}-CN, wobei R^{c} und R^{d} wie oben definiert sind, oder
(D) Dehydrierung eines Thiosemicarbazids der Formel R^{c}CSNHNHCONR^{s}R^{t}, worin R^{c} wie oben definiert ist, und R^{s} und R^{t} Wasserstoff oder eine Alkylgruppe sind, gefolgt von Anbindung der R^{d}-Gruppe durch herkömmliche Mittel, oder
(E) Umsetzung eines Amids oder Thioamids der Formel XII mit einem α-Halogenketon der Formel XIII: 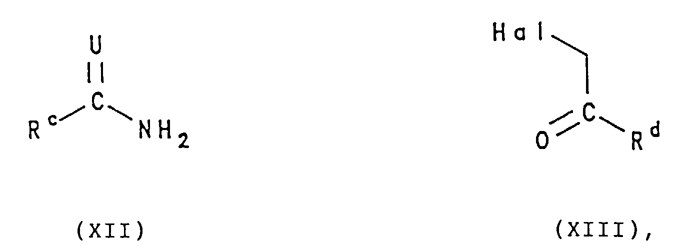 worin U Sauerstoff oder Schwefel ist, Hal Halogen bedeutet, und R^{c} und R^{d} wie oben definiert sind, oder
(F) Umsetzung einer Verbindung der Formel XV: 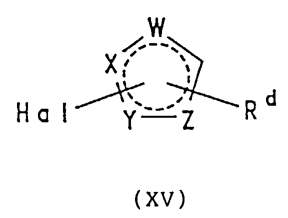 mit einem Reagenz, das ein Anion ⁻R^{c} zur Verfügung stellt, wobei W, X, Y und Z wie in Anspruch 1 definiert sind, R^{c} und R^{d} wie zuvor definiert sind, und Hal Halogen bedeutet, oder
(G) Umsetzung einer Verbindung der Formel XVI: 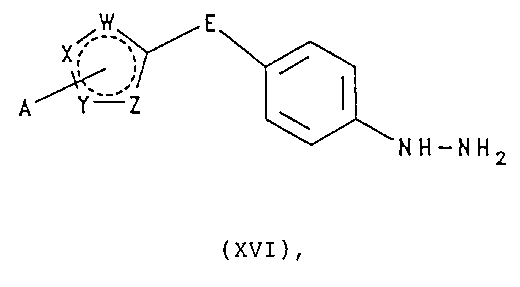
worin W, X, Y, Z, A und E wie in Anspruch definiert sind, mit einer Verbindung der Formel VII oder einer carbonylgeschützten Form davon: 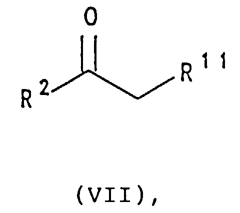 worin R² wie in Anspruch 1 definiert ist, und R¹¹ der Gruppe R¹ entspricht, wie sie in Anspruch 1 definiert ist, oder eine Gruppe der Formel -CH₂.CHR⁴D bedeutet, in der R⁴ Wasserstoff ist und D eine leicht ersetzbare Gruppe bedeutet, gefolgt, wo erforderlich, von N-Alkylierung durch Standardverfahren, um den wie in Anspruch 1 definierten Rest R³ einzuführen.

## Patentansprüche (Patentansprüche für folgende(n) Vertragsstaat(en): ES, GR)

1. Ein Verfahren zur Herstellung einer Verbindung der Formel I oder eines Salzes davon: 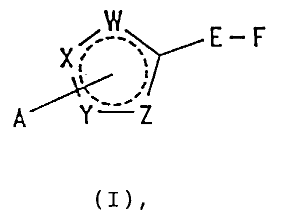 worin
der gestrichelte Kreis zwei nichtbenachbarte Doppelbindungen in irgendeiner Stellung in dem fünfgliedrigen Ring bedeutet,
der fünfgliedrige Ring, der die Substituenten W bis Z enthält, einen 1,2,4-Oxadiazol-, 1,3,4-Oxadiazol-, 1,2,4-Thiadiazol-, 1,3,4-Thiadiazol-, 1,3-Oxazol- oder 1,3-Thiazolring bedeutet,
A Methyl, Methoxymethyl, Aminomethyl, Dimethylaminomethyl, Acetylaminomethyl, Benzoylaminomethyl, t-Butoxycarbonylaminomethyl, Methylsulfonylaminomethyl, Phenylsulfonylaminomethyl, Aminocarbonylmethyl, Ethyl, Aminoethyl, Acetylaminoethyl, Benzoylaminoethyl, Methoxycarbonylaminoethyl, Ethoxycarbonylaminoethyl, t-Butoxycarbonylaminoethyl, Methylsulfonylaminoethyl, Aminocarbonylaminoethyl, Methylaminocarbonylaminoethyl, t-Butylaminocarbonylaminoethyl, Phenylaminocarbonylaminoethyl, Pyrrolidylcarbonylaminoethyl, Cyclopropyl, Phenyl, Methylsulfonylaminophenyl, Aminocarbonylphenyl, Methylaminocarbonylphenyl, Methylsulfonylaminomethylphenyl, Aminosulfonylmethylphenyl, Methylaminosulfonylmethylphenyl, Dimethylaminosulfonylmethylphenyl, Naphthyl, Benzyl, Diphenylmethyl, Trifluormethylbenzyl, Methoxybenzyl, Acetylaminobenzyl, Methylsulfonylaminobenzyl, Aminocarbonylaminobenzyl, Aminocarbonylbenzyl, Methylaminocarbonylbenzyl, Methylsulfonylbenzyl, Methylaminosulfonylbenzyl, Phenethyl, Phenylpropyl, Acetylpiperazinyl, Methoxycarbonylpiperazinyl, t-Butoxycarbonylpiperazinyl, Methylaminocarbonylpiperazinyl, Methylsulfonylpiperazinyl, Phenylsulfonylpiperazinyl, Pyridylmethyl, Methoxypyridylmethyl, Amino, Methylamino, Benzylamino, Dimethylamino, t-Butoxycarbonylaminoethylamino, Methylsulfonylaminoethylamino, Aminocarbonyl, Methylaminocarbonyl, Azetidinylcarbonyl oder Pyrrolidylcarbonyl bedeutet,
E eine Bindung oder eine gerade oder verzweigte Alkylenkette mit 1 bis 4 Kohlenstoffatomen bedeutet,
F eine Gruppe der Formel 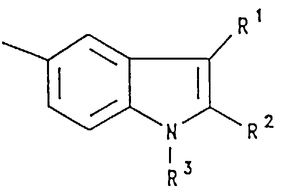 bedeutet,
R¹ Aminoethyl, N-Methylaminoethyl, N,N-Dimethylaminoethyl oder 1-Methyl-4-piperidyl bedeutet, und
R² und R³ unabhängig voneinander Wasserstoff oder C₁₋₆-Alkyl bedeuten,
wobei das Verfahren umfaßt:
(A) Umsetzung eines reaktiven Derivats einer Carbonsäure der Formel R^{c}-CO₂H mit einer Verbindung entweder der Formel III oder der Formel IV oder einem Salz davon: 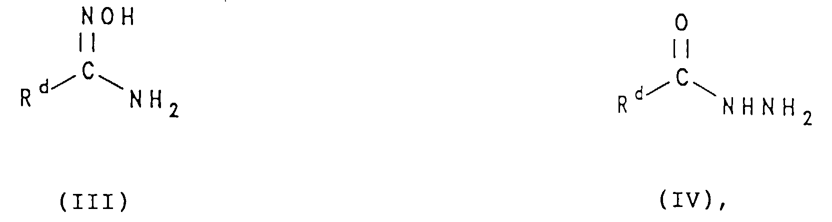 worin einer der Reste R^{c} und R^{d} eine Gruppe der Formel A ist, und der andere eine Gruppe der Formel -E-F, wie sie oben definiert ist, ist, oder
(B) Cyclisierung einer Verbindung der Formel XI: 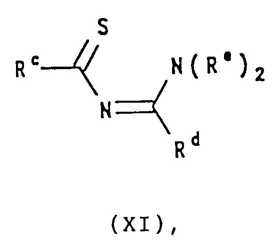 worin R^{c} und R^{d} wie oben definiert sind, und R^{e} Wasserstoff oder eine Alkylgruppe ist, oder
(C) Cycloaddition eines Nitrilsulfids R^{c}-C≡N⁺-S⁻ mit einem Nitril der Formel R^{d}-CN, wobei R^{c} und R^{d} wie oben definiert sind, oder
(D) Dehydrierung eines Thiosemicarbazids der Formel R^{c}CSNHNHCONR^{s}R^{t}, worin R^{c} wie oben definiert ist, und R^{s} und R^{t} Wasserstoff oder eine Alkylgruppe sind, gefolgt von Anbindung der R^{d}-Gruppe durch herkömmliche Mittel, oder
(E) Umsetzung eines Amids oder Thioamids der Formel XII mit einem α-Halogenketon der Formel XIII: 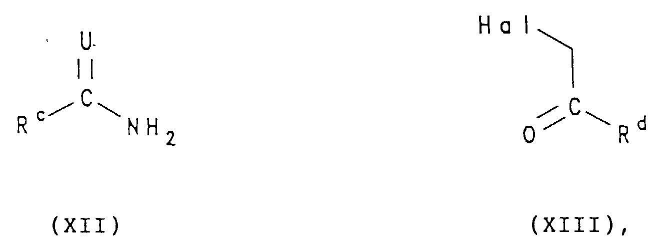 worin U Sauerstoff oder Schwefel ist, Hal Halogen bedeutet, und R^{c} und R^{d} wie oben definiert sind, oder
(F) Umsetzung einer Verbindung der Formel XV: 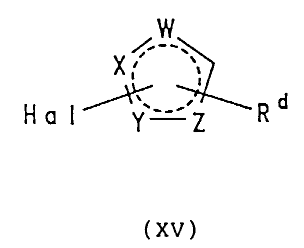 mit einem Reagenz, das ein Anion ⁻R^{c} zur Verfügung stellt, wobei W, X, Y, Z, R^{c} und R^{d} wie oben definiert sind, und Hal Halogen bedeutet, oder
(G) Umsetzung einer Verbindung der Formel XVI: 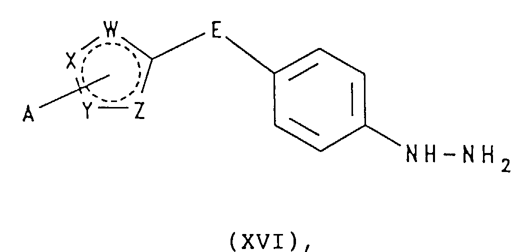 worin W, X, Y, Z, A und E wie oben definiert sind, mit einer Verbindung der Formel VII oder einer carbonylgeschützten Form davon: 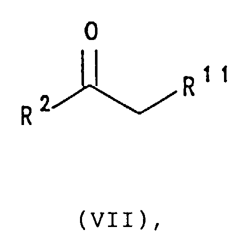
worin R² wie oben definiert ist, und R¹¹ der Gruppe R¹ entspricht, wie sie oben definiert ist, oder eine Gruppe der Formel -CH₂.CHR⁴D bedeutet, in der R⁴ Wasserstoff ist und D eine leicht ersetzbare Gruppe bedeutet, gefolgt, wo erforderlich, von N-Alkylierung durch Standardverfahren, um den wie oben definierten Rest R³ einzuführen.

2. Ein wie in Anspruch 1 beanspruchtes Verfahren zur Herstellung einer Verbindung, dargestellt durch Formel IIA, und von Salzen davon: 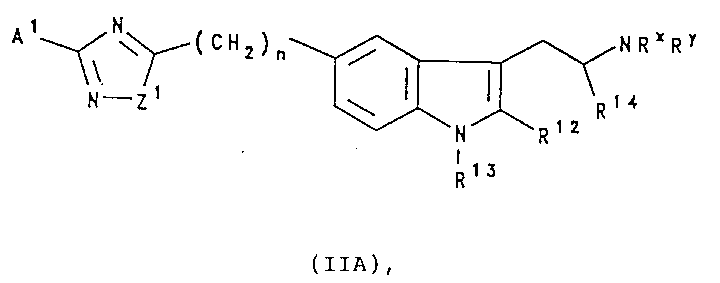 worin
Z¹ Sauerstoff oder Schwefel bedeutet,
n null, 1, 2 oder 3 ist,
A¹ der wie in Anspruch 1 definierten Gruppe A entspricht,
R¹², R¹³ und R¹⁴ jeweils Wasserstoff bedeuten, und
R^{x} und R^{y} unabhängig voneinander Wasserstoff oder Methyl bedeuten.

3. Ein wie in Anspruch 1 beanspruchtes Verfahren zur Herstellung einer Verbindung, dargestellt durch Formel IIB, und von Salzen davon: 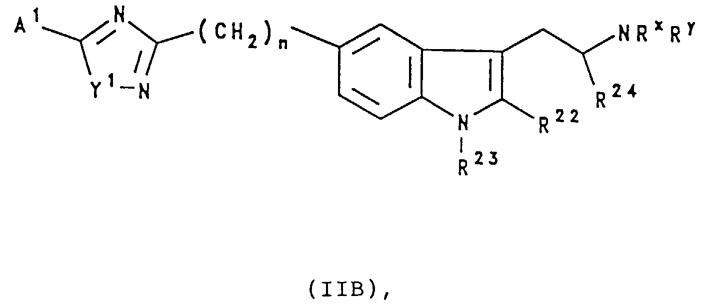 worin
Y¹ Sauerstoff oder Schwefel bedeutet,
n null, 1, 2 oder 3 ist,
A¹ wie in Anspruch 2 definiert ist,
R²², R²³ und R²⁴ jeweils Wasserstoff bedeuten, und
R^{x} und R^{y} unabhängig voneinander Wasserstoff oder Methyl bedeuten.

4. Ein wie in Anspruch 1 beanspruchtes Verfahren zur Herstellung einer Verbindung, dargestellt durch Formel IIC, und von Salzen davon: 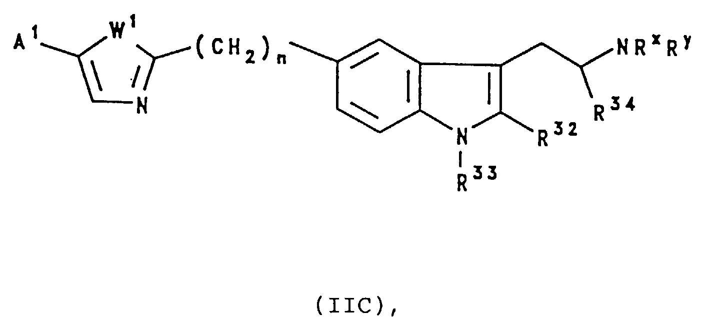 worin
W¹ Sauerstoff oder Schwefel bedeutet,
n null, 1, 2 oder 3 ist,
A¹ wie in Anspruch 2 definiert ist,
R³², R³³ und R³⁴ jeweils Wasserstoff bedeuten, und
R^{x} und R^{y} unabhängig voneinander Wasserstoff oder Methyl bedeuten.

5. Ein wie in Anspruch 1 beanspruchtes Verfahren zur Herstellung einer Verbindung, dargestellt durch Formel IID, und von Salzen davon: 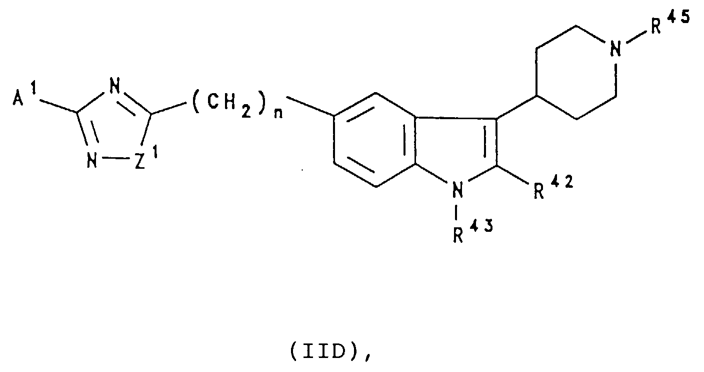 worin
Z¹ Sauerstoff oder Schwefel bedeutet,
n null, 1, 2 oder 3 ist,
A¹ wie in Anspruch 2 definiert ist,
R⁴² und R⁴³ jeweils Wasserstoff bedeuten, und
R⁴⁵ Methyl bedeutet.

6. Ein wie in Anspruch 1 beanspruchtes Verfahren zur Herstellung einer Verbindung, ausgewählt aus:
2-[5-(3-Benzyl-1,2,4-oxadiazol-5-yl)-1H-indol-3-yl]ethylamin,
2-[5-(3-Methyl-1,2,4-oxadiazol-5-yl)-1H-indol-3-yl]ethylamin,
N,N-Dimethyl-2-[5-(3-benzyl-1,2,4-oxadiazol-5-yl)-1H-indol-3-yl]ethylamin,
2-[5-(3-Benzyl-1,2,4-oxadiazol-5-ylmethyl)-1H-indol-3-yl]-ethylamin,
2-[5-(3-Methyl-1,2,4-oxadiazol-5-ylmethyl)-1H-indol-3-yl]-ethylamin,
2-[5-(3-Amino-1,2,4-oxadiazol-5-ylmethyl)-1H-indol-3-yl]ethylamin,
2-[5-(3-Phenyl-1,2,4-oxadiazol-5-ylmethyl)-1H-indol-3-yl]ethylamin,
2-[5-[3-(2-Methoxybenzyl)-1,2,4-oxadiazol-5-ylmethyl]-1H-indol-3-yl]ethylamin,
N,N-Dimethyl-2-[5-(3-benzyl-1,2,4-oxadiazol-5-ylmethyl)-1H-indol-3-yl]ethylamin,
2-[5-[2-(3-Benzyl-1,2,4-oxadiazol-5-yl)ethyl]-1H-indol-3-yl]-ethylamin,
N,N-Dimethyl-2-[5-(3-methyl-1,2,4-oxadiazol-5-ylmethyl)-1H-indol-3-yl]ethylamin,
2-[5-(3-Diphenylmethyl-1,2,4-oxadiazol-5-yl)-1H-indol-3-yl]-ethylamin,
2-[5-(3-Phenyl-1,2,4-oxadiazol-5-yl)-1H-indol-3-yl]ethylamin,
2-[5-[3-(2-Methoxybenzyl)-1,2,4-oxadiazol-5-yl]-1H-indol-3-yl]-ethylamin,
2-[5-[3-(3-Benzyl-1,2,4-oxadiazol-5-yl)propyl]-1H-indol-3-yl]ethylamin,
2-[5-(3-Phenethyl-1,2,4-oxadiazol-5-yl)-1H-indol-3-yl]ethylamin,
2-[5-(5-Benzyl-1,2,4-oxadiazol-3-yl)-1H-indol-3-yl]ethylamin,
2-[5-(5-Benzyl-1,2,4-oxadiazol-3-ylmethyl)-1H-indol-3-yl]ethylamin,
N,N-Dimethyl-2-[5-[3-(2-methoxybenzyl)-1,2,4-oxadiazol-5-yl]-1H-indol-3-yl]ethylamin,
N,N-Dimethyl-2-[5-[2-(3-benzyl-1,2,4-oxadiazol-5-yl)ethyl]-1H-indol-3-yl]ethylamin,
2-[5-[3-(1-Naphthyl)-1,2,4-oxadiazol-5-ylmethyl]-1H-indol-3-yl]ethylamin,
2-[5-[3-(3-Methyl-1,2,4-oxadiazol-5-yl)propyl]-1H-indol-3-yl]-ethylamin,
2-[5-[3-(3-Cyclopropyl-1,2,4-oxadiazol-5-yl)propyl]-1H-indol-3-yl]ethylamin,
2-[5-[3-(3-Methoxybenzyl)-1,2,4-oxadiazol-5-yl]-1H-indol-3-yl]-ethylamin,
2-[5-[3-(4-Methoxybenzyl)-1,2,4-oxadiazol-5-yl]-1H-indol-3-yl]-ethylamin,
2-[5-[3-(4-Acetylaminobenzyl)-1,2,4-oxadiazol-5-yl]-1H-indol-3-yl]ethylamin,
2-[5-[3-(4-Methylsulfonylaminobenzyl)-1,2,4-oxadiazol-5-yl]-1H-indol-3-yl]ethylamin,
2-[5-[3-(3-Phenylpropyl)-1,2,4-oxadiazol-5-yl]-1H-indol-3-yl]-ethylamin,
2-[5-(3-Cyclopropyl-1,2,4-oxadiazol-5-yl)-1H-indol-3-yl]ethylamin,
2-[5-(3-Ethyl-1,2,4-oxadiazol-5-yl)-1H-indol-3-yl]ethylamin,
2-[5-[3-(4-Trifluormethylbenzyl)-1,2,4-oxadiazol-5-yl]-1H-indol-3-yl]ethylamin,
N,N-Dimethyl-2-[5-[3-(4-acetylaminobenzyl)-1,2,4-oxadiazol-5-yl]-1H-indol-3-yl]ethylamin,
N,N-Dimethyl-2-[5-[3-(4-methylsulfonylaminobenzyl)-1,2,4-oxadiazol-5-yl]-1H-indol-3-yl]ethylamin,
N,N-Dimethyl-2-[5-(3-amino-1,2,4-oxadiazol-5-ylmethyl)-1H-indol-3-yl]ethylamin,
2-[5-[2-(3-Amino-1,2,4-oxadiazol-5-yl)ethyl]-1H-indol-3-yl]-ethylamin,
2-[5-[2-(3-Dimethylamino-1,2,4-oxadiazol-5-yl)ethyl]-1H-indol-3-yl]ethylamin,
2-[5-(5-Methyl-1,2,4-oxadiazol-3-yl)-1H-indol-3-yl]ethylamin,
2-[5-(5-Methyl-1,2,4-oxadiazol-3-ylmethyl)-1H-indol-3-yl]-ethylamin,
N,N-Dimethyl-2-[5-(5-benzyl-1,2,4-oxadiazol-3-ylmethyl)-1H-indol-3-yl]ethylamin,
2-[5-(3-Methoxymethyl-1,2,4-oxadiazol-5-yl)-1H-indol-3-yl]-ethylamin,
2-[5-[3-(4-Methylaminocarbonylbenzyl)-1,2,4-oxadiazol-5-yl]-1H-indol-3-yl]ethylamin,
2-[5-[3-(4-Methylaminocarbonylphenyl)-1,2,4-oxadiazol-5-yl]-1H-indol-3-yl]ethylamin,
2-[5-[3-(4-Methylaminosulfonylbenzyl)-1,2,4-oxadiazol-5-yl]-1H-indol-3-yl]ethylamin,
2-[5-[3-(4-Methylsulfonylbenzyl)-1,2,4-oxadiazol-5-yl]-1H-indol-3-yl]ethylamin,
2-[5-[3-(3-Methylsulfonylaminobenzyl)-1,2,4-oxadiazol-5-yl]-1H-indol-3-yl]ethylamin,
2-[5-[3-(4-Aminocarbonylaminobenzyl)-1,2,4-oxadiazol-5-yl]-1H-indol-3-yl]ethylamin,
2-[5-(3-Amino-1,2,4-oxadiazol-5-yl)-1H-indol-3-yl]ethylamin,
2-[5-(3-Acetylaminomethyl-1,2,4-oxadiazol-5-yl)-1H-indol-3-yl]-ethylamin,
2-[5-[3-(2-Acetylaminoethyl)-1,2,4-oxadiazol-5-yl]-1H-indol-3-yl]ethylamin,
2-[5-(3-Aminomethyl-1,2,4-oxadiazol-5-yl)-1H-indol-3-yl]ethylamin,
N,N-Dimethyl-2-[5-(3-amino-1,2,4-oxadiazol-5-yl)-1H-indol-3-yl]ethylamin,
N,N-Dimethyl-2-[5-(3-acetylaminomethyl-1,2,4-oxadiazol-5-yl)-1H-indol-3-yl]ethylamin,
N,N-Dimethyl-2-[5-[3-(2-acetylaminoethyl)-1,2,4-oxadiazol-5-yl]-1H-indol-3-yl]ethylamin,
N,N-Dimethyl-2-[5-[3-(4-aminocarbonylaminobenzyl)-1,2,4-oxadiazol-5-yl]-1H-indol-3-yl]ethylamin,
N,N-Dimethyl-2-[5-[3-(2-(t-butoxycarbonylamino)ethyl)-1,2,4-oxadiazol-5-yl]-1H-indol-3-yl]ethylamin,
N,N-Dimethyl-2-[5-[3-(4-methylaminocarbonylbenzyl)-1,2,4-oxadiazol-5-yl]-1H-indol-3-yl]ethylamin,
N,N-Dimethyl-2-[5-[3-(2-aminoethyl)-1,2,4-oxadiazol-5-yl]-1H-indol-3-yl]ethylamin,
N,N-Dimethyl-2-[5-[3-(2-methylsulfonylaminoethyl)-1,2,4-oxadiazol-5-yl]-1H-indol-3-yl]ethylamin,
N,N-Dimethyl-2-[5-[3-(2-aminocarbonylaminoethyl)-1,2,4-oxadiazol-5-yl]-1H-indol-3-yl]ethylamin,
N,N-Dimethyl-2-[5-[3-(2-methylaminocarbonylaminoethyl)-1,2,4-oxadiazol-5-yl]-1H-indol-3-yl]ethylamin,
N,N-Dimethyl-2-[5-[3-(2-methylaminocarbonylaminoethyl)-1,2,4-oxadiazol-5-yl]-1H-indol-3-yl]ethylamin,
N,N-Dimethyl-2-[5-[3-(2-methoxycarbonylaminoethyl)-1,2,4-oxadiazol-5-yl]-1H-indol-3-yl]ethylamin,
N,N-Dimethyl-2-[5-[3-(2-ethoxycarbonylaminoethyl)-1,2,4-oxadiazol-5-yl]-1H-indol-3-yl]ethylamin,
N,N-Dimethyl-2-[5-[2-(3-amino-1,2,4-oxadiazol-5-yl)ethyl]-1H-indol-3-yl]ethylamin,
N,N-Dimethyl-2-[5-(3-methylamino-1,2,4-oxadiazol-5-ylmethyl)-1H-indol-3-yl]ethylamin,
N,N-Dimethyl-2-[5-[3-(4-aminocarbonylbenzyl)-1,2,4-oxadiazol-5-ylmethyl]-1H-indol-3-yl]ethylamin,
N,N-Dimethyl-2-[5-[3-(4-acetylaminobenzyl)-1,2,4-oxadiazol-5-ylmethyl]-1H-indol-3-yl]ethylamin,
N,N-Dimethyl-2-[5-[3-(4-methylaminosulfonylbenzyl)-1,2,4-oxadiazol-5-ylmethyl]-1H-indol-3-yl]ethylamin,
N,N-Dimethyl-2-[5-[3-(4-aminocarbonylaminobenzyl)-1,2,4-oxadiazol-5-ylmethyl]-1H-indol-3-yl]ethylamin,
N,N-Dimethyl-2-[5-[3-(4-methylsulfonylaminobenzyl)-1,2,4-oxadiazol-5-ylmethyl]-1H-indol-3-yl]ethylamin,
N,N-Dimethyl-2-[5-[3-(4-methylaminocarbonylphenyl)-1,2,4-oxadiazol-5-ylmethyl]-1H-indol-3-yl]ethylamin,
N,N-Dimethyl-2-[5-(3-acetylaminomethyl-1,2,4-oxadiazol-5-yl-methyl)-1H-indol-3-yl]ethylamin,
N,N-Dimethyl-2-[5-(3-methylsulfonylaminomethyl-1,2,4-oxadiazol-5-ylmethyl)-1H-indol-3-yl]ethylamin,
N,N-Dimethyl-2-[5-(3-aminocarbonylmethyl-1,2,4-oxadiazol-5-yl-methyl)-1H-indol-3-yl]ethylamin,
N,N-Dimethyl-2-[5-[3-(3-methylsulfonylaminobenzyl)-1,2,4-oxadiazol-5-ylmethyl]-1H-indol-3-yl]ethylamin,
N,N-Dimethyl-2-[5-[3-(3-acetylaminobenzyl)-1,2,4-oxadiazol-5-ylmethyl]-1H-indol-3-yl]ethylamin,
N,N-Dimethyl-2-[5-[3-(4-aminocarbonylphenyl)-1,2,4-oxadiazol-5-ylmethyl]-1H-indol-3-yl]ethylamin,
N,N-Dimethyl-2-[5-[3-(3-aminocarbonylphenyl)-1,2,4-oxadiazol-5-ylmethyl]-1H-indol-3-yl]ethylamin,
N,N-Dimethyl-2-[5-[3-(4-methylsulfonylaminophenyl)-1,2,4-oxadiazol-5-ylmethyl]-1H-indol-3-yl]ethylamin,
N,N-Dimethyl-2-[5-[3-(4-methylaminosulfonylmethylphenyl)-1,2,4-oxadiazol-5-ylmethyl]-1H-indol-3-yl]ethylamin,
N,N-Dimethyl-2-[5-[3-(3-methylaminosulfonylmethylphenyl)-1,2,4-oxadiazol-5-ylmethyl]-1H-indol-3-yl]ethylamin,
N,N-Dimethyl-2-[5-[3-(4-aminosulfonylmethylphenyl)-1,2,4-oxadiazol-5-ylmethyl]-1H-indol-3-yl]ethylamin,
N,N-Dimethyl-2-[5-[3-(4-dimethylaminosulfonylmethylphenyl)-1,2,4-oxadiazol-5-ylmethyl]-1H-indol-3-yl]ethylamin,
N,N-Dimethyl-2-[5-[3-(t-butoxycarbonylamino)methyl-1,2,4-oxadiazol-5-ylmethyl]-1H-indol-3-yl]ethylamin,
N,N-Dimethyl-2-[5-[3-(2-(t-butoxycarbonylamino)ethyl)-1,2,4-oxadiazol-5-ylmethyl]-1H-indol-3-yl]ethylamin,
N,N-Dimethyl-2-[5-(3-aminomethyl-1,2,4-oxadiazol-5-ylmethyl)-1H-indol-3-yl]ethylamin,
N,N-Dimethyl-2-[5-(3-methoxycarbonylaminomethyl-1,2,4-oxadiazol-5-ylmethyl)-1H-indol-3-yl]ethylamin,
N,N-Dimethyl-2-[5-(3-dimethylaminomethyl-1,2,4-oxadiazol-5-yl-methyl)-1H-indol-3-yl]ethylamin,
N,N-Dimethyl-2-[5-[3-(2-methylsulfonylaminoethyl)-1,2,4-oxadiazol-5-ylmethyl]-1H-indol-3-yl]ethylamin,
N,N-Dimethyl-2-[5-[3-(2-ethoxycarbonylaminoethyl)-1,2,4-oxadiazol-5-ylmethyl]-1H-indol-3-yl]ethylamin,
N,N-Dimethyl-2-[5-(3-benzoylaminomethyl-1,2,4-oxadiazol-5-yl-methyl)-1H-indol-3-yl]ethylamin,
N,N-Dimethyl-2-[5-[3-(2-benzoylaminoethyl)-1,2,4-oxadiazol-5-ylmethyl]-1H-indol-3-yl]ethylamin,
N,N-Dimethyl-2-[5-[3-(2-phenylaminocarbonylaminoethyl)-1,2,4-oxadiazol-5-ylmethyl]-1H-indol-3-yl]ethylamin,
N,N-Dimethyl-2-[5-[3-(2-(t-butylaminocarbonylamino)ethyl)-1,2,4-oxadiazol-5-ylmethyl]-1H-indol-3-yl]ethylamin,
N-Methyl-2-[5-(3-amino-1,2,4-oxadiazol-5-ylmethyl)-1H-indol-3-yl]ethylamin,
N,N-Dimethyl-2-[5-[3-(4-(t-butoxycarbonyl)piperazin-1-yl)-1,2,4-oxadiazol-5-ylmethyl]-1H-indol-3-yl]ethylamin,
N,N-Dimethyl-2-[5-[3-(4-methylsulfonylpiperazin-1-yl)-1,2,4-oxadiazol-5-ylmethyl]-1H-indol-3-yl]ethylamin,
N,N-Dimethyl-2-[5-[3-(4-methoxycarbonylpiperazin-1-yl)-1,2,4-oxadiazol-5-ylmethyl]-1H-indol-3-yl]ethylamin,
N,N-Dimethyl-2-[5-[3-(4-methylaminocarbonylpiperazin-1-yl)-1,2,4-oxadiazol-5-ylmethyl]-1H-indol-3-yl]ethylamin,
N,N-Dimethyl-2-[5-[3-(4-acetylpiperazin-1-yl)-1,2,4-oxadiazol-5-ylmethyl]-1H-indol-3-yl]ethylamin,
N,N-Dimethyl-2-[5-[3-(4-methylsulfonylaminomethylphenyl)-1,2,4-oxadiazol-5-yl-methyl]-1H-indol-3-yl]ethylamin,
N,N-Dimethyl-2-[5-(3-phenylsulfonylaminomethyl-1,2,4-oxadiazol-5-ylmethyl)-1H-indol-3-yl]ethylamin,
N,N-Dimethyl-2-[5-(3-benzylamino-1,2,4-oxadiazol-5-ylmethyl)-1H-indol-3-yl]ethylamin,
N,N-Dimethyl-2-[5-[3-(3-pyridyl)methyl-1,2,4-oxadiazol-5-yl-methyl]-1H-indol-3-yl]ethylamin,
N,N-Dimethyl-2-[5-[3-(2-methoxypyrid-5-yl)methyl-1,2,4-oxadiazol-5-ylmethyl]-1H-indol-3-yl]ethylamin,
2-[5-[3-(4-Acetylaminobenzyl)-1,2,4-oxadiazol-5-ylmethyl]-1H-indol-3-yl]ethylamin,
2-[5-[3-(4-Methylsulfonylaminobenzyl)-1,2,4-oxadiazol-5-yl-methyl]-1H-indol-3-yl]ethylamin,
2-[5-[2-[3-(4-Acetylaminobenzyl)-1,2,4-oxadiazol-5-yl]ethyl]-1H-indol-3-yl]ethylamin,
2-[5-[2-[3-(4-Methoxybenzyl)-1,2,4-oxadiazol-5-yl]ethyl]-1H-indol-3-yl]ethylamin,
N,N-Dimethyl-2-[5-(5-methyl-1,3-oxazol-2-yl)-1H-indol-3-yl]-ethylamin,
N,N-Dimethyl-2-[5-[2-(5-methyl-1,3-oxazol-2-yl)ethyl]-1H-indol-3-yl]ethylamin,
1-Methyl-4-[5-(3-amino-1,2,4-oxadiazol-5-yl)-1H-indol-3-yl]-piperidin,
1-Methyl-4-[5-(3-amino-1,2,4-oxadiazol-5-ylmethyl)-1H-indol-3-yl]piperidin,
1-Methyl-4-[5-[3-(4-methylsulfonylaminobenzyl)-1,2,4-oxadiazol-5-ylmethyl]-1H-indol-3-yl]piperidin,
1-Methyl-4-[5-[3-(3-pyridyl)methyl-1,2,4-oxadiazol-5-ylmethyl]-1H-indol-3-yl]piperidin,
N,N-Dimethyl-2-[5-[3-(4-pyridyl)methyl-1,2,4-oxadiazol-5-yl-methyl]-1H-indol-3-yl]ethylamin,
N,N-Dimethyl-2-[5-[3-(2-(t-butoxycarbonylamino)ethyl)amino-1,2,4-oxadiazol-5-ylmethyl]-1H-indol-3-yl]ethylamin,
2-[5-(3-Aminocarbonyl-1,2,4-oxadiazol-5-ylmethyl)-1H-indol-3-yl]ethylamin,
2-[5-(3-Methylaminocarbonyl-1,2,4-oxadiazol-5-ylmethyl)-1H-indol-3-yl]ethylamin,
2-[5-[3-(Pyrrolid-1-yl)carbonyl-1,2,4-oxadiazol-5-ylmethyl]-1H-indol-3-yl]ethylamin,
2-[5-[3-(Azetidin-1-yl)carbonyl-1,2,4-oxadiazol-5-ylmethyl]-1H-indol-3-yl]ethylamin,
N,N-Dimethyl-2-[5-[3-(4-phenylsulfonylpiperazin-1-yl)-1,2,4-oxadiazol-5-ylmethyl]-1H-indol-3-yl]ethylamin,
N,N-Dimethyl-2-[5-[3-(2-(pyrrolid-1-ylcarbonylamino)ethyl)-1,2,4-oxadiazol-5-ylmethyl]-1H-indol-3-yl]ethylamin,
N,N-Dimethyl-2-[5-[3-(2-methylsulfonylaminoethyl)amino-1,2,4-oxadiazol-5-ylmethyl]-1H-indol-3-yl]ethylamin,
N,N-Dimethyl-2-[5-(3-amino-1,4-thiadiazol-5-ylmethyl)-1H-indol-3-yl]ethylamin
und von Salzen davon.

7. Ein Verfahren zur Herstellung einer pharmazeutischen Zusammensetzung, das das Vermischen einer Verbindung, hergestellt wie in irgendeinem der vorhergehenden Ansprüche beansprucht, mit einem pharmazeutisch annehmbaren Träger oder Hilfsstoff umfaßt.

8. Die Verwendung einer Verbindung, hergestellt wie in irgendeinem der Ansprüche 1 bis 6 beansprucht, zur Herstellung eines Medikaments zur Behandlung und/oder Prävention von klinischen Zuständen, für die ein selektiver Agonist von 5-HT₁-ähnlichen Rezeptoren indiziert ist.

## Revendications (Revendications pour l'(les) Etat(s) contractant(s) suivant(s): AT, BE, CH, DE, DK, FR, GB, IT, LI, LU, NL, SE)

1. Composé de formule I, ou un de ses sels: 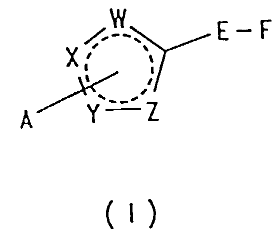 dans laquelle le cercle en pointillés représente deux doubles liaisons non adjacentes en n'importe quelle position dans le noyau à cinq chaînons;
le noyau à cinq chaînons portant les substituants W à Z représente un noyau 1,2,4-oxadiazole, 1,3,4-oxadiazole, 1,2,4-thiadiazole, 1,3,4-thiadiazole, 1,3-oxazole ou 1,3-thiazole;
A représente un groupe méthyle, méthoxyméthyle, aminométhyle, diméthylaminométhyle, acétylaminométhyle, benzoylaminométhyle, t-butoxycarbonylaminométhyle, méthylsulfonylaminométhyle, phénylsulfonylaminométhyle, aminocarbonylméthyle, éthyle, aminoéthyle, acétylaminoéthyle, benzoylaminoéthyle, méthoxycarbonylaminoéthyle, éthoxycarbonylaminoéthyle, t-butoxycarbonylaminoéthyle, méthylsulfonylaminoéthyle, aminocarbonylaminoéthyle, méthylaminocarbonylaminoéthyle, t-butylaminocarbonylaminoéthyle, phénylaminocarbonylaminoéthyle, pyrrolidylcarbonylaminoéthyle, cyclopropyle, phényle, méthylsulfonylaminophényle, aminocarbonylphényle, méthylaminocarbonylphényle, méthylsulfonylaminométhylphényle, aminosulfonylméthylphényle, méthylaminosulfonylméthylphényle, diméthylaminosulfonylméthylphényle, naphtyle, benzyle, diphénylméthyle, trifluorométhylbenzyle, méthoxybenzyle, acétylaminobenzyle, méthylsulfonylaminobenzyle, aminocarbonylaminobenzyle, aminocarbonylbenzyle, méthylaminocarbonylbenzyle, méthylsulfonylbenzyle, méthylaminosulfonylbenzyle, phénéthyle, phénylpropyle, acétylpipérazinyle, méthoxycarbonylpipérazinyle, t-butoxycarbonylpipérazinyle, méthylaminocarbonylpipérazinyle, méthylsulfonylpipérazinyle, phénylsulfonylpipérazinyle, pyridylméthyle, méthoxypyridylméthyle, amino, méthylamino, benzylamino, diméthylamino, t-butoxycarbonylaminoéthylamino, méthylsulfonylaminoéthylamino, aminocarbonyle, méthylaminocarbonyle, azétidinylcarbonyle ou pyrrolidylcarbonyle;
E représente une liaison ou une chaîne alkylène droite ou ramifiée contenant 1 à 4 atomes de carbone;
F représente un groupe de formule 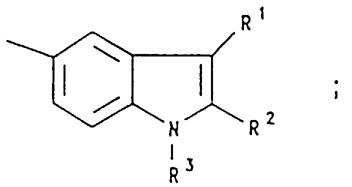
R¹ représente un groupe aminoéthyle, N-méthylaminoéthyle, N,N-diméthylaminoéthyle ou 1-méthyl-4-pipéridyle; et
R² et R³ représentent indépendamment un atome d'hydrogène ou un groupe alkyle en C₁-C₆.

2. Composé selon la revendication 1 représenté par la formule IIA, et ses sels: 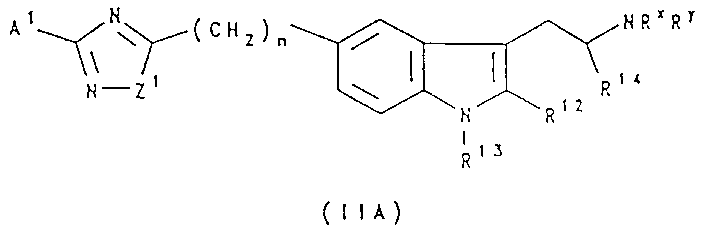 dans laquelle
Z¹ représente un atome d'oxygène ou de soufre;
n est nul ou vaut 1, 2 ou 3;
A¹ correspond au groupe A tel que défini dans la revendication 1;
R¹², R¹³ et R¹⁴ représentent chacun un atome d'hydrogène; et
R^{X} et R^{y} représentent indépendamment un atome d'hydrogène ou un groupe méthyle.

3. Composé selon la revendication 1 représenté par la formule IIB, et ses sels: 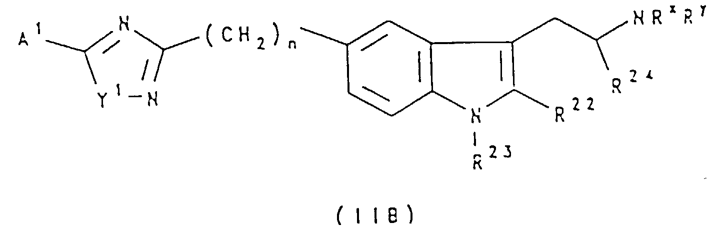 dans laquelle
Y¹ représente un atome d'oxygène ou de soufre;
n est nul ou vaut 1, 2 ou 3;
A¹ est tel que défini dans la revendication 2;
R²², R²³ et R²⁴ représentent chacun un atome d'hydrogène; et
R^{x} et R^{y} représentent indépendamment un atome d'hydrogène ou un groupe méthyle.

4. Composé selon la revendication 1 représenté par la formule IIC, et ses sels: 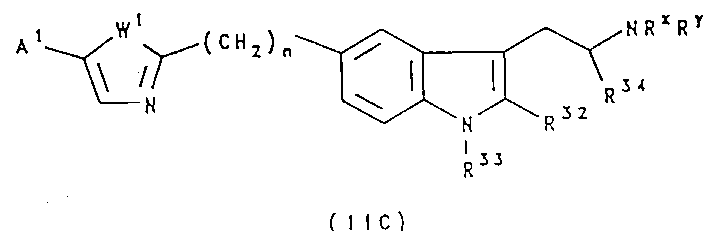 dans laquelle
W¹ représente un atome d'oxygène ou de soufre;
n est nul ou vaut 1, 2 ou 3;
A¹ est tel que défini dans la revendication 2;
R³², R³³ et R³⁴ représentent chacun un atome d'hydrogène; et
R^{x} et R^{y} représentent indépendamment un atome d'hydrogène ou un groupe méthyle.

5. Composé selon la revendication 1 représenté par la formule IID, et ses sels: 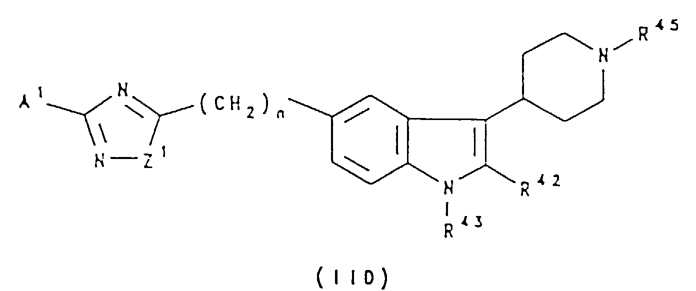 dans laquelle
Z¹ représente un atome d'oxygène ou de soufre;
n est nul ou vaut 1, 2 ou 3;
A¹ est tel que défini dans la revendication 2;
R⁴² et R⁴³ représentent chacun un atome d'hydrogène; et
R⁴⁵ représente un groupe méthyle.

6. Composé selon la revendication 1, choisi parmi:
la 2-[5-(3-benzyl-1,2,4-oxadiazole-5-yl)-1H-indole-3-yl]-éthylamine;
la 2-[5-(3-méthyl-1,2,4-oxadiazole-5-yl)-1H-indole-3-yl]-éthylamine;
la N,N-diméthyl-2-[5-(3-benzyl-1,2,4-oxadiazole-5-yl)-1H-indole-3-yl]éthylamine;
la 2-[5-(3-benzyl-1,2,4-oxadiazole-5-ylméthyl)-1H-indole-3-yl]éthylamine;
la 2-[5-(3-méthyl-1,2,4-oxadiazole-5-ylméthyl)-1H-indole-3-yl]éthylamine;
la 2-[5-(3-amino-1,2,4-oxadiazole-5-ylméthyl)-1H-indole-3-yl]éthylamine;
la 2-[5-(3-phényl-1,2,4-oxadiazole-5-ylméthyl)-1H-indole-3-yl]éthylamine;
la 2-[5-[3-(2-méthoxybenzyl)-1,2,4-oxadiazole-5-ylméthyl]-1H-indole-3-yl]éthylamine;
la N,N-diméthyl-2-[5-(3-benzyl-1,2,4-oxadiazole-5-ylméthyl)-1H-indole-3-yl]éthylamine;
la 2-[5-[2-(3-benzyl-1,2,4-oxadiazole-5-yl)éthyl]-1H-indole-3-yl]éthylamine;
la N,N-diméthyl-2-[5-(3-méthyl-1,2,4-oxadiazole-5-ylméthyl)-1H-indole-3-yl]éthylamine;
la 2-[5-(3-diphénylméthyl-1,2,4-oxadiazole-5-yl)-1H-indole-3-yl]éthylamine;
la 2-[5-(3-phényl-1,2,4-oxadiazole-5-yl)-1H-indole-3-yl]-éthylamine;
la 2-[5-[3-(2-méthoxybenzyl)-1,2,4-oxadiazole-5-yl]-1H-indole-3-yl]éthylamine;
la 2-[5-[3-(3-benzyl-1,2,4-oxadiazole-5-yl)propyl]-1H-indole-3-yl]éthylamine;
la 2-[5-(3-phénéthyl-1,2,4-oxadiazole-5-yl)-1H-indole-3-yl]-éthylamine;
la 2-[5-(5-benzyl-1,2,4-oxadiazole-3-yl)-1H-indole-3-yl]-éthylamine;
la 2-[5-(5-benzyl-1,2,4-oxadiazole-3-ylméthyl)-1H-indole-3-yl]éthylamine;
la N,N-diméthyl-2-[5-[3-(2-méthoxybenzyl)-1,2,4-oxadiazole-5-yl]-1H-indole-3-yl]éthylamine;
la N,N-diméthyl-2-[5-[2-(3-benzyl-1,2,4-oxadiazole-5-yl)-éthyl]-1H-indole-3-yl]éthylamine;
la 2-[5-[3-(1-naphtyl)-1,2,4-oxadiazole-5-ylméthyl]-1H-indole-3-yl]éthylamine;
la 2-[5-[3-(3-méthyl-1,2,4-oxadiazole-5-yl)propyl]-1H-indole-3-yl]ethylamine;
la 2-[5-[3-(3-cyclopropyl-1,2,4-oxadiazole-5-yl)propyl]-1H-indole-3-yl]éthylamine;
la 2-[5-[3-(3-méthoxybenzyl)-1,2,4-oxadiazole-5-yl]-1H-indole-3-yl]éthylamine;
la 2-[5-[3-(4-méthoxybenzyl)-1,2,4-oxadiazole-5-yl]-1H-indole-3-yl]éthylamine;
la 2-[5-[3-(4-acétylaminobenzyl)-1,2,4-oxadiazole-5-yl]-1H-indole-3-yl]éthylamine;
la 2-[5-[3-(4-méthylsulfonylaminobenzyl)-1,2,4-oxadiazole-5-yl]-1H-indole-3-yl]éthylamine;
la 2-[5-[3-(3-phénylpropyl)-1,2,4-oxadiazole-5-yl]-1H-indole-3-yl]éthylamine;
la 2-[5-(3-cyclopropyl-1,2,4-oxadiazole-5-yl)-1H-indole-3-yl]éthylamine;
la 2-[5-(3-éthyl-1,2,4-oxadiazole-5-yl)-1H-indole-3-yl]-éthylamine;
la 2-[5-[3-(4-trifluorométhylbenzyl)-1,2,4-oxadiazole-5-yl]-1H-indole-3-yl]éthylamine;
la N,N-diméthyl-2-[5-[3-(4-acétylaminobenzyl)-1,2,4-oxadiazole-5-yl]-1H-indole-3-yl]éthylamine;
la N,N-diméthyl-2-[5-[3-(4-méthylsulfonylaminobenzyl)-1,2,4-oxadiazole-5-yl]-1H-indole-3-yl]éthylamine;
la N,N-diméthyl-2-[5-(3-amino-1,2,4-oxadiazole-5-ylméthyl)-1H-indole-3-yl]éthylamine;
la 2-[5-[2-(3-amino-1,2,4-oxadiazole-5-yl)éthyl]-1H-indole-3-yl]éthylamine;
la 2-[5-[2-(3-diméthylamino-1,2,4-oxadiazole-5-yl)éthyl]-1H-indole-3-yl]éthylamine;
la 2-[5-(5-méthyl-1,2,4-oxadiazole-3-yl)-1H-indole-3-yl]-éthylamine;
la 2-[5-(5-méthyl-1,2,4-oxadiazole-3-ylméthyl)-1H-indole-3-yl]éthylamine;
la N,N-diméthyl-2-[5-(5-benzyl-1,2,4-oxadiazole-3-ylméthyl)-1H-indole-3-yl]éthylamine;
la 2-[5-(3-méthoxyméthyl-1,2,4-oxadiazole-5-yl)-1H-indole-3-yl]éthylamine;
la 2-[5-[3-(4-méthylaminocarbonylbenzyl)-1,2,4-oxadiazole-5-yl]-1H-indole-3-yl]éthylamine;
la 2-[5-[3-(4-méthylaminocarbonylphényl)-1,2,4-oxadiazole-5-yl]-1H-indole-3-yl]éthylamine;
la 2-[5-[3-(4-méthylaminosulfonylbenzyl)-1,2,4-oxadiazole-5-yl]-1H-indole-3-yl]éthylamine;
la 2-[5-[3-(4-méthylsulfonylbenzyl)-1,2,4-oxadiazole-5-yl]-1H-indole-3-yl]éthylamine;
la 2-[5-[3-(3-méthylsulfonylaminobenzyl)-1,2,4-oxadiazole-5-yl]-1H-indole-3-yl]éthylamine;
la 2-[5-[3-(4-aminocarbonylaminobenzyl)-1,2,4-oxadiazole-5-yl]-1H-indole-3-yl]éthylamine;
la 2-[5-(3-amino-1,2,4-oxadiazole-5-yl)-1H-indole-3-yl]éthylamine;
la 2-[5-(3-acétylaminométhyl-1,2,4-oxadiazole-5-yl)-1H-indole-3-yl]éthylamine;
la 2-[5-[3-(2-acétylaminoéthyl)-1,2,4-oxadiazole-5-yl]-1H-indole-3-yl]éthylamine;
la 2-[5-(3-aminométhyl-1,2,4-oxadiazole-5-yl)-1H-indole-3-yl]éthylamine;
la N,N-diméthyl-2-[5-(3-amino-1,2,4-oxadiazole-5-yl)-1H-indole-3-yl]éthylamine;
la N,N-diméthyl-2-[5-(3-acétylaminométhyl-1,2,4-oxqdiazole-5-yl)-1H-indole-3-yl]éthylamine;
la N,N-diméthyl-2-[5-[3-(2-acétylaminoéthyl)-1,2,4-oxadiazole-5-yl]-1H-indole-3-yl]éthylamine;
la N,N-diméthyl-2-[5-[3-(4-aminocarbonylaminobenzyl)-1,2,4-oxadiazole-5-yl]-1H-indole-3-yl]éthylamine;
la N,N-diméthyl-2-[5-[3-(2-(t-butoxycarbonylamino)éthyl)-1,2,4-oxadiazole-5-yl]-1H-indole-3-yl]éthylamine;
la N,N-diméthyl-2-[5-[3-(4-méthylaminocarbonylbenzyl)-1,2,4-oxadiazole-5-yl]-1H-indole-3-yl]éthylamine;
la N,N-diméthyl-2-[5-[3-(2-aminoéthyl)-1,2,4-oxadiazole-5-yl]-1H-indole-3-yl]éthylamine;
la N,N-diméthyl-2-[5-[3-(2-méthylsulfonylaminoéthyl)-1,2,4-oxadiazole-5-yl]-1H-indole-3-yl]éthylamine;
la N,N-diméthyl-2-[5-[3-(2-aminocarbonylaminoéthyl)-1,2,4-oxadiazole-5-yl]-1H-indole-3-yl]éthylamine;
la N,N-diméthyl-2-[5-[3-(2-méthylaminocarbonylaminoéthyl)-1,2,4-oxadiazole-5-yl]-1H-indole-3-yl]éthylamine;
la N,N-diméthyl-2-[5-[3-(2-méthylaminocarbonylaminoéthyl)-1,2,4-oxadiazole-5-yl]-1H-indole-3-yl]éthylamine;
la N,N-diméthyl-2-[5-[3-(2-méthoxycarbonylaminoéthyl)-1,2,4-oxadiazole-5-yl]-1H-indole-3-yl]éthylamine;
la N,N-diméthyl-2-[5-[3-(2-éthoxycarbonylaminoéthyl)-1,2,4-oxadiazole-5-yl]-1H-indole-3-yl]éthylamine;
la N,N-diméthyl-2-[5-[2-(3-amino-1,2,4-oxadiazole-5-yl)éthyl]-1H-indole-3-yl]éthylamine;
la N,N-diméthyl-2-[5-(3-méthylamino-1,2,4-oxadiazole-5-yl-méthyl)-1H-indole-3-yl]éthylamine;
la N,N-diméthyl-2-[5-[3-(4-aminocarbonylbenzyl)-1,2,4-oxadiazole-5-ylméthyl]-1H-indole-3-yl]éthylamine;
la N,N-diméthyl-2-[5-[3-(4-acétylaminobenzyl)-1,2,4-oxadiazole-5-ylméthyl]-1H-indole-3-yl]éthylamine;
la N,N-diméthyl-2-[5-[3-(4-méthylaminosulfonylbenzyl)-1,2,4-oxadiazole-5-ylméthyl]-1H-indole-3-yl]éthylamine;
la N,N-diméthyl-2-[5-[3-(4-aminocarbonylaminobenzyl)-1,2,4-oxadiazole-5-ylméthyl]-1H-indole-3-yl]éthylamine;
la N,N-diméthyl-2-[5-[3-(4-méthylsulfonylaminobenzyl)-1,2,4-oxadiazole-5-ylméthyl]-1H-indole-3-yl]éthylamine;
la N,N-diméthyl-2-[5-[3-(4-méthylaminocarbonylphényl)-1,2,4-oxadiazole-5-ylméthyl]-1H-indole-3-yl]éthylamine;
la N,N-diméthyl-2-[5-(3-acétylaminométhyl-1,2,4-oxadiazole-5-ylméthyl)-1H-indole-3-yl]éthylamine;
la N,N-diméthyl-2-[5-(3-méthylsulfonylaminométhyl-1,2,4-oxadiazole-5-ylméthyl)-1H-indole-3-yl]éthylamine;
la N,N-diméthyl-2-[5-(3-aminocarbonylméthyl-1,2,4-oxadiazole-5-ylméthyl)-1H-indole-3-yl]éthylamine;
la N,N-diméthyl-2-[5-[3-(3-méthylsulfonylaminobenzyl)-1,2,4-oxadiazole-5-ylméthyl]-1H-indole-3-yl]éthylamine;
la N,N-diméthyl-2-[5-[3-(3-acétylaminobenzyl)-1,2,4-oxadiazole-5-ylméthyl]-1H-indole-3-yl]éthylamine;
la N,N-diméthyl-2-[5-[3-(4-aminocarbonylphényl)-1,2,4-oxadiazole-5-ylméthyl]-1H-indole-3-yl]éthylamine;
la N,N-diméthyl-2-[5-[3-(3-aminocarbonylphényl)-1,2,4-oxadiazole-5-ylméthyl]-1H-indole-3-yl]éthylamine;
la N,N-diméthyl-2-[5-[3-(4-méthylsulfonylaminophényl)-1,2,4-oxadiazole-5-ylméthyl]-1H-indole-3-yl]éthylamine;
la N,N-diméthyl-2-[5-[3-(4-méthylaminosulfonylméthylphényl)-1,2,4-oxadiazole-5-ylméthyl]-1H-indole-3-yl]éthylamine;
la N,N-diméthyl-2-[5-[3-(3-méthylaminosulfonylméthylphényl)-1,2,4-oxadiazole-5-ylméthyl]-1H-indole-3-yl]éthylamine;
la N,N-diméthyl-2-[5-[3-(4-aminosulfonylméthylphényl)-1,2,4-oxadiazole-5-ylméthyl]-1H-indole-3-yl]éthylamine;
la N,N-diméthyl-2-[5-[3-(4-diméthylaminosulfonylméthylphényl)-1,2,4-oxadiazole-5-ylméthyl]-1H-indole-3-yl]éthylamine;
la N,N-diméthyl-2-[5-[3-(t-butoxycarbonylamino)méthyl-1,2,4-oxadiazole-5-ylméthyl]-1H-indole-3-yl]éthylamine;
la N,N-diméthyl-2-[5-[3-(2-(t-butoxycarbonylamino)éthyl)-1,2,4-oxadiazole-5-ylméthyl]-1H-indole-3-yl]éthylamine;
la N,N-diméthyl-2-[5-(3-aminométhyl-1,2,4-oxadiazole-5-ylméthyl)-1H-indole-3-yl]éthylamine;
la N,N-diméthyl-2-[5-(3-méthoxycarbonylaminométhyl-1,2,4-oxadiazole-5-ylméthyl)-1H-indole-3-yl]éthylamine;
la N,N-diméthyl-2-[5-(3-diméthylaminométhyl-1,2,4-oxadiazole-5-ylméthyl)-1H-indole-3-yl]éthylamine;
la N,N-diméthyl-2-[5-[3-(2-méthylsulfonylaminoéthyl)-1,2,4-oxadiazole-5-ylméthyl]-1H-indole-3-yl]éthylamine;
la N,N-diméthyl-2-[5-[3-(2-éthoxycarbonylaminoéthyl)-1,2,4-oxadiazole-5-ylméthyl]-1H-indole-3-yl]éthylamine;
la N,N-diméthyl-2-[5-(3-benzoylaminométhyl-1,2,4-oxadiazole-5-ylméthyl)-1H-indole-3-yl]éthylamine;
la N,N-diméthyl-2-[5-[3-(2-benzoylaminoéthyl)-1,2,4-oxadiazole-5-ylméthyl]-1H-indole-3-yl]éthylamine;
la N,N-diméthyl-2-[5-[3-(2-phénylaminocarbonylaminoéthyl)-1,2,4-oxadiazole-5-ylméthyl]-1H-indole-3-yl]éthylamine;
la N,N-diméthyl-2-[5-[3-(2-(t-butylaminocarbonylamino)éthyl)-1,2,4-oxadiazole-5-ylméthyl]-1H-indole-3-yl]éthylamine;
la N-méthyl-2-[5-(3-amino-1,2,4-oxadiazole-5-ylméthyl)-1H-indole-3-yl]éthylamine;
la N,N-diméthyl-2-[5-[3-(4-(t-butoxycarbonyl)pipérazine-1-yl)-1,2,4-oxadiazole-5-ylméthyl]-1H-indole-3-yl]éthylamine;
la N,N-diméthyl-2-[5-[3-(4-méthylsulfonylpipérazine-1-yl)-1,2,4-oxadiazole-5-ylméthyl]-1H-indole-3-yl]éthylamine;
la N,N-diméthyl-2-[5-[3-(4-méthoxycarbonylpipérazine-1-yl)-1,2,4-oxadiazole-5-ylméthyl]-1H-indole-3-yl]éthylamine;
la N,N-diméthyl-2-[5-[3-(4-méthylaminocarbonylpipérazine-1-yl)-1,2,4-oxadiazole-5-ylméthyl]-1H-indole-3-yl]éthylamine;
la N,N-diméthyl-2-[5-[3-(4-acétylpipérazine-1-yl)-1,2,4-oxadiazole-5-ylméthyl]-1H-indole-3-yl]éthylamine;
la N,N-diméthyl-2-[5-[3-(4-méthylsulfonylaminométhylphényl)-1,2,4-oxadiazole-5-ylméthyl]-1H-indole-3-yl]éthylamine;
la N,N-diméthyl-2-[5-(3-phénylsulfonylaminométhyl-1,2,4-oxadiazole-5-ylméthyl]-1H-indole-3-yl]éthylamine;
la N,N-diméthyl-2-[5-[3-benzylamino-1,2,4-oxadiazole-5-yl-méthyl]-1H-indole-3-yl]éthylamine;
la N,N-diméthyl-2-[5-[3-(3-pyridylméthyl)-1,2,4-oxadiazole-5-ylméthyl]-1H-indole-3-yl]éthylamine;
la N,N-diméthyl-2-[5-[3-(2-méthoxypyrid-5-yl)méthyl-1,2,4-oxadiazole-5-ylméthyl]-1H-indole-3-yl]éthylamine;
la 2-[5-[3-(4-acétylaminobenzyl)-1,2,4-oxadiazole-5-ylméthyl]-1H-indole-3-yl]éthylamine;
la 2-[5-[3-(4-méthylsulfonylaminobenzyl)-1,2,4-oxadiazole-5-ylméthyl]-1H-indole-3-yl]éthylamine;
la 2-[5-[2-[3-(4-acétylaminobenzyl)-1,2,4-oxadiazole-5-yl]éthyl]-1H-indole-3-yl]éthylamine;
la 2-[5-[2-[3-(4-méthoxybenzyl)-1,2,4-oxadiazole-5-yl]éthyl]-1H-indole-3-yl]éthylamine;
la N,N-diméthyl-2-[5-(5-méthyl-1,3-oxazole-2-yl)-1H-indole-3-yl]éthylamine;
la N,N-diméthyl-2-[5-[2-(5-méthyl-1,3-oxazole-2-yl)éthyl]-1H-indole-3-yl]éthylamine;
la 1-méthyl-4-[5-(3-amino-1,2,4-oxadiazole-5-yl)-1H-indole-3-yl]pipéridine;
la 1-méthyl-4-[5-(3-amino-1,2,4-oxadiazole-5-ylméthyl)-1H-indole-3-yl]pipéridine;
la 1-méthyl-4-[5-(3-(4-méthylsulfonylaminobenzyl)-1,2,4-oxadiazole-5-ylméthyl]-1H-indole-3-yl]pipéridine;
la 1-méthyl-4-[5-(3-(3-pyridyl)méthyl-1,2,4-oxadiazole-5-ylméthyl]-1H-indole-3-yl]pipéridine;
la N,N-diméthyl-2-[5-[3-(4-pyridyl)méthyl-1,2,4-oxadiazole-5-ylméthyl)-1H-indole-3-yl]éthylamine;
la N,N-diméthyl-2-[5-[3-(2-(t-butoxycarbonylamino)éthyl)amino-1,2,4-oxadiazole-5-ylméthyl)-1H-indole-3-yl]éthylamine;
la 2-[5-(3-aminocarbonyl-1,2,4-oxadiazole-5-ylméthyl)-1H-indole-3-yl]éthylamine;
la 2-[5-(3-méthylaminocarbonyl-1,2,4-oxadiazole-5-ylméthyl)-1H-indole-3-yl]éthylamine;
la 2-[5-[3-(pyrrolid-1-yl)carbonyl-1,2,4-oxadiazole-5-ylméthyl]-1H-indole-3-yl]éthylamine;
la 2-[5-[3-(azétidin-1-yl)carbonyl-1,2,4-oxadiazole-5-ylméthyl]-1H-indole-3-yl]éthylamine;
la N,N-diméthyl-2-[5-[3-(4-phénylsulfonylpipérazine-1-yl)-1,2,4-oxadiazole-5-ylméthyl]-1H-indole-3-yl]éthylamine;
la N,N-diméthyl-2-[5-[3-(2-(pyrrolid-1-ylcarbonylamino)éthyl)-1,2,4-oxadiazole-5-ylméthyl]-1H-indole-3-yl]éthylamine;
la N,N-diméthyl-2-[5-[3-(2-méthylsulfonylaminoéthyl)amino-1,2,4-oxadiazole-5-ylméthyl]-1H-indole-3-yl]éthylamine;
la N,N-diméthyl-2-[5-(3-amino-1,4-thiadiazole-5-ylméthyl)-1H-indole-3-yl]éthylamine;
et leurs sels.

7. Composition pharmaceutique comprenant un composé selon l'une quelconque des revendications précédentes en association avec un véhicule ou excipient pharmaceutiquement acceptable.

8. Composition selon l'une quelconque des revendications 1 à 6 à utiliser en thérapeutique.

9. Utilisation d'un composé selon l'une quelconque des revendications 1 à 6 pour la fabrication d'un médicament pour le traitement et/ou la prévention de conditions cliniques pour lesquelles un agoniste sélectif des récepteurs de type 5-HT₁ est indiqué.

10. Procédé de préparation d'un composé selon l'une quelconque des revendications 1 à 6 qui comprend le fait de:
(A) faire réagir un dérivé réactif d'un acide carboxylique de formule R^{c}-CO₂H avec un composé de formule III ou de formule IV, ou un de ses sels: 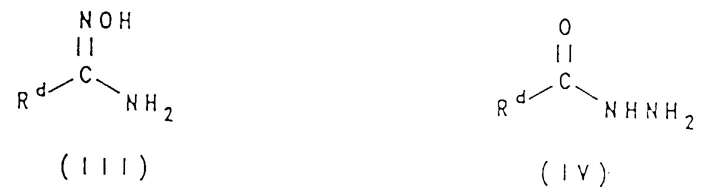 dans lesquelles un des radicaux R^{c} et R^{d} est un groupe de formule A, et l'autre est un groupe de formule -E-F tel que défini dans la revendication 1; ou
(B) cycliser un composé de formule XI: 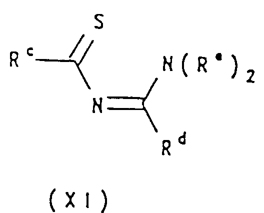 dans laquelle R^{c} et R^{d} sont tels que définis ci-dessus, et R^{e} est un atome d'hydrogène ou un groupe alkyle; ou
(C) réaliser une cycloaddition d'un sulfure de nitrile R^{c}-C≡N⁺ S⁻ avec un nitrile de formule R^{d}-CN, où R^{c} et R^{d} sont tels que définis ci-dessus; ou
(D) déshydrater un thiosemicarbazide de formule R^{c}CSNHNHCONR^{s}R^{t}, où R^{c} est tel que défini ci-dessus, et R^{s} et R^{t} sont un atome d'hydrogène ou un groupe alkyle; puis fixer le groupe R^{d} par un moyen classique; ou
(E) faire réagir un amide ou un thioamide de formule XII avec une α-halogénocétone de formule XIII: 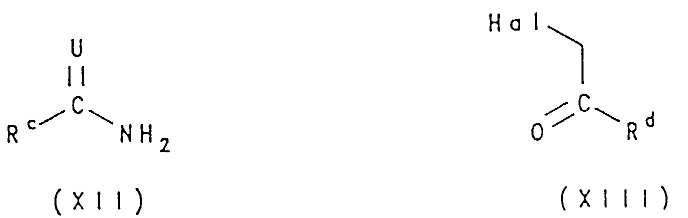 dans lesquelles U est un atome d'oxygène ou de soufre; Hal représente un atome d'halogène, et R^{c} et R^{d} sont tels que définis ci-dessus; ou
(F) faire réagir un composé de formule XV: 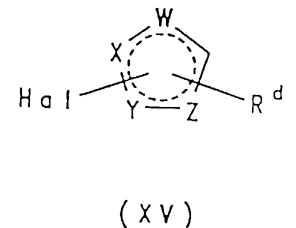 avec un réactif qui fournit un anion ⁻R^{c}, où W, X, Y et Z sont tels que définis dans la revendication 1, et R^{c} et R^{d} sont tels que définis précédemment, et Hal représente un atome d'halogène; ou
(G) faire réagir un composé de formule XVI: 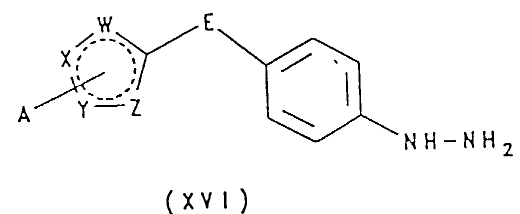
dans laquelle W, X, Y, Z, A et E sont tels que définis dans la revendication 1, avec un composé de formule VII ou une forme protégée par un groupe carbonyle de celui-ci: 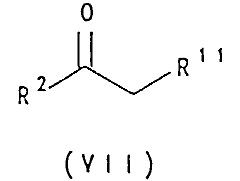 dans laquelle R² est tel que défini dans la revendication 1, et R¹¹ correspond au groupe R¹ défini dans la revendication 1, ou représente un groupe de formule -CH₂CHR⁴D, dans laquelle R⁴ est un atome d'hydrogène et D représente un groupe facile à éliminer; puis, le cas échéant, réaliser une N-alkylation par des procédés standards pour introduire le groupement R³ défini dans la revendication 1.

## Revendications (Revendications pour l'(les) Etat(s) contractant(s) suivant(s): ES, GR)

1. Procédé de préparation d'un composé de formule I, ou un de ses sels: 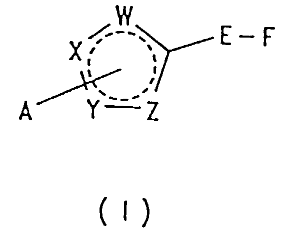 dans laquelle le cercle en pointillés représente deux doubles liaisons non adjacentes en n'importe quelle position dans le noyau à cinq chaînons;
le noyau à cinq chaînons portant les substituants W à Z représente un noyau 1,2,4-oxadiazole, 1,3,4-oxadiazole, 1,2,4-thiadiazole, 1,3,4-thiadiazole, 1,3-oxazole ou 1,3-thiazole;
A représente un groupe méthyle, méthoxyméthyle, aminométhyle, diméthylaminométhyle, acétylaminométhyle, benzoylaminométhyle, t-butoxycarbonylaminométhyle, méthylsulfonylaminométhyle, phénylsulfonylaminométhyle, aminocarbonylméthyle, éthyle, aminoéthyle, acétylaminoéthyle, benzoylaminoéthyle, méthoxycarbonylaminoéthyle, éthoxycarbonylaminoéthyle, t-butoxycarbonylaminoéthyle, méthylsulfonylaminoéthyle, aminocarbonylaminoéthyle, méthylaminocarbonylaminoéthyle, t-butylaminocarbonylaminoéthyle, phénylaminocarbonylaminoéthyle, pyrrolidylcarbonylaminoéthyle, cyclopropyle, phényle, méthylsulfonylaminophényle, aminocarbonylphényle, méthylaminocarbonylphényle, méthylsulfonylaminométhylphényle, aminosulfonylméthylphényle, méthylaminosulfonylméthylphényle, diméthylaminosulfonylméthylphényle, naphtyle, benzyle, diphénylméthyle, trifluorométhylbenzyle, méthoxybenzyle, acétylaminobenzyle, méthylsulfonylaminobenzyle, aminocarbonylaminobenzyle, aminocarbonylbenzyle, méthylaminocarbonylbenzyle, méthylsulfonylbenzyle, méthylaminosulfonylbenzyle, phénéthyle, phénylpropyle, acétylpipérazinyle, méthoxycarbonylpipérazinyle, t-butoxycarbonylpipérazinyle, méthylaminocarbonylpipérazinyle, méthylsulfonylpipérazinyle, phénylsulfonylpipérazinyle, pyridylméthyle, méthoxypyridylméthyle, amino, méthylamino, benzylamino, diméthylamino, t-butoxycarbonylaminoéthylamino, méthylsulfonylaminoéthylamino, aminocarbonyle, méthylaminocarbonyle, azétidinylcarbonyle ou pyrrolidylcarbonyle;
E représente une liaison ou une chaîne alkylène droite ou ramifiée contenant 1 à 4 atomes de carbone;
F représente un groupe de formule 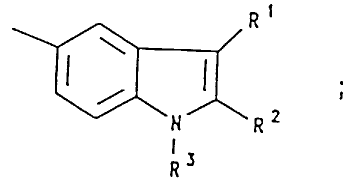
R¹ représente un groupe aminoéthyle, N-méthylaminoéthyle, N,N-diméthylaminoéthyle ou 1-méthyl-4-pipéridyle; et
R² et R³ représentent indépendamment un atome d'hydrogène ou un groupe alkyle en C₁-C₆;
procédé qui comprend le fait de:
(A) faire réagir un dérivé réactif d'un acide carboxylique de formule R^{c}-CO₂H avec un composé de formule III ou de formule IV, ou un de ses sels: 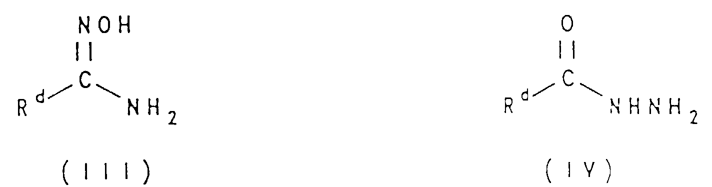 dans lesquelles un des radicaux R^{c} et R^{d} est un groupe de formule A, et l'autre est un groupe de formule -E-F tel que défini ci-dessus; ou
(B) cycliser un composé de formule XI: 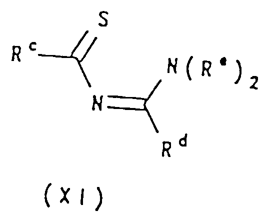 dans laquelle R^{c} et R^{d} sont tels que définis ci-dessus, et R^{e} est un atome d'hydrogène ou un groupe alkyle; ou
(C) réaliser une cycloaddition d'un sulfure de nitrile R^{c}-C≡N⁺ S⁻ avec un nitrile de formule R^{d}-CN, où R^{c} et R^{d} sont tels que définis ci-dessus; ou
(D) déshydrater un thiosemicarbazide de formule R^{c}CSNHNHCONR^{s}R^{t}, où R^{c} est tel que défini ci-dessus, et R^{s} et R^{t} sont un atome d'hydrogène ou un groupe alkyle; puis fixer le groupe R^{d} par un moyen classique; ou
(E) faire réagir un amide ou un thioamide de formule XII avec une α-halogénocétone de formule XIII: 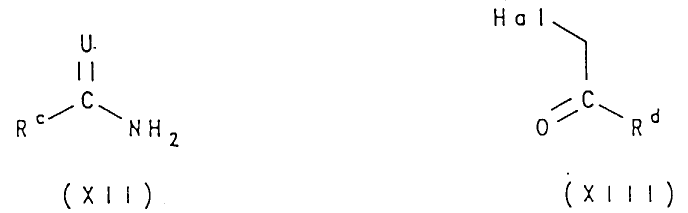 dans lesquelles U est un atome d'oxygène ou de soufre; Hal représente un atome d'halogène, et R^{c} et R^{d} sont tels que définis ci-dessus; ou
(F) faire réagir un composé de formule XV: 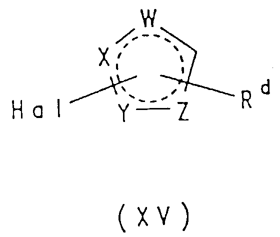 avec un réactif qui fournit un anion R^{c}, où W, X, Y, Z, R^{c} et R^{d} sont tels que définis ci-dessus, et Hal représente un atome d'halogène; ou
(G) faire réagir un composé de formule XVI: 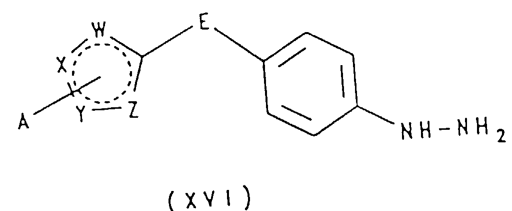 dans laquelle W, X, Y, Z, A et E sont tels que définis ci-dessus, avec un composé de formule VII ou une forme protégée par un groupe carbonyle de celui-ci: 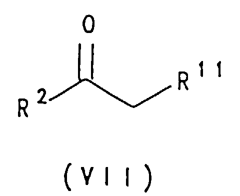
dans laquelle R² est tel que défini ci-dessus, et R¹¹ correspond au groupe R¹ défini ci-dessus, ou représente un groupe de formule -CH₂CHR⁴D, dans laquelle R⁴ est un atome d'hydrogène et D représente un groupe facile à éliminer; puis, le cas échéant, réaliser une N-alkylation par des procédés standards pour introduire le groupement R³ défini ci-dessus.

2. Procédé selon la revendication 1 de préparation d'un composé représenté par la formule IIA, et ses sels: 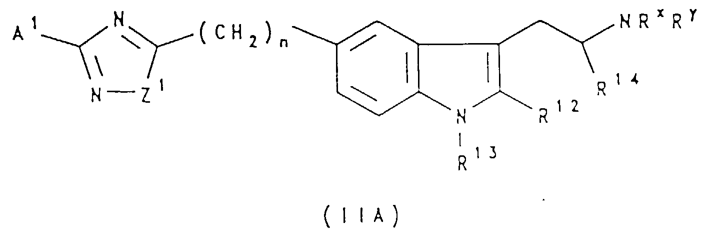 dans laquelle
Z¹ représente un atome d'oxygène ou de soufre;
n est nul ou vaut 1, 2 ou 3;
A¹ correspond au groupe A tel que défini dans la revendication 1;
R¹², R¹³ et R¹⁴ représentent chacun un atome d'hydrogène; et
R^{x} et R^{y} représentent indépendamment un atome d'hydrogène ou un groupe méthyle.

3. Procédé selon la revendication 1 de préparation d'un composé représenté par la formule IIB, et ses sels: 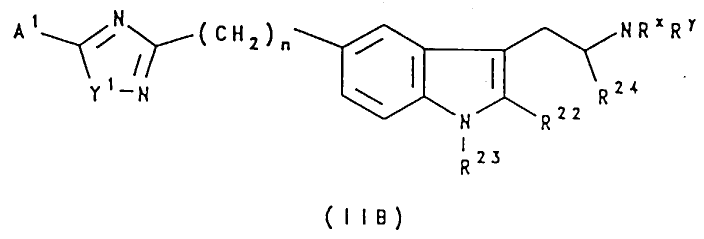 dans laquelle
Y¹ représente un atome d'oxygène ou de soufre;
n est nul ou vaut 1, 2 ou 3;
A¹ est tel que défini dans la revendication 2;
R²², R²³ et R²⁴ représentent chacun un atome d'hydrogène; et
R^{x} et R^{y} représentent indépendamment un atome d'hydrogène ou un groupe méthyle.

4. Procédé selon la revendication 1 de préparation d'un composé représenté par la formule IIC, et ses sels: 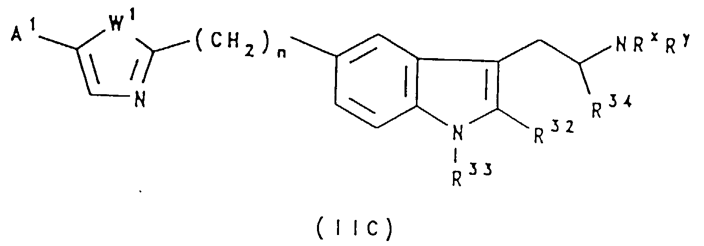 dans laquelle
W¹ représente un atome d'oxygène ou de soufre;
n est nul ou vaut 1, 2 ou 3;
A¹ est tel que défini dans la revendication 2;
R³², R³³ et R³⁴ représentent chacun un atome d'hydrogène; et
R^{x} et R^{y} représentent indépendamment un atome d'hydrogène ou un groupe méthyle.

5. Procédé selon la revendication 1 de préparation d'un composé représenté par la formule IID, et ses sels: 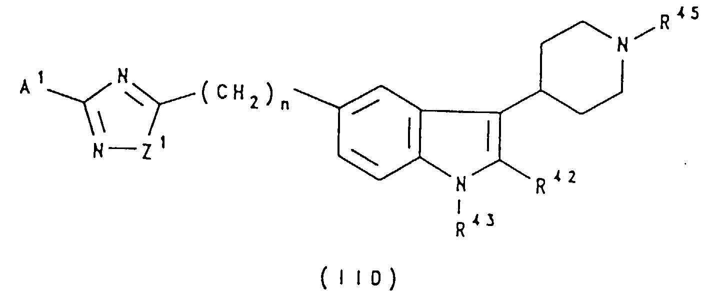 dans laquelle
Z¹ représente un atome d'oxygène ou de soufre;
n est nul ou vaut 1, 2 ou 3;
A¹ est tel que défini dans la revendication 2;
R⁴² et R⁴³ représentent chacun un atome d'hydrogène; et
R⁴⁵ représente un groupe méthyle.

6. Procédé selon la revendication 1 de préparation d'un composé choisi parmi:
la 2-[5-(3-benzyl-1,2,4-oxadiazole-5-yl)-1H-indole-3-yl]éthylamine;
la 2-[5-(3-méthyl-1,2,4-oxadiazole-5-yl)-1H-indole-3-yl]éthylamine;
la N,N-diméthyl-2-[5-(3-benzyl-1,2,4-oxadiazole-5-yl)-1H-indole-3-yl]éthylamine;
la 2-[5-(3-benzyl-1,2,4-oxadiazole-5-ylméthyl)-1H-indole-3-yl]éthylamine;
la 2-[5-(3-méthyl-1,2,4-oxadiazole-5-ylméthyl)-1H-indole-3-yl]éthylamine;
la 2-[5-(3-amino-1,2,4-oxadiazole-5-ylméthyl)-1H-indole-3-yl]éthylamine;
la 2-[5-(3-phényl-1,2,4-oxadiazole-5-ylméthyl)-1H-indole-3-yl]éthylamine;
la 2-[5-[3-(2-méthoxybenzyl)-1,2,4-oxadiazole-5-ylméthyl]-1H-indole-3-yl]éthylamine;
la N,N-diméthyl-2-[5-(3-benzyl-1,2,4-oxadiazole-5-ylméthyl)1H-indole-3-yl]éthylamine;
la 2-[5-[2-(3-benzyl-1,2,4-oxadiazole-5-yl)éthyl]-1H-indole-3-yl]éthylamine;
la N,N-diméthyl-2-[5-(3-méthyl-1,2,4-oxadiazole-5-ylméthyl)-1H-indole-3-yl]éthylamine;
la 2-[5-(3-diphénylméthyl-1,2,4-oxadiazole-5-yl)-1H-indole-3-yl]éthylamine;
la 2-[5-(3-phényl-1,2,4-oxadiazole-5-yl)-1H-indole-3-yl]éthylamine;
la 2-[5-[3-(2-méthoxybenzyl)-1,2,4-oxadiazole-5-yl]-1H-indole-3-yl]éthylamine;
la 2-[5-[3-(3-benzyl-1,2,4-oxadiazole-5-yl)propyl]-1H-indole-3-yl]éthylamine;
la 2-[5-(3-phénéthyl-1,2,4-oxadiazole-5-yl)-1H-indole-3-yl]éthylamine;
la 2-[5-(5-benzyl-1,2,4-oxadiazole-3-yl)-1H-indole-3-yl]éthylamine;
la 2-[5-(5-benzyl-1,2,4-oxadiazole-3-ylméthyl)-1H-indole-3-yl]éthylamine;
la N,N-diméthyl-2-[5-[3-(2-méthoxybenzyl) -1,2,4-oxadiazole-5-yl]-1H-indole-3-yl]éthylamine;
la N,N-diméthyl-2-[5-[2-(3-benzyl-1,2,4-oxadiazole-5-yl)éthyl]-1H-indole-3-yl]éthylamine;
la 2-[5-[3-(1-naphtyl)-1,2,4-oxadiazole-5-ylméthyl]-1H-indole-3-yl]éthylamine;
la 2-[5-[3-(3-méthyl-1,2,4-oxadiazole-5-yl)propyl]-1H-indole-3-yl]éthylamine;
la 2-[5-[3-(3-cyclopropyl-1,2,4-oxadiazole-5-yl)propyl]-1H-indole-3-yl]éthylamine;
la 2-[5-[3-(3-méthoxybenzyl)-1,2,4-oxadiazole-5-yl]-1H-indole-3-yl]éthylamine;
la 2-[5-[3-(4-méthoxybenzyl)-1,2,4-oxadiazole-5-yl]-1H-indole-3-yl]éthylamine;
la 2-[5-[3-(4-acétylaminobenzyl)-1,2,4-oxadiazole-5-yl]-1H-indole-3-yl]éthylamine;
la 2-[5-[3-(4-méthylsulfonylaminobenzyl)-1,2,4-oxadiazole-5-yl]-1H-indole-3-yl]éthylamine;
la 2-[5-[3-(3-phénylpropyl)-1,2,4-oxadiazole-5-yl]-1H-indole-3-yl]éthylamine;
la 2-[5-(3-cyclopropyl-1,2,4-oxadiazole-5-yl)-1H-indole-3-yl]éthylamine;
la 2-[5-(3-éthyl-1,2,4-oxadiazole-5-yl)-1H-indole-3-yl]éthylamine;
la 2-[5-[3-(4-trifluorométhylbenzyl)-1,2,4-oxadiazole-5-yl]-1H-indole-3-yl]éthylamine;
la N,N-diméthyl-2-[5-[3-(4-acétylaminobenzyl)-1,2,4-oxadiazole-5-yl]-1H-indole-3-yl]éthylamine;
la N,N-diméthyl-2-[5-[3-(4-méthylsulfonylaminobenzyl)-1,2,4-oxadiazole-5-yl]-1H-indole-3-yl]éthylamine;
la N,N-diméthyl-2-[5-(3-amino-1,2,4-oxadiazole-5-ylméthyl)-1H-indole-3-yl]éthylamine;
la 2-[5-[2-(3-amino-1,2,4-oxadiazole-5-yl)éthyl]-1H-indole-3-yl]éthylamine;
la 2-[5-[2-(3-diméthylamino-1,2,4-oxadiazole-5-yl)éthyl]-1H-indole-3-yl]éthylamine;
la 2-[5-(5-méthyl-1,2,4-oxadiazole-3-yl)-1H-indole-3-yl]éthylamine;
la 2-[5-(5-méthyl-1,2,4-oxadiazole-3-ylméthyl)-1H-indole-3-yl]éthylamine;
la N,N-diméthyl-2-[5-(5-benzyl-1,2,4-oxadiazole-3-ylméthyl)-1H-indole-3-yl]éthylamine;
la 2-[5-(3-méthoxyméthyl-1,2,4-oxadiazole-5-yl)-1H-indole-3-yl]éthylamine;
la 2-[5-[3-(4-méthylaminocarbonylbenzyl)-1,2,4-oxadiazole-5-yl]-1H-indole-3-yl]éthylamine;
la 2-[5-[3-(4-méthylaminocarbonylphényl)-1,2,4-oxadiazole-5-yl]-1H-indole-3-yl]éthylamine;
la 2-[5-[3-(4-méthylaminosulfonylbenzyl)-1,2,4-oxadiazole-5-yl]-1H-indole-3-yl]éthylamine;
la 2-[5-[3-(4-méthylsulfonylbenzyl)-1,2,4-oxadiazole-5-yl]-1H-indole-3-yl]éthylamine;
la 2-[5-[3-(3-méthylsulfonylaminobenzyl)-1,2,4-oxadiazole-5-yl]-1H-indole-3-yl]éthylamine;
la 2-[5-[3-(4-aminocarbonylaminobenzyl)-1,2,4-oxadiazole-5-yl]-1H-indole-3-yl]éthylamine;
la 2-[5-(3-amino-1,2,4-oxadiazole-5-yl)-1H-indole-3-yl]éthylamine;
la 2-[5-(3-acétylaminométhyl-1,2,4-oxadiazole-5-yl)-1H-indole-3-yl]éthylamine;
la 2-[5-[3-(2-acétylaminoéthyl)-1,2,4-oxadiazole-5-yl]-1H-indole-3-yl]éthylamine;
la 2-[5-(3-aminométhyl-1,2,4-oxadiazole-5-yl)-1H-indole-3-yl]éthylamine;
la N,N-diméthyl-2-[5-(3-amino-1,2,4-oxadiazole-5-yl)-1H-indole-3-yl]éthylamine;
la N,N-diméthyl-2-[5-(3-acétylaminométhyl-1,2,4-oxadiazole-5-yl)-1H-indole-3-yl]éthylamine;
la N,N-diméthyl-2-[5-[3-(2-acétylaminoéthyl)-1,2,4-oxadiazole-5-yl]-1H-indole-3-yl]éthylamine;
la N,N-diméthyl-2-[5-[3-(4-aminocarbonylaminobenzyl)-1,2,4-oxadiazole-5-yl]-1H-indole-3-yl]éthylamine;
la N,N-diméthyl-2-[5-[3-(2-(t-butoxycarbonylamino)éthyl)-1,2,4-oxadiazole-5-yl]-1H-indole-3-yl]éthylamine;
la N,N-diméthyl-2-[5-[3-(4-méthylaminocarbonylbenzyl)-1,2,4-oxadiazole-5-yl]-1H-indole-3-yl]éthylamine;
la N,N-diméthyl-2-[5-[3-(2-aminoéthyl)-1,2,4-oxadiazole-5-yl]-1H-indole-3-yl]éthylamine;
la N,N-diméthyl-2-[5-[3-(2-méthylsulfonylaminoéthyl)-1,2,4-oxadiazole-5-yl]-1H-indole-3-yl]éthylamine;
la N,N-diméthyl-2-[5-[3-(2-aminocarbonylaminoéthyl)-1,2,4-oxadiazole-5-yl]-1H-indole-3-yl]éthylamine;
la N,N-diméthyl-2-[5-[3-(2-méthylaminocarbonylaminoéthyl)-1,2,4-oxadiazole-5-yl]-1H-indole-3-yl]éthylamine;
la N,N-diméthyl-2-[5-[3-(2-méthylaminocarbonylaminoéthyl)-1,2,4-oxadiazole-5-yl]-1H-indole-3-yl]éthylamine;
la N,N-diméthyl-2-[5-[3-(2-méthoxycarbonylaminoéthyl)-1,2,4-oxadiazole-5-yl]-1H-indole-3-yl]éthylamine;
la N,N-diméthyl-2-[5-[3-(2-éthoxycarbonylaminoéthyl)-1,2,4-oxadiazole-5-yl]-1H-indole-3-yl]éthylamine;
la N,N-diméthyl-2-[5-[2-(3-amino-1,2,4-oxadiazole-5-yl)éthyl]-1H-indole-3-yl]éthylamine;
la N,N-diméthyl-2-[5-(3-méthylamino-1,2,4-oxadiazole-5-ylméthyl)-1H-indole-3-yl]éthylamine;
la N,N-diméthyl-2-[5-[3-(4-aminocarbonylbenzyl)-1,2,4-oxadiazole-5-ylméthyl]-1H-indole-3-yl]éthylamine;
la N,N-diméthyl-2-[5-[3-(4-acétylaminobenzyl)-1,2,4-oxadiazole-5-ylméthyl]-1H-indole-3-yl]éthylamine;
la N,N-diméthyl-2-[5-[3-(4-méthylaminosulfonylbenzyl)-1,2,4-oxadiazole-5-ylméthyl]-1H-indole-3-yl]éthylamine;
la N,N-diméthyl-2-[5-[3-(4-aminocarbonylaminobenzyl)-1,2,4-oxadiazole-5-ylméthyl]-1H-indole-3-yl]éthylamine;
la N,N-diméthyl-2-[5-[3-(4-méthylsulfonylaminobenzyl)-1,2,4-oxadiazole-5-ylméthyl]-1H-indole-3-yl]éthylamine;
la N,N-diméthyl-2-[5-[3-(4-méthylaminocarbonylphényl)-1,2,4-oxadiazole-5-ylméthyl]-1H-indole-3-yl]éthylamine;
la N,N-diméthyl-2-[5-(3-acétylaminométhyl-1,2,4-oxadiazole-5-ylméthyl)-1H-indole-3-yl]éthylamine;
la N,N-diméthyl-2-[5-(3-méthylsulfonylaminométhyl-1,2,4-oxadiazole-5-ylméthyl)-1H-indole-3-yl]éthylamine;
la N,N-diméthyl-2-[5-(3-aminocarbonylméthyl-1,2,4-oxadiazole-5-ylméthyl)-1H-indole-3-yl]éthylamine;
la N,N-diméthyl-2-[5-[3-(3-méthylsulfonylaminobenzyl)-1,2,4-oxadiazole-5-ylméthyl]-1H-indole-3-yl]éthylamine;
la N,N-diméthyl-2-[5-[3-(3-acétylaminobenzyl)-1,2,4-oxadiazole-5-ylméthyl]-1H-indole-3-yl]éthylamine;
la N,N-diméthyl-2-[5-[3-(4-aminocarbonylphényl)-1,2,4-oxadiazole-5-ylméthyl]-1H-indole-3-yl]éthylamine;
la N,N-diméthyl-2-[5-[3-(3-aminocarbonylphényl)-1,2,4-oxadiazole-5-ylméthyl]-1H-indole-3-yl]éthylamine;
la N,N-diméthyl-2-[5-[3-(4-méthylsulfonylaminophényl)-1,2,4-oxadiazole-5-ylméthyl]-1H-indole-3-yl]éthylamine;
la N,N-diméthyl-2-[5-[3-(4-méthylaminosulfonylméthylphényl)-1,2,4-oxadiazole-5-ylméthyl]-1H-indole-3-yl]éthylamine;
la N,N-diméthyl-2-[5-[3-(3-méthylaminosulfonylméthylphényl)-1,2,4-oxadiazole-5-ylméthyl]-1H-indole-3-yl]éthylamine;
la N,N-diméthyl-2-[5-[3-(4-aminosulfonylméthylphényl)-1,2,4-oxadiazole-5-ylméthyl]-1H-indole-3-yl]éthylamine;
la N,N-diméthyl-2-[5-[3-(4-diméthylaminosulfonylméthylphényl)-1,2,4-oxadiazole-5-ylméthyl]-1H-indole-3-yl]éthylamine;
la N,N-diméthyl-2-[5-[3-(t-butoxycarbonylamino)méthyl-1,2,4-oxadiazole-5-ylméthyl]-1H-indole-3-yl]éthylamine;
la N,N-diméthyl-2-[5-[3-(2-(t-butoxycarbonylamino)éthyl)-1,2,4-oxadiazole-5-ylméthyl]-1H-indole-3-yl]éthylamine;
la N,N-diméthyl-2-[5-(3-aminométhyl-1,2,4-oxadiazole-5-ylméthyl)-1H-indole-3-yl]éthylamine;
la N,N-diméthyl-2-[5-(3-méthoxycarbonylaminométhyl-1,2,4-oxadiazole-5-ylméthyl)-1H-indole-3-yl]éthylamine;
la N,N-diméthyl-2-[5-(3-diméthylaminométhyl-1,2,4-oxadiazole-5-ylméthyl)-1H-indole-3-yl]éthylamine;
la N,N-diméthyl-2-[5-[3-(2-méthylsulfonylaminoéthyl)-1,2,4-oxadiazole-5-ylméthyl]-1H-indole-3-yl]éthylamine;
la N,N-diméthyl-2-[5-[3-(2-éthoxycarbonylaminoéthyl)-1,2,4-oxadiazole-5-ylméthyl]-1H-indole-3-yl]éthylamine;
la N,N-diméthyl-2-[5-(3-benzoylaminométhyl-1,2,4-oxadiazole-5-ylméthyl)-1H-indole-3-yl]éthylamine;
la N,N-diméthyl-2-[5-[3-(2-benzoylaminoéthyl)-1,2,4-oxadiazole-5-ylméthyl]-1H-indole-3-yl]éthylamine;
la N,N-diméthyl-2-[5-[3-(2-phénylaminocarbonylaminoéthyl)-1,2,4-oxadiazole-5-ylméthyl]-1H-indole-3-yl]éthylamine;
la N,N-diméthyl-2-[5-[3-(2-(t-butylaminocarbonylamino)éthyl)-1,2,4-oxadiazole-5-ylméthyl]-1H-indole-3-yl]éthylamine;
la N-méthyl-2-[5-(3-amino-1,2,4-oxadiazole-5-ylméthyl)-1H-indole-3-yl]éthylamine;
la N,N-diméthyl-2-[5-[3-(4-(t-butoxycarbonyl)pipérazine-1-yl)-1,2,4-oxadiazole-5-ylméthyl]-1H-indole-3-yl]éthylamine;
la N,N-diméthyl-2-[5-[3-(4-méthylsulfonylpipérazine-1-yl)-1,2,4-oxadiazole-5-ylméthyl]-1H-indole-3-yl]éthylamine;
la N,N-diméthyl-2-[5-[3-(4-méthoxycarbonylpipérazine-1-yl)-1,2,4-oxadiazole-5-ylméthyl]-1H-indole-3-yl]éthylamine;
la N,N-diméthyl-2-[5-[3-(4-méthylaminocarbonylpipérazine-1-yl)-1,2,4-oxadiazole-5-ylméthyl]-1H-indole-3-yl]éthylamine;
la N,N-diméthyl-2-[5-[3-(4-acétylpipérazine-1-yl)-1,2,4-oxadiazole-5-ylméthyl]-1H-indole-3-yl]éthylamine;
la N,N-diméthyl-2-[5-[3-(4-méthylsulfonylaminométhylphényl)-1,2,4-oxadiazole-5-ylméthyl]-1H-indole-3-yl]éthylamine;
la N,N-diméthyl-2-[5-(3-phénylsulfonylaminométhyl-1,2,4-oxadiazole-5-ylméthyl]-1H-indole-3-yl]éthylamine;
la N,N-diméthyl-2-[5-[3-benzylamino-1,2,4-oxadiazole-5-ylméthyl]-1H-indole-3-yl]éthylamine;
la N,N-diméthyl-2-[5-[3-(3-pyridyl)méthyl-1,2,4-oxadiazole-5-ylméthyl]-1H-indole-3-yl]éthylamine;
la N,N-diméthyl-2-[5-[3-(2-méthoxypyrid-5-yl)méthyl-1,2,4-oxadiazole-5-ylméthyl]-1H-indole-3-yl]éthylamine;
la 2-[5-[3-(4-acétylaminobenzyl)-1,2,4-oxadiazole-5-ylméthyl]-1H-indole-3-yl]éthylamine;
la 2-[5-[3-(4-méthylsulfonylaminobenzyl)-1,2,4-oxadiazole-5-ylméthyl]-1H-indole-3-yl]éthylamine;
la 2-[5-[2-[3-(4-acétylaminobenzyl)-1,2,4-oxadiazole-5-yl]éthyl]-1H-indole-3-yl]éthylamine;
la 2-[5-[2-[3-(4-méthoxybenzyl)-1,2,4-oxadiazole-5-yl]éthyl]-1H-indole-3-yl]éthylamine;
la N,N-diméthyl-2-[5-(5-méthyl-1,3-oxazole-2-yl)-1H-indole-3-yl]éthylamine;
la N,N-diméthyl-2-[5-[2-(5-méthyl-1,3-oxazole-2-yl)éthyl]-1H-indole-3-yl]éthylamine;
la 1-méthyl-4-[5-(3-amino-1,2,4-oxadiazole-5-yl)-1H-indole-3-yl]pipéridine;
la 1-méthyl-4-[5-(3-amino-1,2,4-oxadiazole-5-ylméthyl)-1H-indole-3-yl]pipéridine;
la 1-méthyl-4-[5-(3-(4-méthylsulfonylaminobenzyl)-1,2,4-oxadiazole-5-ylméthyl]-1H-indole-3-yl]pipéridine;
la 1-méthyl-4-[5-(3-(3-pyridyl)méthyl-1,2,4-oxadiazole-5-ylméthyl]-1H-indole-3-yl]pipéridine;
la N,N-diméthyl-2-[5-[3-(4-pyridyl)méthyl-1,2,4-oxadiazole-5-ylméthyl)-1H-indole-3-yl]éthylamine;
la N,N-diméthyl-2-[5-[3-(2-(t-butoxycarbonylamino)éthyl)amino-1,2,4-oxadiazole-5-ylméthyl )-1H-indole-3-yl]éthylamine;
la 2-[5-(3-aminocarbonyl-1,2,4-oxadiazole-5-ylméthyl)-1H-indole-3-yl]éthylamine;
la 2-[5-(3-méthylaminocarbonyl-1,2,4-oxadiazole-5-ylméthyl)-1H-indole-3-yl]éthylamine;
la 2-[5-[3-(pyrrolid-1-yl)carbonyl-1,2,4-oxadiazole-5-ylméthyl]-1H-indole-3-yl]éthylamine;
la 2-[5-[3-(azétidin-1-yl)carbonyl-1,2,4-oxadiazole-5-yl-méthyl]-1H-indole-3-yl]éthylamine;
la N,N-diméthyl-2-[5-[3-(4-phénylsulfonylpipérazine-1-yl)-1,2,4-oxadiazole-5-ylméthyl]-1H-indole-3-yl]éthylamine;
la N,N-diméthyl-2-[5-[3-(2-(pyrrolid-1-ylcarbonylaminoéthyl)-1,2,4-oxadiazole-5-ylméthyl]-1H-indole-3-yl]éthylamine;
la N,N-diméthyl-2-[5-[3-(2-méthylsulfonylaminoéthyl)amino)-1,2,4-oxadiazole-5-ylméthyl]-1H-indole-3-yl]éthylamine;
la N,N-diméthyl-2-[5-(3-amino-1,4-thiadiazole-5-ylméthyl)-1H-indole-3-yl]éthylamine;
et leurs sels.

7. Procédé de préparation d'une composition pharmaceutique qui comprend le mélange d'un composé préparé selon l'une quelconque des revendications précédentes avec un véhicule ou excipient pharmaceutiquement acceptable.

8. Utilisation d'un composé selon l'une quelconque des revendications 1 à 6 pour la fabrication d'un médicament pour le traitement et/ou la prévention de conditions cliniques pour lesquelles un agoniste sélectif des récepteurs de type 5-HT₁ est indiqué.
